# EUROPEAN PATENT APPLICATION

(11) **EP 4 844 776 A2**
(43) Date of publication of application: **25.11.2026**
(21) Application number: 26190140.9
(22) Date of filing: 06.04.2023
(51) Int. Cl.: G16H 15/00

(54) **FACILITATING ACCESS TO ANALYTE DATA**

(30) Priority: 06.04.2022 US 202263328078 P
(62) Document number(s) of the earlier application(s) in accordance with Art. 76 EPC: 23720474.8 / 4 505 473
(71) Applicant: Abbott Diabetes Care Inc., Alameda, California 94502 (US)
(72) Inventor: TSUCHIDA, Roy, San Jose, CA 95130 (US); HAYTER, Gary Alan, Oakland, CA 94618 (US)
(74) Representative: Mathys & Squire

(57) **Abstract**

Embodiments described herein include a glucose monitoring system. The glucose monitoring system is configured to use various methods and additional systems to facilitate the transfer of glucose data from a data receiving device without wireless network capabilities. Certain embodiments facilitate the transfer of data to multi-purpose devices, user devices, and electronic medical records (EMR) systems. Certain embodiments facilitate the transfer of data to report generating systems that interpret the provided data and reports based on the glucose data. Certain embodiments relate to techniques for facilitating the integration of EMR system data management and reporting.

## Description

### CROSS REFERENCE TO RELATED APPLICATIONS

This application claims the benefit of U.S. Provisional Patent Application No. 63/328,078 filed April 6, 2022, which is hereby incorporated by reference in its entirety.

### FIELD

The subject matter described herein relates to systems and devices for receiving data, and methods for control of systems and devices for receiving data, for example, with respect to operation of a handheld device forming part of an in vivo analyte monitoring system.

### BACKGROUND

The frequent monitoring and management of analyte levels, such as glucose, ketones, lactate, oxygen, hemoglobin A1C, or the like, can improve the overall health of people and, in particular, people with diabetes. As an example, diabetics are generally required to monitor their glucose levels to ensure that they are being maintained within a clinically safe range, and can also use this information to determine when insulin is needed to manage glucose levels in their bodies or when glucose is needed to raise the level of glucose in their bodies. Clinical data demonstrates a strong correlation between the frequency of glucose monitoring and glycemic control. Despite such correlation, however, many individuals diagnosed with a diabetic condition do not monitor their glucose levels as frequently as they should due to a combination of factors including convenience, testing discretion, pain associated with glucose testing, and cost.

To increase patient adherence to a plan of frequent glucose monitoring, in vivo analyte monitoring systems can be utilized, in which a sensor control device can be worn on the body of an individual who requires analyte monitoring. To increase comfort and convenience for the individual, the sensor control device can have a small form-factor, and can be assembled and applied by the individual with a sensor applicator. The application process includes inserting a sensor, such as an in vivo sensor that senses a user's analyte level in a bodily fluid located in the dermal layer of the human body, using an applicator or insertion mechanism, such that the sensor comes into contact with a bodily fluid. The sensor control device can also be configured to transmit analyte data to one or more data receiving devices, from which the individual, her health care provider ("HCP"), or others can review the data and make therapy decisions.

In vivo analyte monitoring systems can be broadly classified based on the manner in which data is communicated between the reader device and the sensor control device. One type of in vivo system is a "Continuous Analyte Monitoring" system, where data can be broadcast from the sensor control device to a data receiving device continuously without prompting, e.g., in an automatic fashion according to a broadcast schedule. Another type of in vivo system is a "Flash Analyte Monitoring" system, where data can be transferred from the sensor control device in response to a scan or request for data by the data receiving device, such as with a Near Field Communication (NFC) or Radio Frequency Identification (RFID) protocol. A data receiving device can include a variety of hardware components to enable the processing of analyte data received from the sensor control device, and must include telecommunications components to enable the communication with the sensor control device. Data receiving devices can further include additional testing or sending hardware to assist the individual or their HCP in making therapy decisions.

Data receiving devices can be designed with restricted features to control cost and complexity. As such, data receiving devices can lack wide area networking capabilities that might facilitate transferring data from a data receiving to a central application server. Central application servers are often used to analyze data on a per-patient level as well as to analyze larger trends in patient data. Instead of simple wireless connectivity, data receiving devices can be connected to a user device, such as a laptop computer, using a wired connection. The user device then relays relevant data to the remote server. However, it can be cumbersome and inconvenient to provide the wired connection between the data receiving device and the user device. For example, software updates on either device can necessitate updating drivers to ensure the connection is stable and secure. Additionally, patients visiting their HCPs who wish to use the reports must remember to upload their data prior to their appointments. Additionally, users are often requested to enter account credentials, such as a username, user id, password, etc., in order for data from their data receiving device to be uploaded to the application server for processing and made available to their HCP.

Accordingly, it would be beneficial to incorporate alternative methods for retrieving analyte data from a data receiving device so that the analyte data can be used to generate reports or be interpreted by an HCP or other user. In particular, it would be beneficial to incorporate a method for retrieving analyte data from a data receiving device without requiring a wired connection to a user device, the data receiving device to wirelessly connect to an application server, or any user to have an account with the application server.

### SUMMARY

The purpose and advantages of the disclosed subject matter will be set forth in and apparent from the description that follows, as well as will be learned by practice of the disclosed subject matter. Additional advantages of the disclosed subject matter will be realized and attained by the methods and systems particularly pointed out in the written description and claims hereof, as well as from the drawings.

Examples described herein include analyte monitoring systems and methods and operations performed thereby. An example analyte monitoring system can be configured to use various methods and additional systems to facilitate the transfer of analyte data from a data receiving device without wireless network capabilities. In certain examples, the analyte is glucose. In examples where a specific analyte is being monitored, and the data relates to that specific analyte, the analyte monitoring system can be referred to as a monitoring system for that analyte. For example, where the analyte is glucose, the analyte monitoring system can be referred to as a glucose monitoring system without deviating from the scope of the disclosure herein for an analyte monitoring system. The analyte monitoring system may optionally comprise an analyte sensor. The analyte sensor may optionally form part of a sensor control device of the analyte monitoring system. Wherein the monitored analyte is glucose, for example, the glucose monitoring system may optionally comprise a glucose sensor. The glucose sensor may optionally form part of a sensor control device of the glucose monitoring system.

Certain examples facilitate the transfer of data to multi-purpose devices, user devices, and electronic medical records (EMR) systems. Certain examples facilitate the transfer of data to report generating systems that interpret the provided data and reports based on the analyte data. Certain examples relate to techniques for facilitating the integration of EMR system data management and reporting. In all examples where the data is transferred to systems other than personal data receiving devices (including, as described herein analyte data receiving devices, multi-purpose devices, and user devices), the data can be transferred to server computing devices (commonly referred to as "servers") operated by and embodying the function of those systems. For ease of understanding and clarity, this disclosure describes transferring data to and from those systems rather than explicitly stating transferring data to and from servers operated by and embodying the functions of those systems in each instance. As such, when this disclosure described transferred data to or from, for example, analyte monitoring systems EMR systems, report generating systems, and components thereof, this disclosure contemplates transferring data to or from servers operated by and embodying the functions of those systems, respectively.

To achieve these and other advantages and in accordance with the purpose of the disclosed subject matter, as embodied and broadly described, the disclosed subject matter includes systems and devices for receiving and processing data in analyte monitoring systems, and methods for control of said systems and devices. Exemplary systems and methods can include a server computing device of a glucose monitoring system. The server computing device includes one or more processors and a memory in communication with the one or more processors that stores instructions that, when executed by the one or more processors, are configured to cause the one or more processors to perform operations. The operations can include receiving, from an electronic medical records (EMR) system, a request to establish a connection between a set of patient records stored by the EMR system and associated with a patient and a set of patient records stored by the glucose monitoring system and associated with the patient. The request can include patient identifying information. The operations can include comparing the patient identifying information received with the request with patient identifying information stored by the glucose monitoring system. The operations can include, in response to determining, based on the comparison, that the set of patient records stored by the glucose monitoring system and associated with the patient exist, generating an association between the set of patient records stored by the EMR system and associated with the patient and the set of patient records stored by the glucose monitoring system and associated with the patient and sending, to the EMR system, a confirmation of the association. The operations can include, in response to determining, based on the comparison, that the set of patient records stored by the glucose monitoring system does not exist, sending, to the EMR system, a notification that the request to establish the connection cannot been completed. In some examples, the patient identifying information includes patient name, patient date of birth, patient address, patient email address, patient phone number, or patient health care provider information. In some examples, patient identifying information includes a patient identification code that is an identifier unique to the patient in the EMR system. In some examples, the operations can include determining that new glucose data associated with the set of patient records stored by the glucose monitoring system and associated with the patient is available and sending, to the EMR system, the new glucose data associated with the set of patient records stored by the glucose monitoring system and associated with the patient. In some examples, the operations can include requesting, from the EMR system, patient data associated with the set of patient records stored by the EMR system and associated with the patient and receiving, from the EMR system in lieu of the requested patient data, a message indicating that no new patient data associated with the set of patient records stored by the EMR system and associated with the patient is available. In some examples, the operations can include receiving, from the EMR system, a request for new glucose data associated with the set of patient records stored by the glucose monitoring system and associated with the patient, determining that no new glucose data associated with the set of patient records stored by the glucose monitoring system and associated with the patient is available, and sending, to the EMR system in lieu of the requested patient data, a message indicating that no new glucose data associated with the set of patient records stored by the glucose monitoring system and associated with the patient is available. In some examples, the operations can include, and optionally, after the confirmation of the association, generating, with an account access code service, an access code associated with the set of patient records stored by the glucose monitoring system and associated with the patient and sending, to the EMR system, the access code associated with the confirmation of the association. In some examples, the access code functions as or is interpreted as the confirmation of the association. In some examples, the access code is transmitted with the confirmation of association. In some examples, the operations can include, and optionally, after the confirmation of the association, sending, to the EMR system, glucose data associated with the set of patient records stored by the glucose monitoring system and associated with the patient. In some examples, the patient data associated with the set of patient records stored by the EMR system and associated with the patient are new patient data received from the EMR system when the new patient data becomes available.

In some examples, the operations can include, and optionally, after the confirmation of the association, periodically sending, to the EMR system, glucose data associated with the set of patient records stored by the glucose monitoring system and associated with the patient. In some examples, the operations can include, and optionally, after the confirmation of the association, periodically receiving, from the EMR system, patient data associated with the set of patient records stored by the EMR system and associated with the patient. In some examples, the request to establish a connection further includes an access code generated by an account access code service. The operations can further include, prior to sending the confirmation of the association determining, using the account access code service, that the access code is associated with the set of patient records stored by the glucose monitoring system and associated with the patient, and determining, using the account access code service, the validity of the access code. In some examples, the access code is configured to be valid for a predetermined period of time. In some examples, the notification that the request to establish the connection cannot been completed further includes instructions to establish the connection. In some examples, the operations further include, subsequent to sending the confirmation of the association: receiving, by the server computing device and from the EMR system, a request to access a report generated based on glucose data stored in the set of patient records stored by the glucose monitoring system and associated with the patient, generating, by the server computing device and with a report generating system, the requested report, and sending, by the server computing device and to the EMR system, the generated report. In some examples, the request to access the report identifies the glucose data for which the report is requested. In some examples, the operations can include requesting, from the EMR system, updated patient data associated with the set of patient records stored by the EMR system and associated with the patient and receiving, from the EMR system, the updated patient data associated with the set of patient records stored by the EMR system and associated with the patient, wherein the requested report is generated based on the updated patient data. In some examples, the operations further include identifying glucose data to include in the requested report based on a datestamp or timestamp associated with the identified glucose data.

According to other aspects of the disclosed subject matter, systems and methods include a method of establishing a connection or association between a set of patient records stored by an EMR system and associated with a patient and a set of patient records stored by a glucose monitoring system and associated with the patient. The method can include receiving, from an electronic medical records (EMR) system, a request to establish a connection between a set of patient records stored by the EMR system and associated with a patient and a set of patient records stored by the glucose monitoring system and associated with the patient. The request can include patient identifying information. The method can include comparing the patient identifying information received with the request with patient identifying information stored by the glucose monitoring system. The method can include, in response to determining, based on the comparison, that the set of patient records stored by the glucose monitoring system and associated with the patient exist, generating an association between the set of patient records stored by the EMR system and associated with the patient and the set of patient records stored by the glucose monitoring system and associated with the patient and sending, to the EMR system, a confirmation of the association. The method can include, in response to determining, based on the comparison, that the set of patient records stored by the glucose monitoring system does not exist, sending, to the EMR system, a notification that the request to establish the connection cannot been completed. In some examples, the patient identifying information includes patient name, patient date of birth, patient address, patient email address, patient phone number, or patient health care provider information. In some examples, patient identifying information includes a patient identification code that is an identifier unique to the patient in the EMR system. In some examples, the method can include determining that new glucose data associated with the set of patient records stored by the glucose monitoring system and associated with the patient is available and sending, to the EMR system, the new glucose data associated with the set of patient records stored by the glucose monitoring system and associated with the patient. In some examples, the method can include requesting, from the EMR system, patient data associated with the set of patient records stored by the EMR system and associated with the patient and receiving, from the EMR system in lieu of the requested patient data, a message indicating that no new patient data associated with the set of patient records stored by the EMR system and associated with the patient is available. In some examples, the method can include receiving, from the EMR system, a request for new glucose data associated with the set of patient records stored by the glucose monitoring system and associated with the patient, determining that no new glucose data associated with the set of patient records stored by the glucose monitoring system and associated with the patient is available, and sending, to the EMR system in lieu of the requested patient data, a message indicating that no new glucose data associated with the set of patient records stored by the glucose monitoring system and associated with the patient is available. In some examples, the method can include generating, with an account access code service, an access code associated with the set of patient records stored by the glucose monitoring system and associated with the patient and sending, to the EMR system, the access code associated with the confirmation of the association. In some examples, the method can include sending, to the EMR system, glucose data associated with the set of patient records stored by the glucose monitoring system and associated with the patient. In some examples, the patient data associated with the set of patient records stored by the EMR system and associated with the patient are new patient data received from the EMR system when the new patient data becomes available.

In some examples, the method can include periodically sending, to the EMR system, glucose data associated with the set of patient records stored by the glucose monitoring system and associated with the patient. In some examples, the method can include periodically receiving, from the EMR system, patient data associated with the set of patient records stored by the EMR system and associated with the patient. In some examples, the request to establish a connection further includes an access code generated by an account access code service. The method can further include, prior to sending the confirmation of the association determining, using the account access code service, that the access code is associated with the set of patient records stored by the glucose monitoring system and associated with the patient, and determining, using the account access code service, the validity of the access code. In some examples, the access code is configured to be valid for a predetermined period of time. In some examples, the notification that the request to establish the connection cannot been completed further includes instructions to establish the connection. In some examples, the method can further include, subsequent to sending the confirmation of the association: receiving, by the server computing device and from the EMR system, a request to access a report generated based on glucose data stored in the set of patient records stored by the glucose monitoring system and associated with the patient, generating, by the server computing device and with a report generating system, the requested report, and sending, by the server computing device and to the EMR system, the generated report. In some examples, the request to access the report identifies the glucose data for which the report is requested. In some examples, the method can include requesting, from the EMR system, updated patient data associated with the set of patient records stored by the EMR system and associated with the patient and receiving, from the EMR system, the updated patient data associated with the set of patient records stored by the EMR system and associated with the patient, wherein the requested report is generated based on the updated patient data. In some examples, the method can further include identifying glucose data to include in the requested report based on a datestamp or timestamp associated with the identified glucose data.

According to other aspects of the disclosed subject matter, systems and methods include server computing device of a glucose monitoring system. The server computing device includes one or more processors and a memory in communication with the one or more processors that stores instructions that, when executed by the one or more processors, are configured to cause the one or more processors to perform operations. The operations can include receiving a first web request including one or more parameters from a multi-purpose device of the glucose monitoring system. The one or more parameters include glucose data associated with a user of the glucose monitoring system. The operations can include generating a report based on the received glucose data. The operations can include associating an access code with the report. The operations can include receiving a second web request including one or more parameters. The one or more parameters can include the access code. The operations can include retrieving the report based on the access code. The operations can include causing the report to be output on a display. In some examples, the operations can include sending the access code to the multi-purpose device in response to receiving the first web request. In some examples, the second web request is received from a computing device associated with an electronic medical records (EMR) system. In some examples, the second web request is received from the multi-purpose device. In some examples, the second web request is received form a computing device associated with the user of the glucose monitoring system. In some examples, the first web request is sent by the multi-purpose device subsequent to scanning a matrix barcode. In some examples, the one or more parameters further include a device identifier associated with the multi-purpose device, a device identifier associated with a data receiving device, or a patient identifier associated with the user.

According to other aspects of the disclosed subject matter, systems and methods include a method of providing access to a glucose report. The method can include receiving a first web request including one or more parameters from a multi-purpose device of the glucose monitoring system. The one or more parameters include glucose data associated with a user of the glucose monitoring system. The method can include generating a report based on the received glucose data. The method can include associating an access code with the report. The method can include receiving a second web request including one or more parameters. The one or more parameters can include the access code. The method can include retrieving the report based on the access code. The method can include causing the report to be output on a display. In some examples, the method can include sending the access code to the multi-purpose device in response to receiving the first web request. In some examples, the second web request is received from a computing device associated with an electronic medical records (EMR) system. In some examples, the second web request is received from the multi-purpose device. In some examples, the second web request is received form a computing device associated with the user of the glucose monitoring system. In some examples, the first web request is sent by the multi-purpose device subsequent to scanning a matrix barcode. In some examples, the one or more parameters further include a device identifier associated with the multi-purpose device, a device identifier associated with a data receiving device, or a patient identifier associated with the user.

According to other aspects of the disclosed subject matter, systems and methods include a data receiving device of a glucose monitoring system. The data receiving device can include a display, one or more processors, and a memory in communication with the one or more processors that stores instructions that cause the one or more processors to perform operations. The operations can include receiving, from a user of the glucose monitoring system, a request to transfer glucose data associated with the user to another computing device. The operations can include encoding the glucose data. The operations can include generating a matrix barcode comprising a uniform resource locator (URL) and the encoded glucose data. The glucose data can be formatted as parameters to be passed with the URL by a web browser. The operations can include causing the matrix barcode to be presented on the display. In some examples, the operations can include selecting the glucose data for encoding based on the glucose data corresponding to a predetermined range of time. In some examples, the operations can include calculating one or more derivative values from the glucose data and encoding the derivative values with the glucose data. In some examples, encoding the glucose data can include reducing a level of precision associated with the glucose data. The level of precision can be selected based on an actual value of the glucose data. In some examples. The data receiving device is not configured for wireless communication. In some examples, the operations can include generating report elements for a glucose report based on the glucose data. In some examples, the operations can include encoding the report elements for the glucose report. The matrix barcode can further include the encoded glucose report elements.

According to other aspects of the disclosed subject matter, systems and methods include a method of generating a matrix barcode by a data receiving device of a glucose monitoring system. The method can include receiving, from a user of the glucose monitoring system, a request to transfer glucose data associated with the user to another computing device. The method can include encoding the glucose data. The method can include generating a matrix barcode comprising a uniform resource locator (URL) and the encoded glucose data. The glucose data can be formatted as parameters to be passed with the URL by a web browser. The method can include causing the matrix barcode to be presented on the display. In some examples, the method can include selecting the glucose data for encoding based on the glucose data corresponding to a predetermined range of time. In some examples, the method can include calculating one or more derivative values from the glucose data and encoding the derivative values with the glucose data. In some examples, encoding the glucose data can include reducing a level of precision associated with the glucose data. The level of precision can be selected based on an actual value of the glucose data. In some examples. The data receiving device is not configured for wireless communication. In some examples, the method can include generating report elements for a glucose report based on the glucose data. In some examples, the method can include encoding the report elements for the glucose report. The matrix barcode can further include the encoded glucose report elements.

According to other aspects of the disclosed subject matter, systems and methods include a multi-purpose device of a glucose monitoring system. The multi-purpose device can include a camera, a display, one or more processors, and a memory in communication with the one or more processors that stores instructions that cause the one or more processors to perform operations. The operations can include causing a matrix barcode to be scanned via the camera. The operations can include decoding the matrix barcode to obtain a uniform resource locator (URL) and one or more parameters. The one or more parameters can include at least glucose data. The operations can include presenting, using the URL and the one or more parameters, a web request to a server associated with the URL. The operations can include receiving, by the multi-purpose device and from the user associated with the user, an access code associated with the one or more parameters. The operations can include causing the access code to be output via the display. In some examples, the server associated with the URL is further associated with the glucose monitoring system. In some examples, the access code is a temporary one-time password associated with the one or more parameters by the server. In some examples, the access code is configured to expired after a predetermined period of time. In some examples, the operations can include receiving, by the multi-purpose device and from the server associated with the URL, a report based on the glucose data included in the parameters. The report can be generated by the server based on the parameters presented with the URL. The operations can include causing the report to be output via a display. In some examples, the matrix barcode is generated and displayed by a data receiving device of the glucose monitoring system, and the one or more parameters include a device identifier associated with the multi-purpose device, a device identifier associated with the data receiving device, or a patient identifier associated with the user. In some examples, the data receiving device is not configured for wireless communication.

According to other aspects of the disclosed subject matter, systems and methods include a method of generating an access code. The method can include causing a matrix barcode to be scanned via the camera. The method can include decoding the matrix barcode to obtain a uniform resource locator (URL) and one or more parameters. The one or more parameters can include at least glucose data. The method can include presenting, using the URL and the one or more parameters, a web request to a server associated with the URL. The method can include receiving, by the multi-purpose device and from the user associated with the user, an access code associated with the one or more parameters. The method can include causing the access code to be output via the display. In some examples, the server associated with the URL is further associated with the glucose monitoring system. In some examples, the access code is a temporary one-time password associated with the one or more parameters by the server. In some examples, the access code is configured to expired after a predetermined period of time. In some examples, the method can include receiving, by the multi-purpose device and from the server associated with the URL, a report based on the glucose data included in the parameters. The report can be generated by the server based on the parameters presented with the URL. The method can include causing the report to be output via a display. In some examples, the matrix barcode is generated and displayed by a data receiving device of the glucose monitoring system, and the one or more parameters include a device identifier associated with the multi-purpose device, a device identifier associated with the data receiving device, or a patient identifier associated with the user. In some examples, the data receiving device is not configured for wireless communication.

According to other aspects of the disclosed subject matter, systems and methods include a glucose monitoring system. The glucose monitoring system includes one or more processors and a memory in communication with the one or more processors that stores instructions that, when executed by the one or more processors, are configured to cause the one or more processors to perform operations. The operations can include establishing a communication session between the glucose monitoring system and an electronic medical records (EMR) system. The operations can include identifying therapy information associated with a user and stored in the EMR system. The operations can include causing the therapy information associated with the user to be transferred from the EMR system to the application server. The operations can include converting the therapy information for use by the glucose monitoring system. The operations can include storing the converted therapy information in a database accessible to the glucose monitoring system. The operations can include causing the therapy information to be displayed for a user of the glucose monitoring system. In some examples, the therapy information is stored a first structured format and converting the therapy information includes mapping the first structure format to a second structured format associated with glucose monitoring system. In some examples, the therapy information is stored in a free text format and converting the therapy information includes interpreting the therapy information using a natural language processing and storing the information in a structured format associated with the glucose monitoring system. In some examples, the operations include receiving additional therapy information through a structured format input, converting the additional therapy information for use by the EMR system, and transferring the converted additional therapy information to the EMR system for storage.

According to other aspects of the disclosed subject matter, systems and methods include a method of receiving and storing therapy information for use by the glucose monitoring system. The method can include establishing a communication session between the glucose monitoring system and an electronic medical records (EMR) system. The method can include identifying therapy information associated with a user and stored in the EMR system. The method can include causing the therapy information associated with the user to be transferred from the EMR system to the application server. The method can include converting the therapy information for use by the glucose monitoring system. The method can include storing the converted therapy information in a database accessible to the glucose monitoring system. The method can include causing the therapy information to be displayed for a user of the glucose monitoring system. In some examples, the therapy information is stored a first structured format and converting the therapy information includes mapping the first structure format to a second structured format associated with glucose monitoring system. In some examples, the therapy information is stored in a free text format and converting the therapy information includes interpreting the therapy information using a natural language processing and storing the information in a structured format associated with the glucose monitoring system. In some examples, the method include receiving additional therapy information through a structured format input, converting the additional therapy information for use by the EMR system, and transferring the converted additional therapy information to the EMR system for storage.

According to other aspects of the disclosed subject matter, systems and methods include a glucose monitoring system. The glucose monitoring system includes one or more processors and a memory in communication with the one or more processors that stores instructions that, when executed by the one or more processors, are configured to cause the one or more processors to perform operations. The operations can include establishing a communication session between the glucose monitoring system and an electronic medical records (EMR) system. The operations can include receiving a request from a user device for the EMR system to access glucose data associated with a patient and stored in a database accessible to the glucose monitoring system. The operations can include causing a multi-purpose device associated with the glucose monitoring system and the patient to display an access code associated with the glucose data associated with the patient. The operations can include receiving a second access code from the user device. The access code associated with the glucose data associated with the patient may be referred to as a first access code and the access code from the user device may be referred to as a second access code herein. The operations can include comparing the access code displayed by the multi-purpose device and the second access code. The operations can include, in response to determining that the access code displayed by the multi-purpose device and the second access code are equivalent, granting access to the EMR system to access the glucose data associated with the patient. In some examples, the access code is generated by the multi-purpose device and the glucose monitoring system receives the access code from the multi-purpose device. In some examples, the access code is generated by the glucose monitoring system and the multi-purpose device receives the access code from the glucose monitoring system. In some examples, the operations include causing the multi-purpose device to display an authorization request and the EMR system is granted access in response to an affirmative response to the authorization request. In some examples, one or more of the access code and the second access code includes a matrix barcode.

According to other aspects of the disclosed subject matter, systems and methods include a method of receiving and storing therapy information for use by the glucose monitoring system. The method can include establishing a communication session between the glucose monitoring system and an electronic medical records (EMR) system. The method can include receiving a request from a user device for the EMR system to access glucose data associated with a patient and stored in a database accessible to the glucose monitoring system. The method can include causing a multi-purpose device associated with the glucose monitoring system and the patient to display an access code associated with the glucose data associated with the patient. The method can include receiving a second access code from the user device. The method can include comparing the access code displayed by the multi-purpose device and the second access code. The method can include, in response to determining that the access code displayed by the multi-purpose device and the second access code are equivalent, granting access to the EMR system to access the glucose data associated with the patient. In some examples, the access code is generated by the multi-purpose device and the glucose monitoring system receives the access code from the multi-purpose device. In some examples, the access code is generated by the glucose monitoring system and the multi-purpose device receives the access code from the glucose monitoring system. In some examples, the method include causing the multi-purpose device to display an authorization request and the EMR system is granted access in response to an affirmative response to the authorization request. In some examples, one or more of the access code and the second access code includes a matrix barcode.

Other systems, methods, features and advantages of the subject matter described herein will be or will become apparent to one with skill in the art upon examination of the following figures and detailed description. It is intended that all such additional systems, methods, features and advantages be included within this description, be within the scope of the subject matter described herein, and be protected by the accompanying claims. In no way should the features of the examples be construed as limiting the appended claims, absent express recitation of those features in the claims.

### BRIEF DESCRIPTION OF THE FIGURES

The details of the subject matter set forth herein, both as to its structure and operation, may be apparent by study of the accompanying figures, in which like reference numerals refer to like parts. The components in the figures are not necessarily to scale, emphasis instead being placed upon illustrating the principles of the subject matter. Moreover, all illustrations are intended to convey concepts, where relative sizes, shapes and other detailed attributes may be illustrated schematically rather than literally or precisely.
FIG. 1A is a system overview of a sensor applicator, reader device, monitoring system, network, and remote system.
FIG. 1B is a diagram illustrating an operating environment of an example analyte monitoring system for use with the techniques described herein.
FIG. 2A is a block diagram depicting an example embodiment of a reader device.
FIG. 2B is a block diagram illustrating an example data receiving device for communicating with the sensor according to exemplary embodiments of the disclosed subject matter.
FIGS. 2C and 2D are block diagrams depicting example embodiments of sensor control devices.
FIG. 2E is a block diagram illustrating an example analyte sensor according to exemplary embodiments of the disclosed subject matter.
FIG. 3A is a proximal perspective view depicting an example embodiment of a user preparing a tray for an assembly.
FIG. 3B is a side view depicting an example embodiment of a user preparing an applicator device for an assembly.
FIG. 3C is a proximal perspective view depicting an example embodiment of a user inserting an applicator device into a tray during an assembly.
FIG. 3D is a proximal perspective view depicting an example embodiment of a user removing an applicator device from a tray during an assembly.
FIG. 3E is a proximal perspective view depicting an example embodiment of a patient applying a sensor using an applicator device.
FIG. 3F is a proximal perspective view depicting an example embodiment of a patient with an applied sensor and a used applicator device.
FIG. 4A is a side view depicting an example embodiment of an applicator device coupled with a cap.
FIG. 4B is a side perspective view depicting an example embodiment of an applicator device and cap decoupled.
FIG. 4C is a perspective view depicting an example embodiment of a distal end of an applicator device and electronics housing.
FIG. 4D is a top perspective view of an exemplary applicator device in accordance with the disclosed subject matter.
FIG. 4E is a bottom perspective view of the applicator device of FIG. 4D.
FIG. 4F is an exploded view of the applicator device of FIG. 4D.
FIG. 4G is a side cutaway view of the applicator device of FIG. 4D.
FIG. 5 is a proximal perspective view depicting an example embodiment of a tray with sterilization lid coupled.
FIG. 6A is a proximal perspective cutaway view depicting an example embodiment of a tray with sensor delivery components.
FIG. 6B is a proximal perspective view depicting sensor delivery components.
FIGS. 7A and 7B are isometric exploded top and bottom views, respectively, of an exemplary sensor control device.
FIG. 8A-8C are assembly and cross-sectional views of an on-body device including an integrated connector for the sensor assembly.
FIGS. 9A and 9B are side and cross-sectional side views, respectively, of an example embodiment of the sensor applicator of FIG. 1A with the cap of FIG. 2C coupled thereto.
FIGS. 10A and 10B are isometric and side views, respectively, of another example sensor control device.
FIGS. 11A-11C are progressive cross-sectional side views showing assembly of the sensor applicator with the sensor control device of FIGS. 10A-10B.
FIGS. 12A-12C are progressive cross-sectional side views showing assembly and disassembly of an example embodiment of the sensor applicator with the sensor control device of FIGS. 10A-10B.
FIGS. 13A-13F illustrate cross-sectional views depicting an example embodiment of an applicator during a stage of deployment.
FIG. 14 is a graph depicting an example of an in vitro sensitivity of an analyte sensor.
FIG. 15 is a diagram illustrating example operational states of the sensor according to exemplary embodiments of the disclosed subject matter.
FIG. 16 is a diagram illustrating an example operational and data flow for over-the-air programming of a sensor according to the disclosed subject matter.
FIG. 17 is a diagram illustrating an example data flow for secure exchange of data between two devices according to the disclosed subject matter.
FIG. 18 illustrates an example environment and dataflow for an analyte monitoring system.
FIG. 19 illustrates an example dataflow for an analyte monitoring system.
FIG. 20 illustrates example user interfaces of an application executing according to certain embodiments.
FIG. 21 illustrates example user interfaces of another device according to certain embodiments.
FIG. 22 illustrates a procedure for requesting and generating a report based on analyte data provided via a matrix barcode according to certain embodiments.
FIG. 23 illustrates a procedure for requesting and generating a report based on analyte data provided via a matrix barcode according to certain embodiments.
FIG. 24 illustrates example interfaces of an application executing according to certain embodiments.
FIG. 25 illustrates example interface of an application executing according to certain embodiments.
FIG. 26 illustrates an example flow of data between the components of an analyte monitoring system according to certain embodiments.
FIG. 27 illustrates an example interface of an application executing to certain embodiments.
FIG. 28 illustrates example interfaces of an application executing according to certain embodiments.
FIG. 29 illustrates an example interface of an application executing to certain embodiments.
FIG. 30 illustrates an example interface of an application executing to certain embodiments.
FIG. 31 illustrates an example dataflow for an analyte monitoring system.
FIG. 32 illustrates a procedure for requesting and generating a report according to certain embodiments.
FIG. 33 illustrates an example dataflow for an analyte monitoring system.
FIG. 34 illustrates a procedure for requesting and generating a report according to certain embodiments.
FIG. 35 illustrates an example dataflow for an analyte monitoring system.
FIG. 36 illustrates a procedure for requesting and generating a report according to certain embodiments.
FIG. 37 illustrates an example dataflow for an analyte monitoring system.
FIGS. 38A-38B illustrate a procedure for requesting and generating a report according to certain embodiments.
FIG. 39 illustrates an example dataflow for an analyte monitoring system.
FIGS. 40A-40B illustrate a procedure for requesting and generating a report according to certain embodiments.
FIG. 41 illustrates an example dataflow for an analyte monitoring system.
FIG. 42 illustrates a procedure for requesting and generating a report according to certain embodiments.
FIG. 43 illustrates an example dataflow for an analyte monitoring system.
FIG. 44 illustrates a procedure for requesting and generating a report according to certain embodiments.

### DETAILED DESCRIPTION

The present disclosure provides systems, devices, and methods for the use of analyte monitoring systems. An example analyte monitoring system can be configured to use various methods and additional systems to facilitate the transfer of analyte data from a data receiving device without wireless network capabilities. In certain examples, the analyte is glucose. Certain examples facilitate the transfer of data to multi-purpose devices, user devices, and electronic medical records (EMR) systems. Certain examples facilitate the transfer of data to report generating systems that interpret the provided data and reports based on the analyte data. Certain examples relate to techniques for facilitating the integration of EMR system data management and reporting.

The present disclosure provides systems, devices, and methods for the use of analyte sensor insertion applicators for use with *in vivo* analyte monitoring systems. An applicator can be provided to the user in a sterile package with an electronics housing of the sensor control device contained therein. According to some examples, a structure separate from the applicator, such as a container, can also be provided to the user as a sterile package with a sensor module and a sharp module contained therein. The user can couple the sensor module to the electronics housing, and can couple the sharp to the applicator with an assembly process that involves the insertion of the applicator into the container in a specified manner. In other examples, the applicator, sensor control device, sensor module, and sharp module can be provided in a single package. The applicator can be used to position the sensor control device on a human body with a sensor in contact with the wearer's bodily fluid. The examples provided herein are improvements to reduce the likelihood that a sensor is improperly inserted or damaged or elicit an adverse physiological response.

Furthermore, many examples include *in vivo* analyte sensors structurally configured so that at least a portion of the sensor is, or can be, positioned in the body of a user to obtain information about at least one analyte of the body. It should be noted, however, that the examples disclosed herein can be used with *in vivo* analyte monitoring systems that incorporate *in vitro* capability, as well as purely *in vitro* or *ex vivo* analyte monitoring systems, including systems that are entirely non-invasive.

Furthermore, for each and every example of a method or procedure disclosed herein, systems and devices capable of performing each of those examples are covered within the scope of the present disclosure. For example, examples of sensor control devices are disclosed and these devices can have one or more sensors, analyte monitoring circuits (e.g., an analog circuit), memories (e.g., for storing instructions), power sources, communication circuits, transmitters, receivers, processors and/or controllers (e.g., for executing instructions) that can perform any and all procedures or facilitate the execution thereof. These sensor control device examples can be used and can be capable of use to implement those procedures performed by a sensor control device from any of the methods described herein.

Furthermore, the systems and methods presented herein can be used for operations of a sensor used in an analyte monitoring system, such as but not limited to wellness, fitness, dietary, research, information or any purposes involving analyte sensing over time. As used herein, "sensor" can refer to any device capable of receiving sensor information from a user, including for purpose of illustration but not limited to, body temperature sensors, blood pressure sensors, pulse or heart-rate sensors, glucose level sensors, analyte sensors, physical activity sensors, body movement sensors, or any other sensors for collecting physical or biological information. Analytes measured by the analyte sensors can include, by way of example and not limitation, glucose, ketones, lactate, oxygen, hemoglobin A1C, albumin, alcohol, alkaline phosphatase, alanine transaminase, aspartate aminotransferase, bilirubin, blood urea nitrogen, calcium, carbon dioxide, chloride, creatinine, hematocrit, lactate, magnesium, oxygen, pH, phosphorus, potassium, sodium, total protein, uric acid, etc.

Before describing these aspects of the embodiments in detail, however, it is first desirable to describe examples of devices that can be present within, for example, an *in vivo* analyte monitoring system, as well as examples of their operation, all of which can be used with the examples described herein.

There are various types of *in vivo* analyte monitoring systems. "Continuous Analyte Monitoring" systems (or "Continuous Glucose Monitoring" systems), for example, can transmit data from a sensor control device to a reader device continuously without prompting, e.g., automatically according to a schedule. "Flash Analyte Monitoring" systems (or "Flash Glucose Monitoring" systems or simply "Flash" systems), as another example, can transfer data from a sensor control device in response to a scan or request for data by a reader device, such as with a Near Field Communication (NFC) or Radio Frequency Identification (RFID) protocol. *In vivo* analyte monitoring systems can also operate without the need for finger stick calibration.

*In vivo* analyte monitoring systems can be differentiated from "*in vitro*" systems that contact a biological sample outside of the body (or "*ex vivo*") and that typically include a meter device that has a port for receiving an analyte test strip carrying bodily fluid of the user, which can be analyzed to determine the user's blood sugar level.

*In vivo* monitoring systems can include a sensor that, while positioned *in vivo*, makes contact with the bodily fluid of the user and senses the analyte levels contained therein. The sensor can be part of the sensor control device that resides on the body of the user and contains the electronics and power supply that enable and control the analyte sensing. The sensor control device, and variations thereof, can also be referred to as a "sensor control unit," an "on-body electronics" device or unit, an "on-body" device or unit, or a "sensor data communication" device or unit, to name a few.

*In vivo* monitoring systems can also include a device that receives sensed analyte data from the sensor control device and processes and/or displays that sensed analyte data, in any number of forms, to the user. This device, and variations thereof, can be referred to as a "handheld reader device," "reader device" (or simply a "reader"), "handheld electronics" (or simply a "handheld"), a "portable data processing" device or unit, a "data receiver," a "receiver" device or unit (or simply a "receiver"), a "remote" device or unit, a "receiving device," a "data receiving device," or "receiver device" to name a few. Unless specifically indicated otherwise, this description uses these terms interchangeably. Other devices such as personal computers have also been utilized with or incorporated into *in vivo* and *in vitro* monitoring systems.

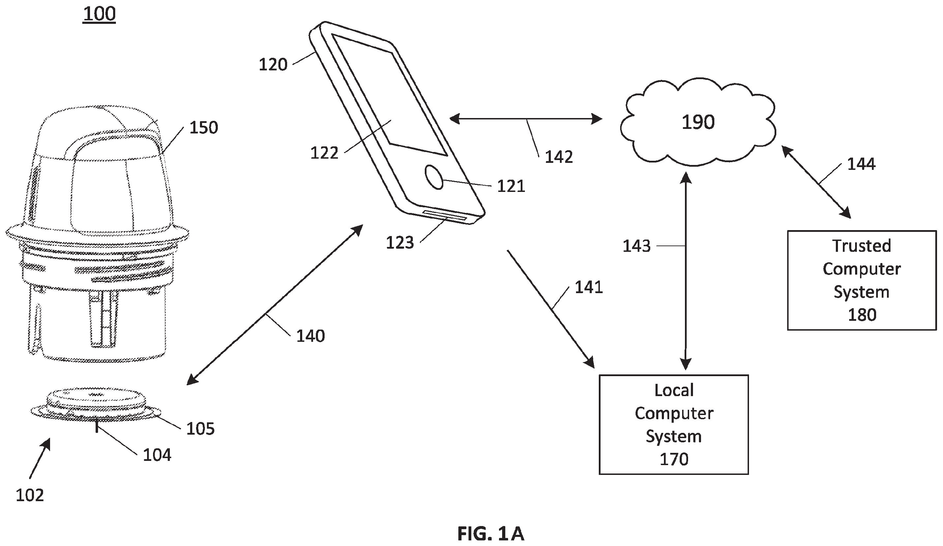

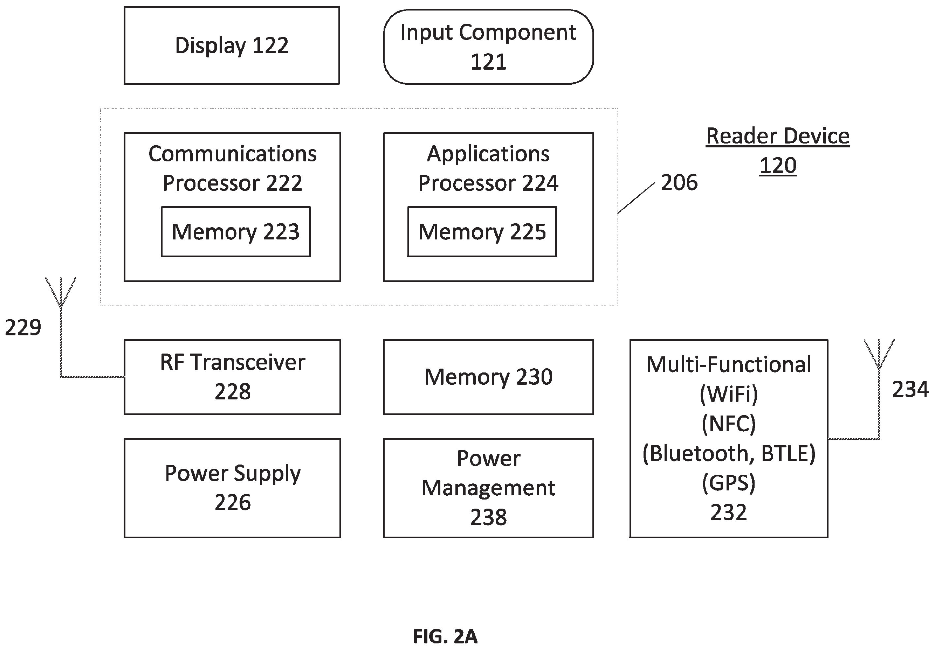

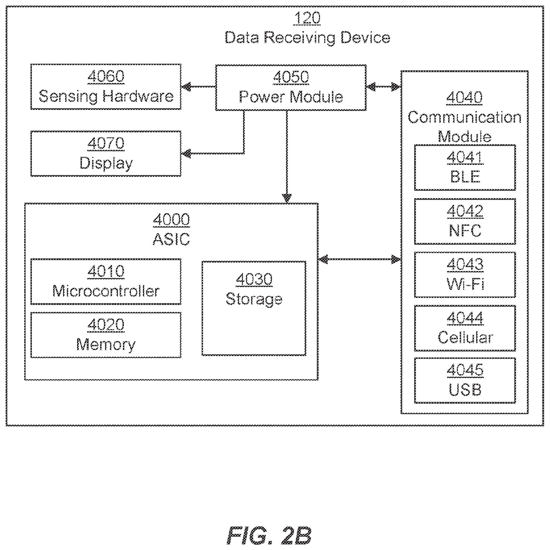

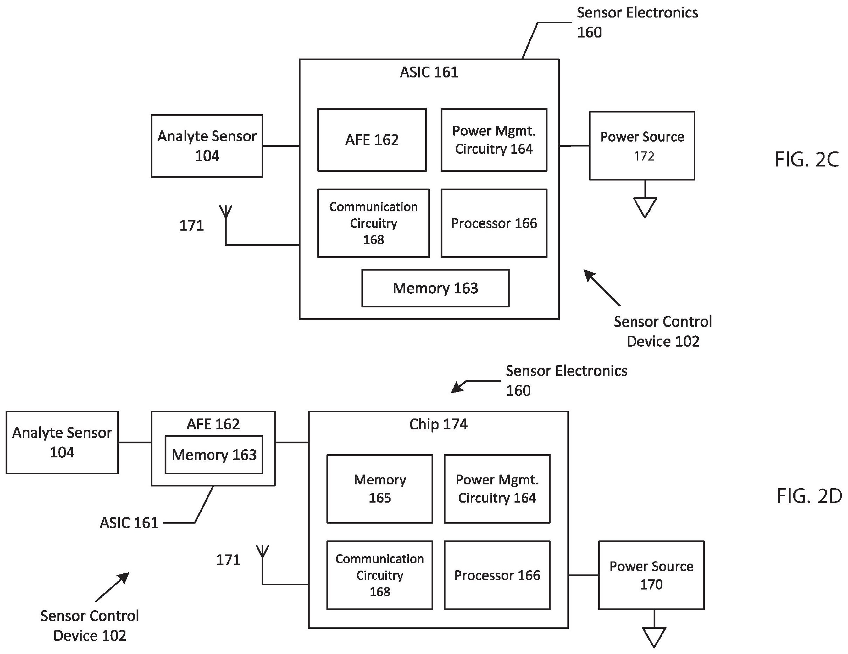

FIG. 1A is a conceptual diagram depicting an example of an analyte monitoring system 100 that includes a sensor applicator 150, a sensor control device 102, and a data receiving device 120 (e.g., a "receiving device"). Here, sensor applicator 150 can be used to deliver sensor control device 102 to a monitoring location on a user's skin where a sensor 104 is maintained in position for a period of time by an adhesive patch 105. Sensor control device 102 is further described in FIGS. 2B and 2C, and can communicate with data receiving device 120 via a communication path 140 using a wired or wireless technique. Example wireless protocols include Bluetooth, Bluetooth Low Energy (BLE, BTLE, Bluetooth SMART, etc.), Near Field Communication (NFC) and others. Users can monitor applications installed in memory on data receiving device 120 using screen 122 and input 121 and the device battery can be recharged using power port 123. More detail about data receiving device 120 is set forth with respect to FIG. 2A below. Data receiving device 120 can communicate with local computer system 170 via a communication path 141 using a wired or wireless technique. Local computer system 170 can include one or more of a laptop, desktop, tablet, phablet, smartphone, set-top box, video game console, or other computing device and wireless communication can include any of a number of applicable wireless networking protocols including Bluetooth, Bluetooth Low Energy (BTLE), Wi-Fi or others. Local computer system 170 can communicate via communications path 143 with a network 190 similar to how data receiving device 120 can communicate via a communications path 142 with network 190, by wired or wireless technique as described previously. Network 190 can be any of a number of networks, such as private networks and public networks, local area or wide area networks, and so forth. A trusted computer system 180 can include a server and can provide authentication services and secured data storage and can communicate via communications path 144 with network 190 by wired or wireless technique.

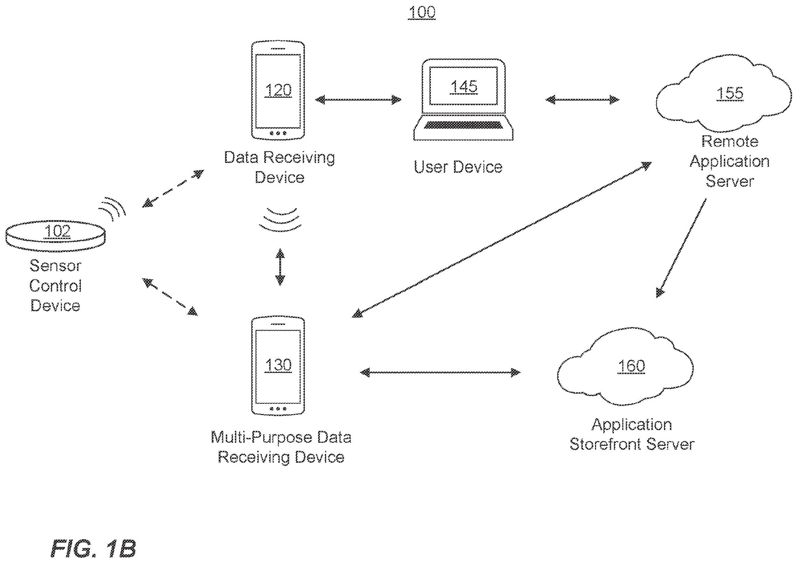

FIG. 1B illustrates another example of an operating environment of an analyte monitoring system 100 capable of embodying the techniques described herein. As illustrated, the analyte monitoring system 100 can include a system of components designed to provide monitoring of parameters, such as analyte levels, of a human or animal body or can provide for other operations based on the configurations of the various components. As embodied herein, the system can include a low-power sensor control device 102 worn by the user or attached to the body for which information is being collected. As embodied herein, the sensor control device 102 can be a sealed, disposable device with a predetermined active use lifetime (e.g., 1 day, 14 days, 30 days, etc.). Sensors 110 can be applied to the skin of the user body and remain adhered over the duration of the sensor lifetime or can be designed to be selectively removed and remain functional when reapplied. The low-power analyte monitoring system 100 can further include a data reading device 120 or multi-purpose device 130 configured as described herein to facilitate retrieval and delivery of data, including analyte data, from the sensor control device 102.

As embodied herein, the analyte monitoring system 100 can include a software or firmware library or application provided, for example via a remote application server 155 or application storefront server 160, to a third-party and incorporated into a multi-purpose hardware device 130 such as a mobile phone, tablet, personal computing device, or other similar computing device capable of communicating with the sensor control device 102 over a communication link. Multi-purpose hardware can further include embedded devices, including, but not limited to insulin pumps or insulin pens, having an embedded library configured to communicate with the sensor control device 102. Although the illustrated examples of the analyte monitoring system 100 include only one of each of the illustrated devices, this disclosure contemplates the analyte monitoring system 100 incorporate multiples of each components interacting throughout the system. For example and without limitation, as embodied herein, data receiving device 120 and/or multi-purpose device 130 can include multiples of each. As embodied herein, multiple data receiving devices 130 can communicate directly with sensor control device 102 as described herein. Additionally or alternatively, a data receiving device 130 can communicate with secondary data receiving devices 130 to provide analyte data, or visualization or analysis of the data, for secondary display to the user or other authorized parties.

FIG. 2A is a block diagram depicting an example of a data receiving device 120 configured as a smartphone. Here, data receiving device 120 can include a display 122, input component 121, and a processing core 206 including a communications processor 222 coupled with memory 223 and an applications processor 224 coupled with memory 225. Also included can be separate memory 230, RF transceiver 228 with antenna 229, and power supply 226 with power management module 238. Further included can be a multi-functional transceiver 232 which can communicate over Wi-Fi, NFC, Bluetooth, BTLE, and GPS with an antenna 234. As understood by one of skill in the art, these components are electrically and communicatively coupled in a manner to make a functional device. In some examples, where the data receiving device 120 is configured as a smartphone with other functions, the data receiving device 120 can be referred to as a "multi-purpose device."

Data receiving device 120 can be a mobile communication device such as, for example, a Wi-Fi or internet enabled smartphone, tablet, or personal digital assistant (PDA). Examples of smartphones can include, but are not limited to, those phones based on a WINDOWS operating system, ANDROID operating system, IPHONE operating system, PALM, WEBOS, BLACKBERRY operating system, or SYMBIAN operating system, with data network connectivity functionality for data communication over an internet connection and/or a local area network (LAN).

Data receiving device 120 can also be configured as a mobile smart wearable electronics assembly, such as an optical assembly that is worn over or adjacent to the user's eye (e.g., a smart glass or smart glasses, such as GOOGLE GLASSES). This optical assembly can have a transparent display that displays information about the user's analyte level (as described herein) to the user while at the same time allowing the user to see through the display such that the user's overall vision is minimally obstructed. The optical assembly can be capable of wireless communications similar to a smartphone. Other examples of wearable electronics include devices that are worn around or in the proximity of the user's wrist (e.g., a smart watch, etc.), neck (e.g., a necklace, etc.), head (e.g., a headband, hat, etc.), chest, or the like.

For purpose of illustration and not limitation, reference is made to another example of a data receiving device 120 for use with the disclosed subject matter as shown in FIG. 2B. The data receiving device 120, and the related multi-purpose device 130, includes components germane to the discussion of the sensor control device 102 and its operations and additional components can be included. In some examples, the data receiving device 120 and multi-purpose device 130 can be or include components provided by a third party and are not necessarily restricted to include devices made by the same manufacturer as the sensor control device 102.

As illustrated in FIG. 2B, the data receiving device 120 includes an ASIC 4000 including a microcontroller 4010, memory 4020, and storage 4030 and communicatively coupled with a communication module 4040. Power for the components of the data receiving device 120 can be delivered by a power module 4050, which as embodied herein can include a rechargeable battery. The data receiving device 120 can further include a display 4070 for facilitating review of analyte data received from an sensor control device 102 or other device (e.g., user device 145 or remote application server 155). The data receiving device 120 can include separate user interface components (e.g., physical keys, light sensors, microphones, etc.).

The communication module 4040 can include a BLE module 4041 and an NFC module 4042. The data receiving device 120 can be configured to wirelessly couple with the sensor control device 102 and transmit commands to and receive data from the sensor control device 102. As embodied herein, the data receiving device 120 can be configured to operate, with respect to the sensor control device 102 as described herein, as an NFC scanner and a BLE end point via specific modules (e.g., BLE module 4042 or NFC module 4043) of the communication module 4040. For example, the data receiving device 120 can issue commands (e.g., activation commands for a data broadcast mode of the sensor; pairing commands to identify the data receiving device 120) to the sensor control device 102 using a first module of the communication module 4040 and receive data from and transmit data to the sensor control device 102 using a second module of the communication module 4040. The data receiving device 120 can be configured for communication with a user device 145 via a Universal Serial Bus (USB) module 4045 of the communication module 4040.

As another example, the communication module 4040 can include, for example, a cellular radio module 4044. The cellular radio module 4044 can include one or more radio transceivers for communicating using broadband cellular networks, including, but not limited to third generation (3G), fourth generation (4G), and fifth generation (5G) networks. Additionally, the communication module 4040 of the data receiving device 120 can include a Wi-Fi radio module 4043 for communication using a wireless local area network according to one or more of the IEEE 802.11 standards (e.g., 802.11a, 802.11b, 802.11g, 802.11n (aka Wi-Fi 4), 802.11ac (aka Wi-Fi 5), 802.11ax (aka Wi-Fi 6)). Using the cellular radio module 4044 or Wi-Fi radio module 4043, the data receiving device 120 can communicate with the remote application server 155 to receive analyte data or provide updates or input received from a user (e.g., through one or more user interfaces). Although not illustrated, the communication module 5040 of the analyte sensor 120 can similarly include a cellular radio module or Wi-Fi radio module.

As embodied herein, the on-board storage 4030 of the data receiving device 120 can store analyte data received from the sensor control device 102. Further, the data receiving device 120, multi-purpose device 130, or a user device 145 can be configured to communicate with a remote application server 155 via a wide area network. As embodied herein, the sensor control device 102 can provide data to the data receiving device 120 or multi-purpose device 130. The data receiving device 120 can transmit the data to the user computing device 145. The user computing device 145 (or the multi-purpose device 130) can in turn transmit that data to a remote application server 155 for processing and analysis.

As embodied herein, the data receiving device 120 can further include sensing hardware 4060 similar to, or expanded from, the sensing hardware 5060 of the sensor control device 102. In some examples, the data receiving device 120 can be configured to operate in coordination with the sensor control device 102 and based on analyte data received from the sensor control device 102. As an example, where the sensor control device 102 glucose sensor, the data receiving device 120 can be or include an insulin pump or insulin injection pen. In coordination, the compatible device 130 can adjust an insulin dosage for a user based on glucose values received from the analyte sensor.

FIGS. 2C and 2D are block diagrams depicting examples of sensor control device 102 having analyte sensor 104 and sensor electronics 160 (including analyte monitoring circuitry) that can have the majority of the processing capability for rendering end-result data suitable for display to the user. In FIG. 2C, a single semiconductor chip 161 is depicted that can be a custom application specific integrated circuit (ASIC). Shown within ASIC 161 are certain high-level functional units, including an analog front end (AFE) 162, power management (or control) circuitry 164, processor 166, and communication circuitry 168 (which can be implemented as a transmitter, receiver, transceiver, passive circuit, or otherwise according to the communication protocol). In this example, both AFE 162 and processor 166 are used as analyte monitoring circuitry, but in other examples either circuit can perform the analyte monitoring function. Processor 166 can include one or more processors, microprocessors, controllers, and/or microcontrollers, each of which can be a discrete chip or distributed amongst (and a portion of) a number of different chips.

A memory 163 is also included within ASIC 161 and can be shared by the various functional units present within ASIC 161, or can be distributed amongst two or more of them. Memory 163 can also be a separate chip. Memory 163 can be volatile and/or nonvolatile memory. In this example, ASIC 161 is coupled with power source 172, which can be a coin cell battery, or the like. AFE 162 interfaces with *in vivo* analyte sensor 104 and receives measurement data therefrom and outputs the data to processor 166 in digital form, which in turn processes the data to arrive at the end-result glucose discrete and trend values, etc. This data can then be provided to communication circuitry 168 for sending, by way of antenna 171, to data receiving device 120 (not shown), for example, where minimal further processing is needed by the resident software application to display the data.

FIG. 2D is similar to FIG. 2C but instead includes two discrete semiconductor chips 162 and 174, which can be packaged together or separately. Here, AFE 162 is resident on ASIC 161. Processor 166 is integrated with power management circuitry 164 and communication circuitry 168 on chip 174. AFE 162 includes memory 163 and chip 174 includes memory 165, which can be isolated or distributed within. In one example, AFE 162 is combined with power management circuitry 164 and processor 166 on one chip, while communication circuitry 168 is on a separate chip. In another example, both AFE 162 and communication circuitry 168 are on one chip, and processor 166 and power management circuitry 164 are on another chip. It should be noted that other chip combinations are possible, including three or more chips, each bearing responsibility for the separate functions described, or sharing one or more functions for fail-safe redundancy.

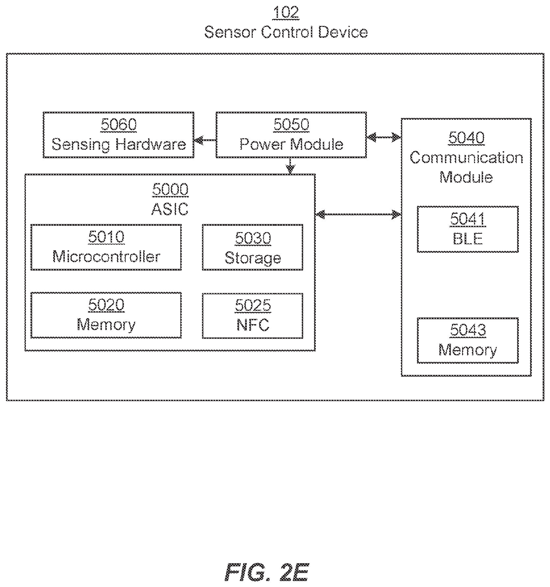

For purpose of illustration and not limitation, FIG. 2E depicts another example of a sensor control device 102 compatible with the security architecture and communication schemes described herein.

As embodied herein, the sensor control device 102 can include an Application-Specific Integrated Circuit ("ASIC") 5000 communicatively coupled with a communication module 5040. The ASIC 5000 can include a microcontroller core 5010, on-board memory 5020, and storage memory 5030. The storage memory 5030 can store data used in an authentication and encryption security architecture. The storage memory 5030 can store programming instructions for sensor control device 102. As embodied herein, certain communication chipsets can be embedded in the ASIC 5000 (e.g., an NFC transceiver 5025). The ASIC 5000 can receive power from a power module 5050, such as an on-board battery or from an NFC pulse. The storage memory 5030 of the ASIC 5000 can be programmed to include information such as an identifier for sensor control device 102 for identification and tracking purposes. The storage memory 5030 can also be programmed with configuration or calibration parameters for use by sensor control device 102 and its various components. The storage memory 5030 can include rewritable or one-time programming (OTP) memory. The storage memory 5030 can be updated using techniques described herein to extend the usefulness of sensor control device 102.

As embodied herein, the communication module 5040 of sensor control device 102 can be or include one or more modules to support communications with other devices of an analyte monitoring system 100. As an example only, and not by way of limitation, example communication modules 5040 can include a Bluetooth Low-Energy ("BLE") module 5041 As used throughout this disclosure, BLE refers to a short-range communication protocol optimized to make pairing of Bluetooth devices simple for end users. The communication module 5040 can transmit and receive data and commands via interaction with similarly-capable communication modules of a data receiving device 120 or user device 145. The communication module 5040 can include additional or alternative chipsets for use with similar short-range communication schemes, such as a personal area network according to IEEE 802.15 protocols, IEEE 802.11 protocols, infrared communications according to the Infrared Data Association standards (IrDA), etc.

To perform its functionalities, the sensor control device 102 can further include suitable sensing hardware 5060 appropriate to its function. As embodied herein, the sensing hardware 5060 can include an analyte sensor transcutaneously or subcutaneously positioned in contact with a bodily fluid of a subject. The analyte sensor can generate sensor data containing values corresponding to levels of one or more analytes within the bodily fluid.

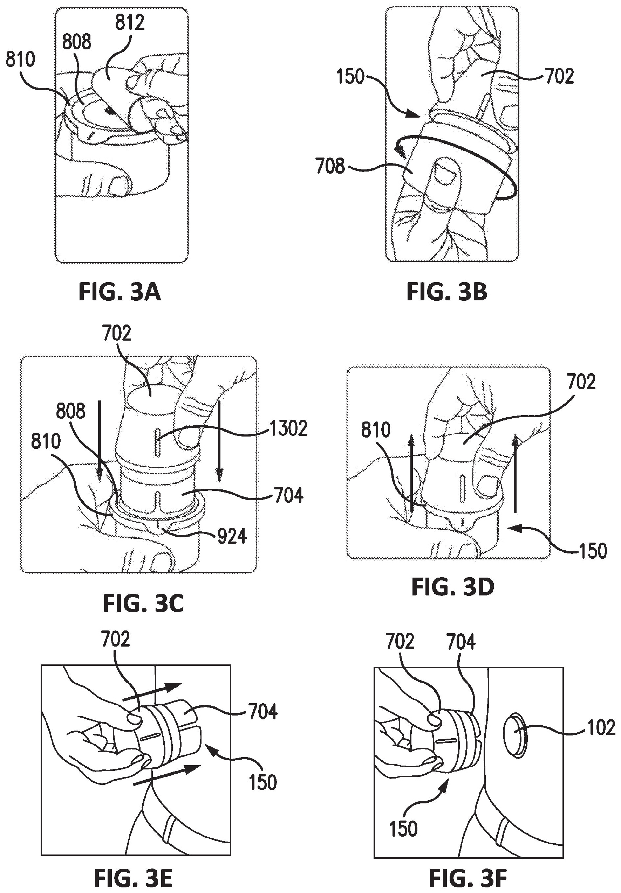

The components of sensor control device 102 can be acquired by a user in multiple packages requiring final assembly by the user before delivery to an appropriate user location. FIGS. 3A-3D depict an example of an assembly process for sensor control device 102 by a user, including preparation of separate components before coupling the components in order to ready the sensor for delivery. FIGS. 3E-3F depict an example of delivery of sensor control device 102 to an appropriate user location by selecting the appropriate delivery location and applying device 102 to the location.

FIG. 3A is a proximal perspective view depicting an example of a user preparing a container 810, configured here as a tray (although other packages can be used), for an assembly process. The user can accomplish this preparation by removing lid 812 from tray 810 to expose platform 808, for instance by peeling a non-adhered portion of lid 812 away from tray 810 such that adhered portions of lid 812 are removed. Removal of lid 812 can be appropriate in various examples so long as platform 808 is adequately exposed within tray 810. Lid 812 can then be placed aside.

FIG. 3B is a side view depicting an example of a user preparing an applicator device 150 for assembly. Applicator device 150 can be provided in a sterile package sealed by an applicator cap 708. Preparation of applicator device 150 can include uncoupling housing 702 from applicator cap 708 to expose sheath 704 (FIG. 3C). This can be accomplished by unscrewing (or otherwise uncoupling) applicator cap 708 from housing 702. Applicator cap 708 can then be placed aside.

FIG. 3C is a proximal perspective view depicting an example of a user inserting an applicator device 150 into a tray 810 during an assembly. Initially, the user can insert sheath 704 into platform 808 inside tray 810 after aligning housing orienting feature 1302 (or slot or recess) and tray orienting feature 924 (an abutment or detent). Inserting sheath 704 into platform 808 temporarily unlocks sheath 704 relative to housing 702 and also temporarily unlocks platform 808 relative to tray 810. At this stage, removal of applicator device 150 from tray 810 will result in the same state prior to initial insertion of applicator device 150 into tray 810 (i.e., the process can be reversed or aborted at this point and then repeated without consequence).

Sheath 704 can maintain position within platform 808 with respect to housing 702 while housing 702 is distally advanced, coupling with platform 808 to distally advance platform 808 with respect to tray 810. This unlocks and collapses platform 808 within tray 810. Sheath 704 can contact and disengage locking features (not shown) within tray 810 that unlock sheath 704 with respect to housing 702 and prevent sheath 704 from moving (relatively) while housing 702 continues to distally advance platform 808. At the end of advancement of housing 702 and platform 808, sheath 704 is permanently unlocked relative to housing 702. A sharp and sensor (not shown) within tray 810 can be coupled with an electronics housing (not shown) within housing 702 at the end of the distal advancement of housing 702. Operation and interaction of the applicator device 150 and tray 810 are further described below.

FIG. 3D is a proximal perspective view depicting an example of a user removing an applicator device 150 from a tray 810 during an assembly. A user can remove applicator 150 from tray 810 by proximally advancing housing 702 with respect to tray 810 or other motions having the same end effect of uncoupling applicator 150 and tray 810. The applicator device 150 is removed with sensor control device 102 (not shown) fully assembled (sharp, sensor, electronics) therein and positioned for delivery.

FIG. 3E is a proximal perspective view depicting an example of a patient applying sensor control device 102 using applicator device 150 to a target area of skin, for instance, on an abdomen or other appropriate location. Advancing housing 702 distally collapses sheath 704 within housing 702 and applies the sensor to the target location such that an adhesive layer on the bottom side of sensor control device 102 adheres to the skin. The sharp is automatically retracted when housing 702 is fully advanced, while the sensor (not shown) is left in position to measure analyte levels.

FIG. 3F is a proximal perspective view depicting an example of a patient with sensor control device 102 in an applied position. The user can then remove applicator 150 from the application site.

System 100, described with respect to FIGS. 3A-3F and elsewhere herein, can provide a reduced or eliminated chance of accidental breakage, permanent deformation, or incorrect assembly of applicator components compared to prior art systems. Since applicator housing 702 directly engages platform 808 while sheath 704 unlocks, rather than indirect engagement via sheath 704, relative angularity between sheath 704 and housing 702 will not result in breakage or permanent deformation of the arms or other components. The potential for relatively high forces (such as in conventional devices) during assembly will be reduced, which in turn reduces the chance of unsuccessful user assembly.

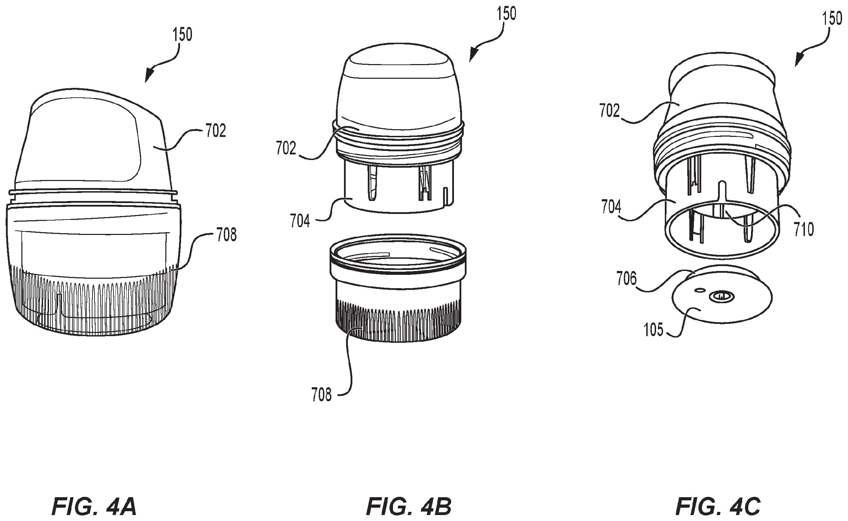

FIG. 4A is a side view depicting an example of an applicator device 150 coupled with a screw applicator cap 708. This is an example of how applicator 150 is shipped to and received by a user, prior to assembly by the user with a sensor. FIG. 4B is a side perspective view depicting applicator 150 and applicator cap 708 after being decoupled. FIG. 4C is a perspective view depicting an example of a distal end of an applicator device 150 with electronics housing 706 and adhesive patch 105 removed from the position they would have retained within sensor carrier 710 of sheath 704, when applicator cap 708 is in place.

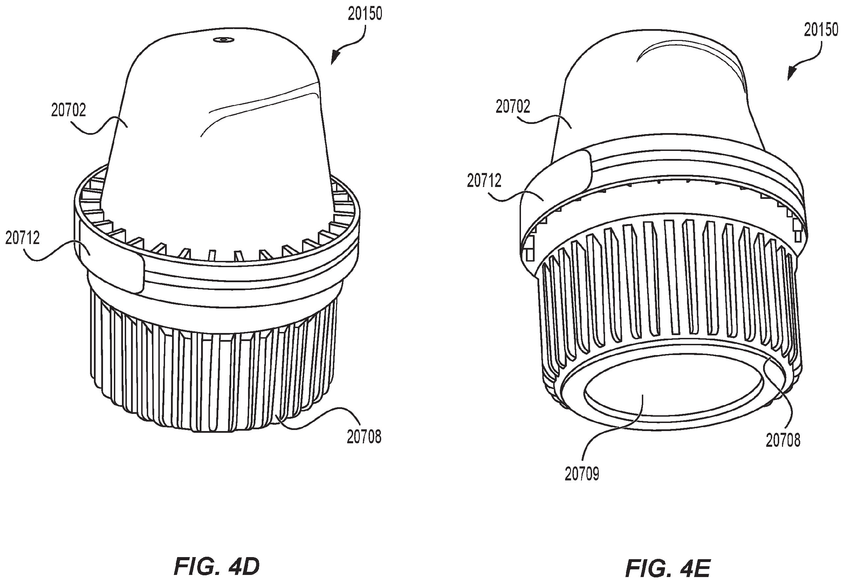

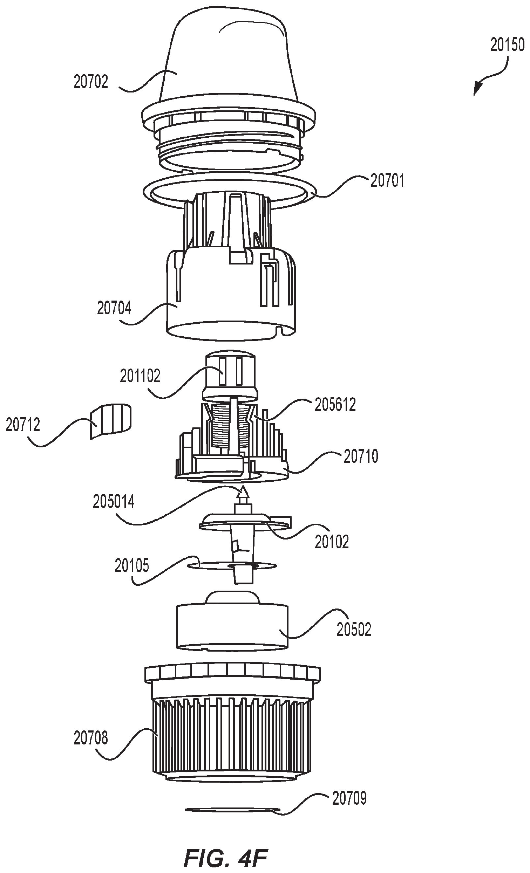

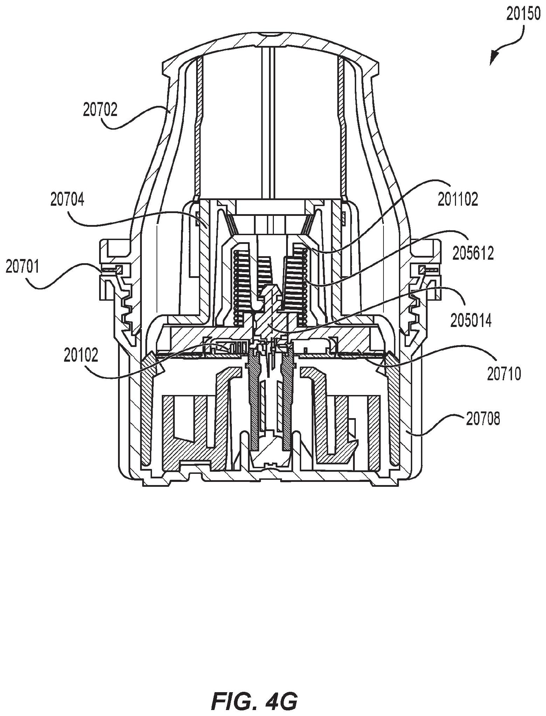

Referring to FIGs. 4D-G for purpose of illustration and not limitation, another example of an applicator device 20150 can be provided to a user as a single integrated assembly. FIGs. 4D and 4E provide perspective top and bottom views, respectively, of the applicator device 20150, FIG. 4F provides an exploded view of the applicator device 20150 and FIG. 4G provides a side cut-away view. The perspective views illustrate how applicator 20150 is shipped to and received by a user. The exploded and cut-away views illustrate the components of the applicator device 20150. The applicator device 20150 can include a housing 20702, gasket 20701, sheath 20704, sharp carrier 201102, spring 205612, sensor carrier 20710 (also referred to as a "puck carrier"), sharp hub 205014, sensor control device (also referred to as a "puck") 20102, adhesive patch 20105, desiccant 20502, applicator cap 20708, serial label 20709, and tamper evidence feature 20712. In some examples, as received by a user, only the housing 20702, applicator cap 20708, tamper evidence feature 20712, and label 20709 are visible. The tamper evidence feature 20712 can be, for example, a sticker coupled to each of the housing 20702 and the applicator cap 20708, and tamper evidence feature 20712 can be damaged, for example, irreparably, by uncoupling housing 20702 and applicator cap 20708, thereby indicating to a user that the housing 20702 and applicator cap 20708 have been previously uncoupled. These features are described in greater detail below.

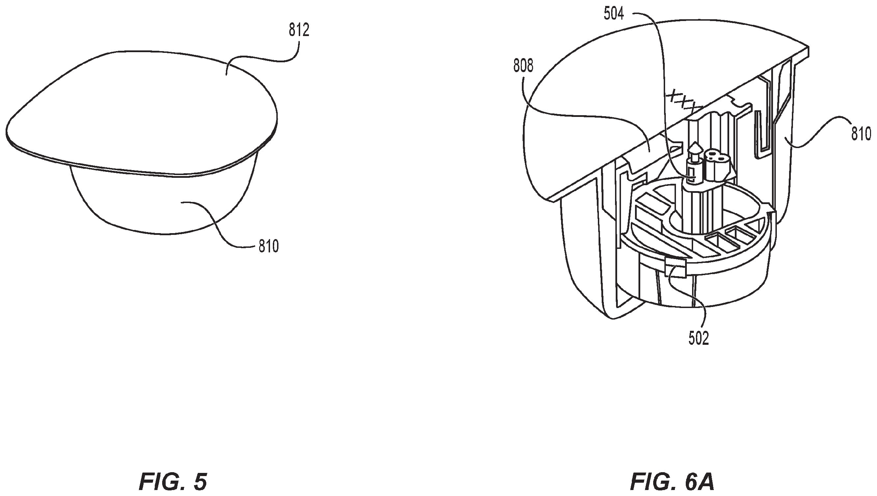

FIG. 5 is a proximal perspective view depicting an example of a tray 810 with sterilization lid 812 removably coupled thereto, which can be representative of how the package is shipped to and received by a user prior to assembly.

FIG. 6A is a proximal perspective cutaway view depicting sensor delivery components within tray 810. Platform 808 is slidably coupled within tray 810. Desiccant 502 is stationary with respect to tray 810. Sensor module 504 is mounted within tray 810.

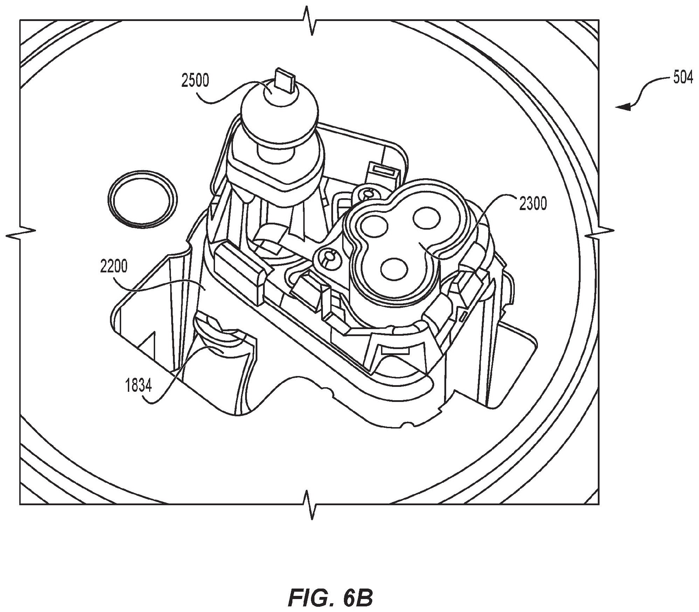

FIG. 6B is a proximal perspective view depicting sensor module 504 in greater detail. Here, retention arm extensions 1834 of platform 808 releasably secure sensor module 504 in position. Module 2200 is coupled with connector 2300, sharp module 2500 and sensor (not shown) such that during assembly they can be removed together as sensor module 504.

Referring briefly again to FIGS. 1A and 3A-3G, for the two-piece architecture system, the sensor tray 810 and the sensor applicator 150 are provided to the user as separate packages, thus requiring the user to open each package and finally assemble the system. In some applications, the discrete, sealed packages allow the sensor tray 810 and the sensor applicator 150 to be sterilized in separate sterilization processes unique to the contents of each package and otherwise incompatible with the contents of the other. More specifically, the sensor tray 810, which includes the plug assembly 207, including the sensor 104 and the sharp 220, can be sterilized using radiation sterilization, such as electron beam (or "e-beam") irradiation. Suitable radiation sterilization processes include, but are not limited to, e-beam irradiation, gamma ray irradiation, X-ray irradiation, or any combination thereof. Radiation sterilization, however, can damage the electrical components arranged within the electronics housing of the sensor control device 102. Consequently, if the sensor applicator 150, which contains the electronics housing of the sensor control device 102, needs to be sterilized, it can be sterilized via another method, such as gaseous chemical sterilization using, for example, ethylene oxide. Gaseous chemical sterilization, however, can damage the enzymes or other chemistry and biologies included on the sensor 104. Because of this sterilization incompatibility, the sensor tray 810 and the sensor applicator 150 are commonly sterilized in separate sterilization processes and subsequently packaged separately, which requires the user to finally assemble the components for use.

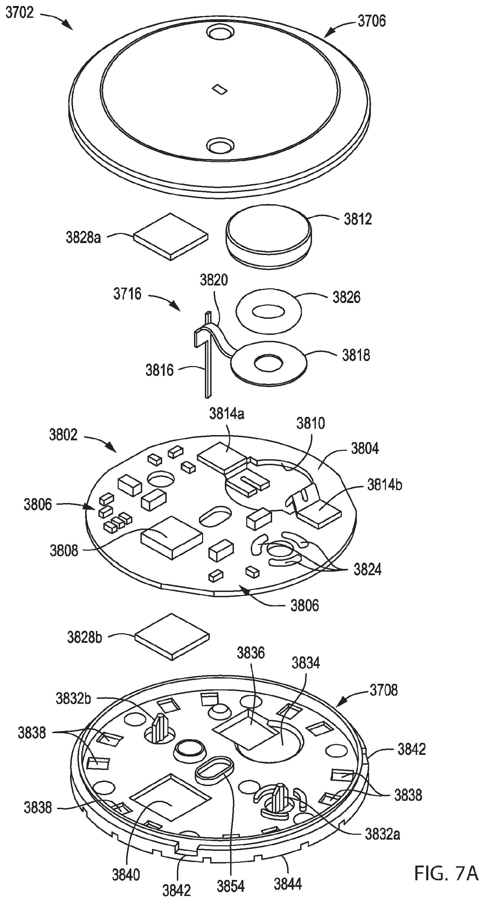

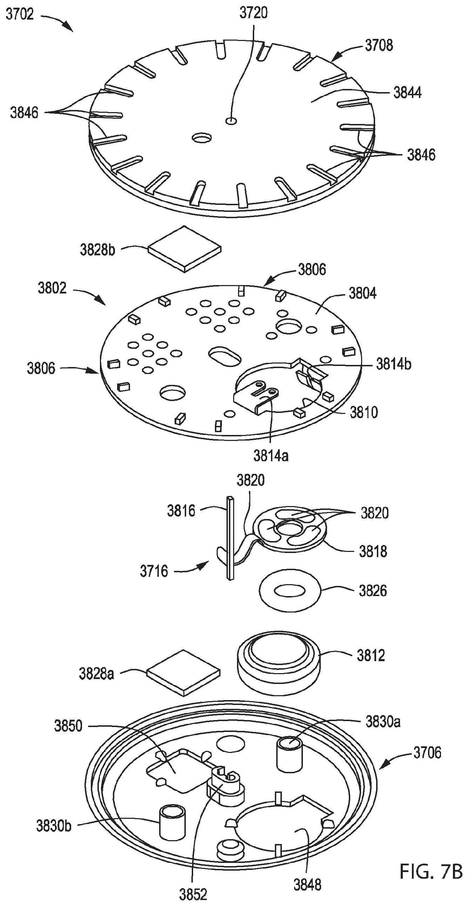

FIGS. 7A and 7B are exploded top and bottom views, respectively, of the sensor control device 3702, according to one or more examples. The shell 3706 and the mount 3708 operate as opposing clamshell halves that enclose or otherwise substantially encapsulate the various electronic components of the sensor control device 3702. As illustrated, the sensor control device 3702 can include a printed circuit board assembly (PCBA) 3802 that includes a printed circuit board (PCB) 3804 having a plurality of electronic modules 3806 coupled thereto. Example electronic modules 3806 include, but are not limited to, resistors, transistors, capacitors, inductors, diodes, and switches. Prior sensor control devices commonly stack PCB components on only one side of the PCB. In contrast, the PCB components 3806 in the sensor control device 3702 can be dispersed about the surface area of both sides (i.e., top and bottom surfaces) of the PCB 3804.

Besides the electronic modules 3806, the PCBA 3802 can also include a data processing unit 3808 mounted to the PCB 3804. The data processing unit 3808 can comprise, for example, an application specific integrated circuit (ASIC) configured to implement one or more functions or routines associated with operation of the sensor control device 3702. More specifically, the data processing unit 3808 can be configured to perform data processing functions, where such functions can include but are not limited to, filtering and encoding of data signals, each of which corresponds to a sampled analyte level of the user. The data processing unit 3808 can also include or otherwise communicate with an antenna for communicating with the reader device 106.

A battery aperture 3810 can be defined in the PCB 3804 and sized to receive and seat a battery 3812 configured to power the sensor control device 3702. An axial battery contact 3814a and a radial battery contact 3814b can be coupled to the PCB 3804 and extend into the battery aperture 3810 to facilitate transmission of electrical power from the battery 3812 to the PCB 3804. As their names suggest, the axial battery contact 3814a can be configured to provide an axial contact for the battery 3812, while the radial battery contact 3814b can provide a radial contact for the battery 3812. Locating the battery 3812 within the battery aperture 3810 with the battery contacts 3814a,b helps reduce the height H of the sensor control device 3702, which allows the PCB 3804 to be located centrally and its components to be dispersed on both sides (i.e., top and bottom surfaces). This also helps facilitate the chamfer 3718 provided on the electronics housing 3704.

The sensor 3716 can be centrally located relative to the PCB 3804 and include a tail 3816, a flag 3818, and a neck 3820 that interconnects the tail 3816 and the flag 3818. The tail 3816 can be configured to extend through the central aperture 3720 of the mount 3708 to be transcutaneously received beneath a user's skin. Moreover, the tail 3816 can have an enzyme or other chemistry included thereon to help facilitate analyte monitoring.

The flag 3818 can include a generally planar surface having one or more sensor contacts 3822 (three shown in FIG. 7B) arranged thereon. The sensor contact(s) 3822 can be configured to align with and engage a corresponding one or more circuitry contacts 3824 (three shown in FIG. 7A) provided on the PCB 3804. In some examples, the sensor contact(s) 3822 can comprise a carbon impregnated polymer printed or otherwise digitally applied to the flag 3818. Prior sensor control devices typically include a connector made of silicone rubber that encapsulates one or more compliant carbon impregnated polymer modules that serve as electrical conductive contacts between the sensor and the PCB. In contrast, the presently disclosed sensor contacts(s) 3822 provide a direct connection between the sensor 3716 and the PCB 3804 connection, which eliminates the need for the prior art connector and advantageously reduces the height H. Moreover, eliminating the compliant carbon impregnated polymer modules eliminates a significant circuit resistance and therefor improves circuit conductivity.

The sensor control device 3702 can further include a compliant member 3826, which can be arranged to interpose the flag 3818 and the inner surface of the shell 3706. More specifically, when the shell 3706 and the mount 3708 are assembled to one another, the compliant member 3826 can be configured to provide a passive biasing load against the flag 3818 that forces the sensor contact(s) 3822 into continuous engagement with the corresponding circuitry contact(s) 3824. In the illustrated example, the compliant member 3826 is an elastomeric O-ring, but can alternatively comprise any other type of biasing device or mechanism, such as a compression spring or the like, without departing from the scope of the disclosure.

The sensor control device 3702 can further include one or more electromagnetic shields, shown as a first shield 3828a and a second shield The shell 3706 can provide or otherwise define a first clocking receptacle 3830a (FIG. 7B) and a second clocking receptacle 3830b (FIG. 7B), and the mount 3708 can provide or otherwise define a first clocking post 3832a (FIG. 7A) and a second clocking post 3832b (FIG. 7A). Mating the first and second clocking receptacles 3830a,b with the first and second clocking posts 3832a,b, respectively, will properly align the shell 3706 to the mount 3708.

Referring specifically to FIG. 7A, the inner surface of the mount 3708 can provide or otherwise define a plurality of pockets or depressions configured to accommodate various component parts of the sensor control device 3702 when the shell 3706 is mated to the mount 3708. For example, the inner surface of the mount 3708 can define a battery locator 3834 configured to accommodate a portion of the battery 3812 when the sensor control device 3702 is assembled. An adjacent contact pocket 3836 can be configured to accommodate a portion of the axial contact 3814a.

Moreover, a plurality of module pockets 3838 can be defined in the inner surface of the mount 3708 to accommodate the various electronic modules 3806 arranged on the bottom of the PCB 3804. Furthermore, a shield locator 3840 can be defined in the inner surface of the mount 3708 to accommodate at least a portion of the second shield 3828b when the sensor control device 3702 is assembled. The battery locator 3834, the contact pocket 3836, the module pockets 3838, and the shield locator 3840 all extend a short distance into the inner surface of the mount 3708 and, as a result, the overall height H of the sensor control device 3702 can be reduced as compared to prior sensor control devices. The module pockets 3838 can also help minimize the diameter of the PCB 3804 by allowing PCB components to be arranged on both sides (i.e., top and bottom surfaces).

Still referring to FIG. 7A, the mount 3708 can further include a plurality of carrier grip features 3842 (two shown) defined about the outer periphery of the mount 3708. The carrier grip features 3842 are axially offset from the bottom 3844 of the mount 3708, where a transfer adhesive (not shown) can be applied during assembly. In contrast to prior sensor control devices, which commonly include conical carrier grip features that intersect with the bottom of the mount, the presently disclosed carrier grip features 3842 are offset from the plane (i.e., the bottom 3844) where the transfer adhesive is applied. This can prove advantageous in helping ensure that the delivery system does not inadvertently stick to the transfer adhesive during assembly. Moreover, the presently disclosed carrier grip features 3842 eliminate the need for a scalloped transfer adhesive, which simplifies the manufacture of the transfer adhesive and eliminates the need to accurately clock the transfer adhesive relative to the mount 3708. This also increases the bond area and, therefore, the bond strength.

Referring to FIG. 7B, the bottom 3844 of the mount 3708 can provide or otherwise define a plurality of grooves 3846, which can be defined at or near the outer periphery of the mount 3708 and equidistantly spaced from each other. A transfer adhesive (not shown) can be coupled to the bottom 3844 and the grooves 3846 can be configured to help convey (transfer) moisture away from the sensor control device 3702 and toward the periphery of the mount 3708 during use. In some examples, the spacing of the grooves 3846 can interpose the module pockets 3838 (FIG. 7A) defined on the opposing side (inner surface) of the mount 3708. As will be appreciated, alternating the position of the grooves 3846 and the module pockets 3838 ensures that the opposing features on either side of the mount 3708 do not extend into each other. This can help maximize usage of the material for the mount 3708 and thereby help maintain a minimal height H of the sensor control device 3702. The module pockets 3838 can also significantly reduce mold sink, and improve the flatness of the bottom 3844 that the transfer adhesive bonds to.

Still referring to FIG. 7B, the inner surface of the shell 3706 can also provide or otherwise define a plurality of pockets or depressions configured to accommodate various component parts of the sensor control device 3702 when the shell 3706 is mated to the mount 3708. For example, the inner surface of the shell 3706 can define an opposing battery locator 3848 arrangeable opposite the battery locator 3834 (FIG. 7A) of the mount 3708 and configured to accommodate a portion of the battery 3812 when the sensor control device 3702 is assembled. The opposing battery locator 3848 extends a short distance into the inner surface of the shell 3706, which helps reduce the overall height H of the sensor control device 3702.

A sharp and sensor locator 3852 can also be provided by or otherwise defined on the inner surface of the shell 3706. The sharp and sensor locator 3852 can be configured to receive both the sharp (not shown) and a portion of the sensor 3716. Moreover, the sharp and sensor locator 3852 can be configured to align and/or mate with a corresponding sharp and sensor locator 2054 (FIG. 7A) provided on the inner surface of the mount 3708.

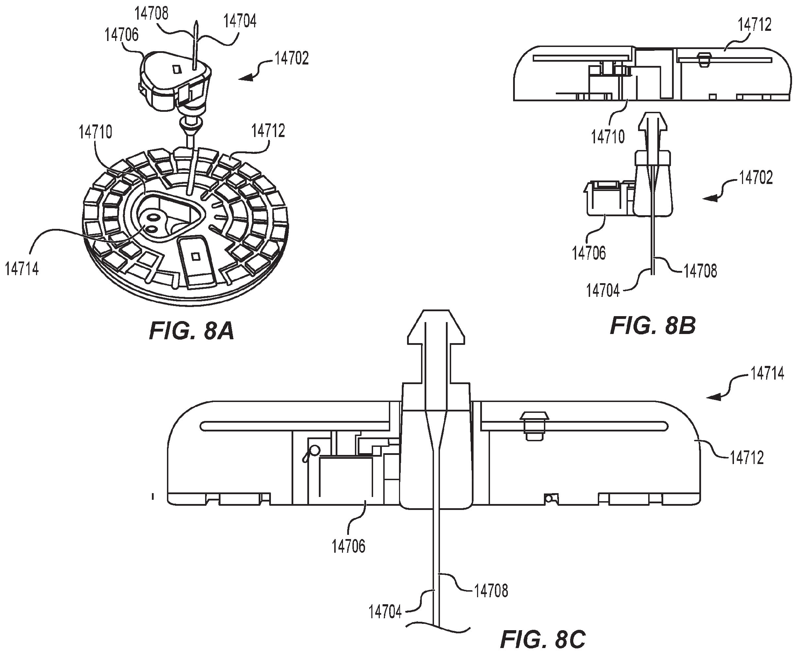

According to examples of the present disclosure, an alternative sensor assembly/electronics assembly connection approach is illustrated in FIGS. 8A to 8C. As shown, the sensor assembly 14702 includes sensor 14704, connector support 14706, and sharp 14708. Notably, a recess or receptacle 14710 can be defined in the bottom of the mount of the electronics assembly 14712 and provide a location where the sensor assembly 14702 can be received and coupled to the electronics assembly 14712 , and thereby fully assemble the sensor control device. The profile of the sensor assembly 14702 can match or be shaped in complementary fashion to the receptacle 14710, which includes an elastomeric sealing member 14714 (including conductive material coupled to the circuit board and aligned with the electrical contacts of the sensor 14704). Thus, when the sensor assembly 14702 is snap fit or otherwise adhered to the electronics assembly 14712 by driving the sensor assembly 14702 into the integrally formed recess 14710 in the electronics assembly 14712, the on-body device 14714 depicted in FIG. 8C is formed. This example provides an integrated connector for the sensor assembly 14702 within the electronics assembly 14712.

Additional information regarding sensor assemblies is provided in U.S. Publication No. 2013/0150691 and U.S. Publication No. 2021/0204841, each of which is incorporated by reference herein in its entirety.

According to examples of the present disclosure, the sensor control device 102 can be modified to provide a one-piece architecture that can be subjected to sterilization techniques specifically designed for a one-piece architecture sensor control device. A one-piece architecture allows the sensor applicator 150 and the sensor control device 102 to be shipped to the user in a single, sealed package that does not require any final user assembly attention. Rather, the user need only open one package and subsequently deliver the sensor control device 102 to the target monitoring location. The one-piece system architecture described herein can prove advantageous in eliminating component parts, various fabrication processes, and user assembly. As a result, packaging and waste are reduced, and the potential for user error or contamination to the system is mitigated.

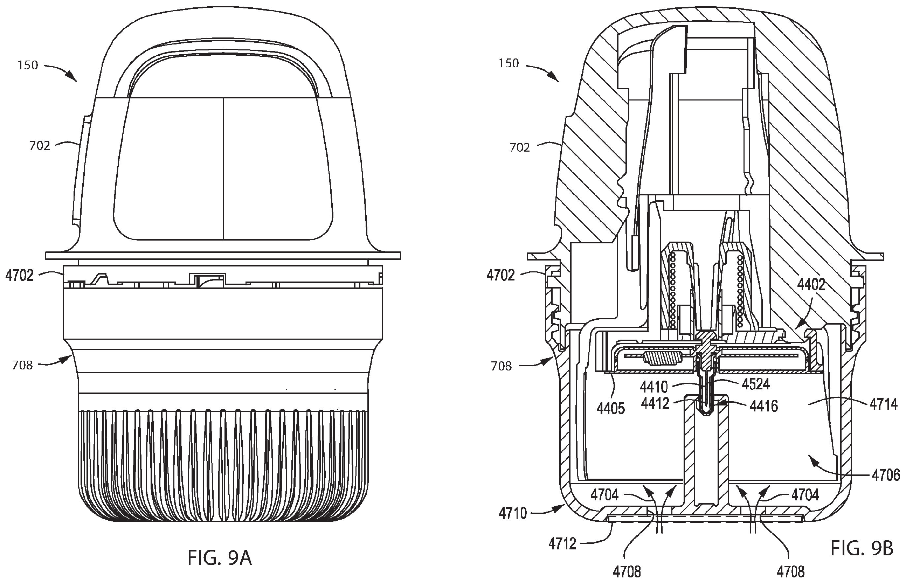

FIGS. 9A and 9B are side and cross-sectional side views, respectively, of an example of the sensor applicator 150 with the applicator cap 708 coupled thereto. More specifically, FIG. 9A depicts how the sensor applicator 150 might be shipped to and received by a user, and FIG. 9B depicts the sensor control device 4402 arranged within the sensor applicator 150. Accordingly, the fully assembled sensor control device 4402 can already be assembled and installed within the sensor applicator 150 prior to being delivered to the user, thus removing any additional assembly that a user would otherwise have to perform.

The fully assembled sensor control device 4402 can be loaded into the sensor applicator 150, and the applicator cap 708 can subsequently be coupled to the sensor applicator 150. In some examples, the applicator cap 708 can be threaded to the housing 702 and include a tamper ring 4702. Upon rotating (e.g., unscrewing) the applicator cap 708 relative to the housing 702, the tamper ring 4702 can shear and thereby free the applicator cap 708 from the sensor applicator 150.

According to the present disclosure, while loaded in the sensor applicator 150, the sensor control device 4402 can be subjected to gaseous chemical sterilization 4704 configured to sterilize the electronics housing 4404 and any other exposed portions of the sensor control device 4402. To accomplish this, a chemical can be injected into a sterilization chamber 4706 cooperatively defined by the sensor applicator 150 and the interconnected cap 210. In some applications, the chemical can be injected into the sterilization chamber 4706 via one or more vents 4708 defined in the applicator cap 708 at its proximal end 610. Example chemicals that can be used for the gaseous chemical sterilization 4704 include, but are not limited to, ethylene oxide, vaporized hydrogen peroxide, nitrogen oxide (e.g., nitrous oxide, nitrogen dioxide, etc.), and steam.

Since the distal portions of the sensor 4410 and the sharp 4412 are sealed within the sensor cap 4416, the chemicals used during the gaseous chemical sterilization process do not interact with the enzymes, chemistry, and biologics provided on the tail 4524 and other sensor components, such as membrane coatings that regulate analyte influx.

Once a desired sterility assurance level has been achieved within the sterilization chamber 4706, the gaseous solution can be removed and the sterilization chamber 4706 can be aerated. Aeration can be achieved by a series of vacuums and subsequently circulating a gas (e.g., nitrogen) or filtered air through the sterilization chamber 4706. Once the sterilization chamber 4706 is properly aerated, the vents 4708 can be occluded with a seal 4712 (shown in dashed lines).

In some examples, the seal 4712 can comprise two or more layers of different materials. The first layer can be made of a synthetic material (e.g., a flash-spun high-density polyethylene fiber), such as Tyvek^{®} available from DuPont^{®}. Tyvek^{®} is highly durable and puncture resistant and allows the permeation of vapors. The Tyvek^{®} layer can be applied before the gaseous chemical sterilization process, and following the gaseous chemical sterilization process, a foil or other vapor and moisture resistant material layer can be sealed (e.g., heat sealed) over the Tyvek^{®} layer to prevent the ingress of contaminants and moisture into the sterilization chamber 4706. In other examples, the seal 4712 can comprise only a single protective layer applied to the applicator cap 708. In such examples, the single layer can be gas permeable for the sterilization process, but can also be capable of protection against moisture and other harmful elements once the sterilization process is complete.

With the seal 4712 in place, the applicator cap 708 provides a barrier against outside contamination, and thereby maintains a sterile environment for the assembled sensor control device 4402 until the user removes (unthreads) the applicator cap 708. The applicator cap 708 can also create a dust-free environment during shipping and storage that prevents the adhesive patch 4714 from becoming dirty.

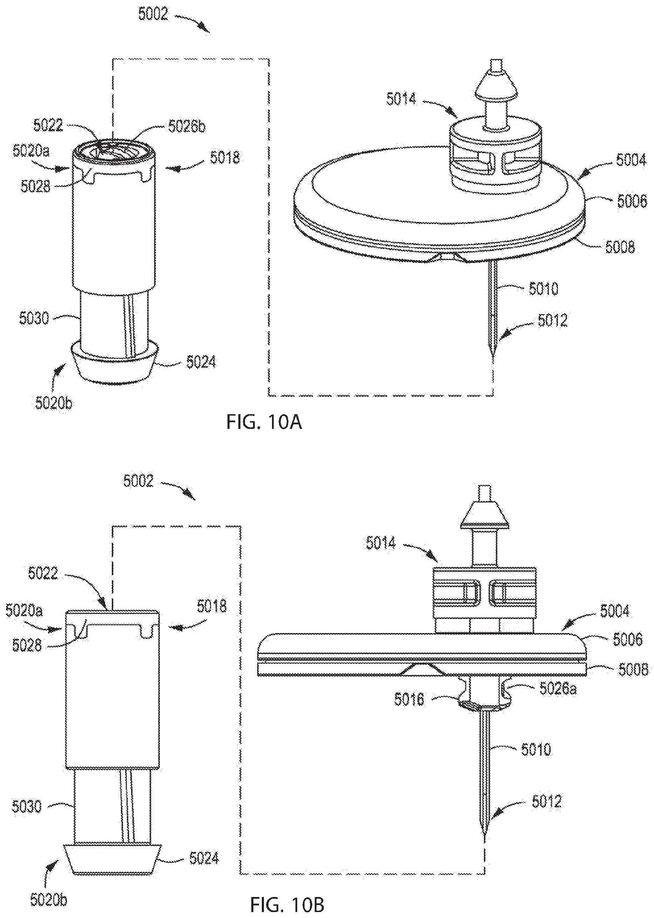

FIGS. 10A and 10B are isometric and side views, respectively, of another example sensor control device 5002, according to one or more examples of the present disclosure. The sensor control device 5002 can be similar in some respects to the sensor control device 102 of FIG. 1A and therefore can be best understood with reference thereto. Moreover, the sensor control device 5002 can replace the sensor control device 102 of FIG. 1A and, therefore, can be used in conjunction with the sensor applicator 150 of FIG. 1A, which can deliver the sensor control device 5002 to a target monitoring location on a user's skin.

Unlike the sensor control device 102 of FIG. 1A, however, the sensor control device 5002 can comprise a one-piece system architecture not requiring a user to open multiple packages and finally assemble the sensor control device 5002 prior to application. Rather, upon receipt by the user, the sensor control device 5002 can already be fully assembled and properly positioned within the sensor applicator 150 (FIG. 1A). To use the sensor control device 5002, the user need only open one barrier (e.g., the applicator cap 708 of FIG. 3B) before promptly delivering the sensor control device 5002 to the target monitoring location for use.

As illustrated, the sensor control device 5002 includes an electronics housing 5004 that is generally disc-shaped and can have a circular cross-section. In other examples, however, the electronics housing 5004 can exhibit other cross-sectional shapes, such as ovoid or polygonal, without departing from the scope of the disclosure. The electronics housing 5004 can be configured to house or otherwise contain various electrical components used to operate the sensor control device 5002. In at least one example, an adhesive patch (not shown) can be arranged at the bottom of the electronics housing 5004. The adhesive patch can be similar to the adhesive patch 105 of FIG. 1A, and can thus help adhere the sensor control device 5002 to the user's skin for use.

As illustrated, the sensor control device 5002 includes an electronics housing 5004 that includes a shell 5006 and a mount 5008 that is mateable with the shell 5006. The shell 5006 can be secured to the mount 5008 via a variety of ways, such as a snap fit engagement, an interference fit, sonic welding, one or more mechanical fasteners (e.g., screws), a gasket, an adhesive, or any combination thereof. In some cases, the shell 5006 can be secured to the mount 5008 such that a sealed interface is generated therebetween.

The sensor control device 5002 can further include a sensor 5010 (partially visible) and a sharp 5012 (partially visible), used to help deliver the sensor 5010 transcutaneously under a user's skin during application of the sensor control device 5002. As illustrated, corresponding portions of the sensor 5010 and the sharp 5012 extend distally from the bottom of the electronics housing 5004 (e.g., the mount 5008). The sharp 5012 can include a sharp hub 5014 configured to secure and carry the sharp 5012. As best seen in FIG. 10B, the sharp hub 5014 can include or otherwise define a mating member 5016. To couple the sharp 5012 to the sensor control device 5002, the sharp 5012 can be advanced axially through the electronics housing 5004 until the sharp hub 5014 engages an upper surface of the shell 5006 and the mating member 5016 extends distally from the bottom of the mount 5008. As the sharp 5012 penetrates the electronics housing 5004, the exposed portion of the sensor 5010 can be received within a hollow or recessed (arcuate) portion of the sharp 5012. The remaining portion of the sensor 5010 is arranged within the interior of the electronics housing 5004.

The sensor control device 5002 can further include a sensor cap 5018, shown exploded or detached from the electronics housing 5004 in FIGS. 10A-10B. The sensor cap 5018 can be removably coupled to the sensor control device 5002 (e.g., the electronics housing 5004) at or near the bottom of the mount 5008. The sensor cap 5018 can help provide a sealed barrier that surrounds and protects the exposed portions of the sensor 5010 and the sharp 5012 from gaseous chemical sterilization. As illustrated, the sensor cap 5018 can comprise a generally cylindrical body having a first end 5020a and a second end 5020b opposite the first end 5020a. The first end 5020a can be open to provide access into an inner chamber 5022 defined within the body. In contrast, the second end 5020b can be closed and can provide or otherwise define an engagement feature 5024. As described herein, the engagement feature 5024 can help mate the sensor cap 5018 to the cap (e.g., the applicator cap 708 of FIG. 3B) of a sensor applicator (e.g., the sensor applicator 150 of FIGS. 1 and 3A-3G), and can help remove the sensor cap 5018 from the sensor control device 5002 upon removing the cap from the sensor applicator 150.

The sensor cap 5018 can be removably coupled to the electronics housing 5004 at or near the bottom of the mount 5008. More specifically, the sensor cap 5018 can be removably coupled to the mating member 5016, which extends distally from the bottom of the mount 5008. In at least one example, for example, the mating member 5016 can define a set of external threads 5026a (FIG. 10B) mateable with a set of internal threads 5026b (FIG. 10A) defined by the sensor cap 5018. In some examples, the external and internal threads 5026a, b can comprise a flat thread design (e.g., lack of helical curvature), which can prove advantageous in molding the parts. Alternatively, the external and internal threads 5026a,b can comprise a helical threaded engagement. Accordingly, the sensor cap 5018 can be threadably coupled to the sensor control device 5002 at the mating member 5016 of the sharp hub 5014. In other examples, the sensor cap 5018 can be removably coupled to the mating member 5016 via other types of engagements including, but not limited to, an interference or friction fit, or a frangible member or substance that can be broken with minimal separation force (e.g., axial or rotational force).

In some examples, the sensor cap 5018 can comprise a monolithic (singular) structure extending between the first and second ends 5020a, b. In other examples, however, the sensor cap 5018 can comprise two or more component parts. In the illustrated example, for example, the sensor cap 5018 can include a seal ring 5028 positioned at the first end 5020a and a desiccant cap 5030 arranged at the second end 5020b. The seal ring 5028 can be configured to help seal the inner chamber 5022, as described in more detail below. In at least one example, the seal ring 5028 can comprise an elastomeric O-ring. The desiccant cap 5030 can house or comprise a desiccant to help maintain preferred humidity levels within the inner chamber 5022. The desiccant cap 5030 can also define or otherwise provide the engagement feature 5024 of the sensor cap 5018.

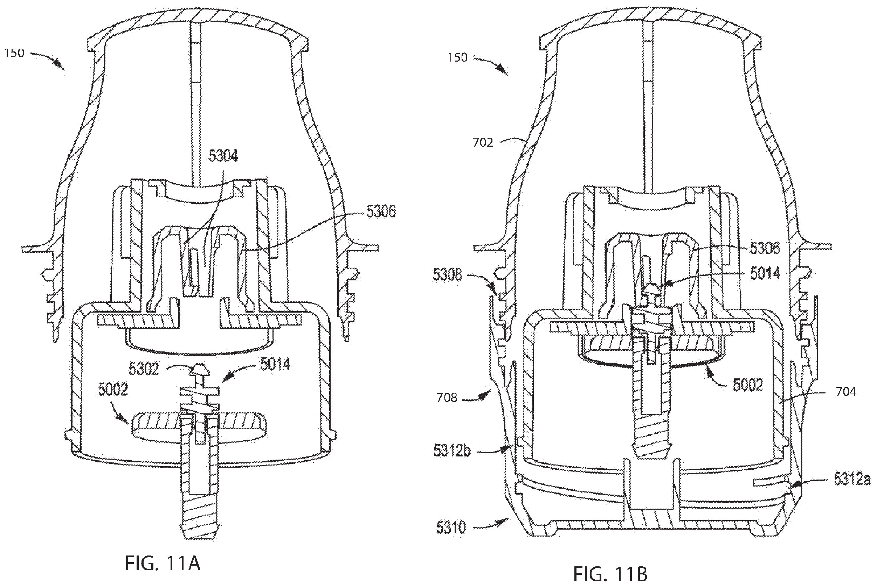

FIGS. 11A-11C are progressive cross-sectional side views showing assembly of the sensor applicator 150 with the sensor control device 5002, according to one or more examples. Once the sensor control device 5002 is fully assembled, it can then be loaded into the sensor applicator 150. With reference to FIG. 11A, the sharp hub 5014 can include or otherwise define a hub snap pawl 5302 configured to help couple the sensor control device 5002 to the sensor applicator 150. More specifically, the sensor control device 5002 can be advanced into the interior of the sensor applicator 150 and the hub snap pawl 5302 can be received by corresponding arms 5304 of a sharp carrier 5306 positioned within the sensor applicator 150.

In FIG. 11B, the sensor control device 5002 is shown received by the sharp carrier 5306 and, therefore, secured within the sensor applicator 150. Once the sensor control device 5002 is loaded into the sensor applicator 150, the applicator cap 708 can be coupled to the sensor applicator 150. In some examples, the applicator cap 708 and the housing 702 can have opposing, mateable sets of threads 5308 that enable the applicator cap 708 to be screwed onto the housing 702 in a clockwise (or counter-clockwise) direction and thereby secure the applicator cap 708 to the sensor applicator 150.

As illustrated, the sheath 704 is also positioned within the sensor applicator 150, and the sensor applicator 150 can include a sheath locking mechanism 5310 configured to ensure that the sheath 704 does not prematurely collapse during a shock event. In the illustrated example, the sheath locking mechanism 5310 can comprise a threaded engagement between the applicator cap 708 and the sheath 704. More specifically, one or more internal threads 53l2a can be defined or otherwise provided on the inner surface of the applicator cap 708, and one or more external threads 5312b can be defined or otherwise provided on the sheath 704. The internal and external threads 5312a,b can be configured to threadably mate as the applicator cap 708 is threaded to the sensor applicator 150 at the threads 5308. The internal and external threads 53l2a,b can have the same thread pitch as the threads 5308 that enable the applicator cap 708 to be screwed onto the housing 702.

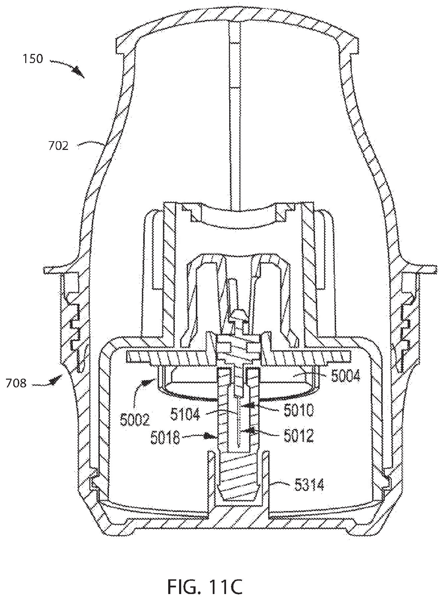

In FIG. 11C, the applicator cap 708 is shown fully threaded (coupled) to the housing 702. As illustrated, the applicator cap 708 can further provide and otherwise define a cap post 5314 centrally located within the interior of the applicator cap 708 and extending proximally from the bottom thereof. The cap post 5314 can be configured to receive at least a portion of the sensor cap 5018 as the applicator cap 708 is screwed onto the housing 702.

With the sensor control device 5002 loaded within the sensor applicator 150 and the applicator cap 708 properly secured, the sensor control device 5002 can then be subjected to a gaseous chemical sterilization configured to sterilize the electronics housing 5004 and any other exposed portions of the sensor control device 5002. Since the distal portions of the sensor 5010 and the sharp 5012 are sealed within the sensor cap 5018, the chemicals used during the gaseous chemical sterilization process are unable to interact with the enzymes, chemistry, and biologies provided on the tail 5104, and other sensor components, such as membrane coatings that regulate analyte influx.

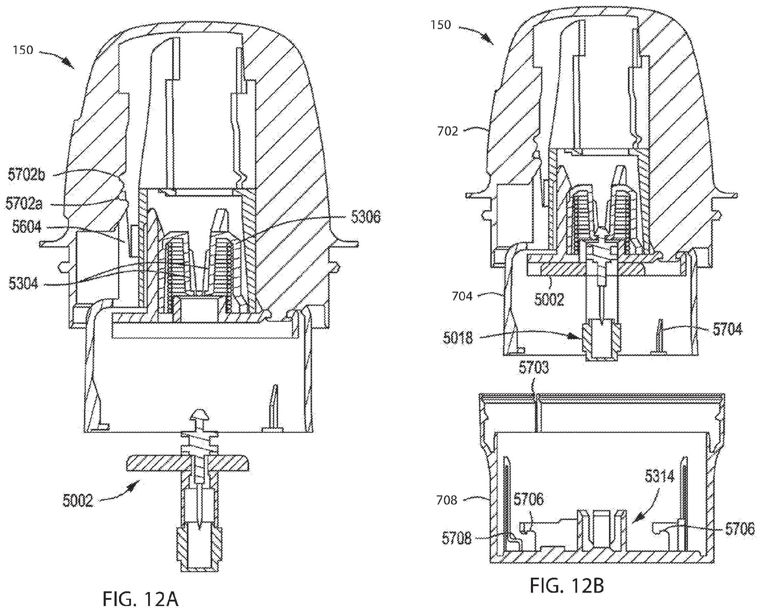

FIGS. 12A-12C are progressive cross-sectional side views showing assembly and disassembly of an another example of the sensor applicator 150 with the sensor control device 5002, according to one or more additional examples. A fully assembled sensor control device 5002 can be loaded into the sensor applicator 150 by coupling the hub snap pawl 5302 into the arms 5304 of the sharp carrier 5306 positioned within the sensor applicator 150, as generally described above.

In the illustrated example, the sheath arms 5604 of the sheath 704 can be configured to interact with a first detent 5702a and a second detent 5702b defined within the interior of the housing 702. The first detent 5702a can alternately be referred to a "locking" detent, and the second detent 5702b can alternately be referred to as a "firing" detent. When the sensor control device 5002 is initially installed in the sensor applicator 150, the sheath arms 5604 can be received within the first detent 5702a. As discussed below, the sheath 704 can be actuated to move the sheath arms 5604 to the second detent 5702b, which places the sensor applicator 150 in firing position.

In FIG. 12B, the applicator cap 708 is aligned with the housing 702 and advanced toward the housing 702 so that the sheath 704 is received within the applicator cap 708. Instead of rotating the applicator cap 708 relative to the housing 702, the threads of the applicator cap 708 can be snapped onto the corresponding threads of the housing 702 to couple the applicator cap 708 to the housing 702. Axial cuts or slots 5703 (one shown) defined in the applicator cap 708 can allow portions of the applicator cap 708 near its threading to flex outward to be snapped into engagement with the threading of the housing 702. As the applicator cap 708 is snapped to the housing 702, the sensor cap 5018 can correspondingly be snapped into the cap post 5314.

Similar to the example of FIGS. 11A-11C, the sensor applicator 150 can include a sheath locking mechanism configured to ensure that the sheath 704 does not prematurely collapse during a shock event. In the illustrated example, the sheath locking mechanism includes one or more ribs 5704 (one shown) defined near the base of the sheath 704 and configured to interact with one or more ribs 5706 (two shown) and a shoulder 5708 defined near the base of the applicator cap 708. The ribs 5704 can be configured to inter-lock between the ribs 5706 and the shoulder 5708 while attaching the applicator cap 708 to the housing 702. More specifically, once the applicator cap 708 is snapped onto the housing 702, the applicator cap 708 can be rotated (e.g., clockwise), which locates the ribs 5704 of the sheath 704 between the ribs 5706 and the shoulder 5708 of the applicator cap 708 and thereby "locks" the applicator cap 708 in place until the user reverse rotates the applicator cap 708 to remove the applicator cap 708 for use. Engagement of the ribs 5704 between the ribs 5706 and the shoulder 5708 of the applicator cap 708 can also prevent the sheath 704 from collapsing prematurely.

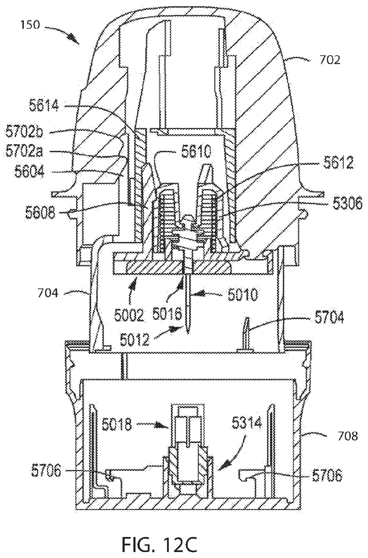

In FIG. 12C, the applicator cap 708 is removed from the housing 702. As with the example of FIGS. 12A-12C, the applicator cap 708 can be removed by reverse rotating the applicator cap 708, which correspondingly rotates the cap post 5314 in the same direction and causes sensor cap 5018 to unthread from the mating member 5016, as generally described above. Moreover, detaching the sensor cap 5018 from the sensor control device 5002 exposes the distal portions of the sensor 5010 and the sharp 5012.

As the applicator cap 708 is unscrewed from the housing 702, the ribs 5704 defined on the sheath 704 can slidingly engage the tops of the ribs 5706 defined on the applicator cap 708. The tops of the ribs 5706 can provide corresponding ramped surfaces that result in an upward displacement of the sheath 704 as the applicator cap 708 is rotated, and moving the sheath 704 upward causes the sheath arms 5604 to flex out of engagement with the first detent 5702a to be received within the second detent 5702b. As the sheath 704 moves to the second detent 5702b, the radial shoulder 5614 moves out of radial engagement with the carrier arm(s) 5608, which allows the passive spring force of the spring 5612 to push upward on the sharp carrier 5306 and force the carrier arm(s) 5608 out of engagement with the groove(s) 5610. As the sharp carrier 5306 moves upward within the housing 702, the mating member 5016 can correspondingly retract until it becomes flush, substantially flush, or sub-flush with the bottom of the sensor control device 5002. At this point, the sensor applicator 150 in firing position. Accordingly, in this example, removing the applicator cap 708 correspondingly causes the mating member 5016 to retract.

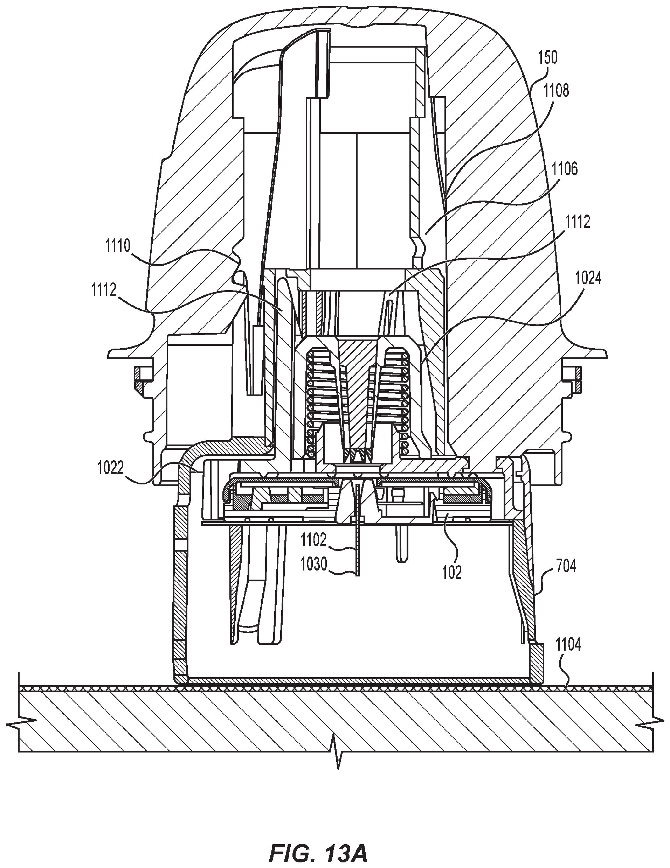

FIGS. 13A-13F illustrate example details of examples of the internal device mechanics of "firing" the applicator 150 to apply sensor control device 102 to a user and including retracting sharp 1030 safely back into used applicator 150. All together, these drawings represent an example sequence of driving sharp 1030 (supporting a sensor coupled to sensor control device 102) into the skin of a user, withdrawing the sharp while leaving the sensor behind in operative contact with interstitial fluid of the user, and adhering the sensor control device to the skin of the user with an adhesive. Modification of such activity for use with the alternative applicator assembly examples and components can be appreciated in reference to the same by those with skill in the art. Moreover, applicator 150 can be a sensor applicator having one-piece architecture or a two-piece architecture as disclosed herein.

Turning now to FIG. 13A, a sensor 1102 is supported within sharp 1030, just above the skin 1104 of the user. Rails 1106 (optionally three of them) of an upper guide section 1108 can be provided to control applicator 150 motion relative to sheath 704. The sheath 704 is held by detent features 1110 within the applicator 150 such that appropriate downward force along the longitudinal axis of the applicator 150 will cause the resistance provided by the detent features 1110 to be overcome so that sharp 1030 and sensor control device 102 can translate along the longitudinal axis into (and onto) skin 1104 of the user. In addition, catch arms 1112 of sensor carrier 1022 engage the sharp retraction assembly 1024 to maintain the sharp 1030 in a position relative to the sensor control device 102.

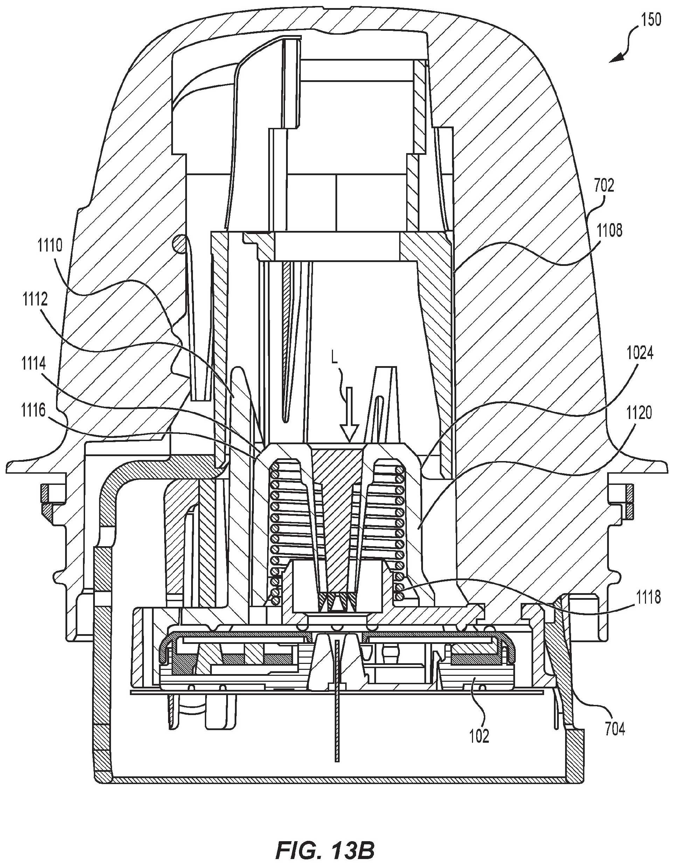

In FIG. 13B, user force is applied to overcome or override detent features 1110 and sheath 704 collapses into housing 702 driving the sensor control device 102 (with associated parts) to translate down as indicated by the arrow L along the longitudinal axis. An inner diameter of the upper guide section 1108 of the sheath 704 constrains the position of carrier arms 1112 through the full stroke of the sensor/sharp insertion process. The retention of the stop surfaces 1114 of carrier arms 1112 against the complimentary faces 1116 of the sharp retraction assembly 1024 maintains the position of the members with return spring 1118 fully energized. According to examples, rather than employing user force to drive the sensor control device 102 to translate down as indicated by the arrow L along the longitudinal axis, housing 702 can include a button (for example, not limitation, a push button) which activates a drive spring (for example, not limitation, a coil spring) to drive the sensor control device 102.

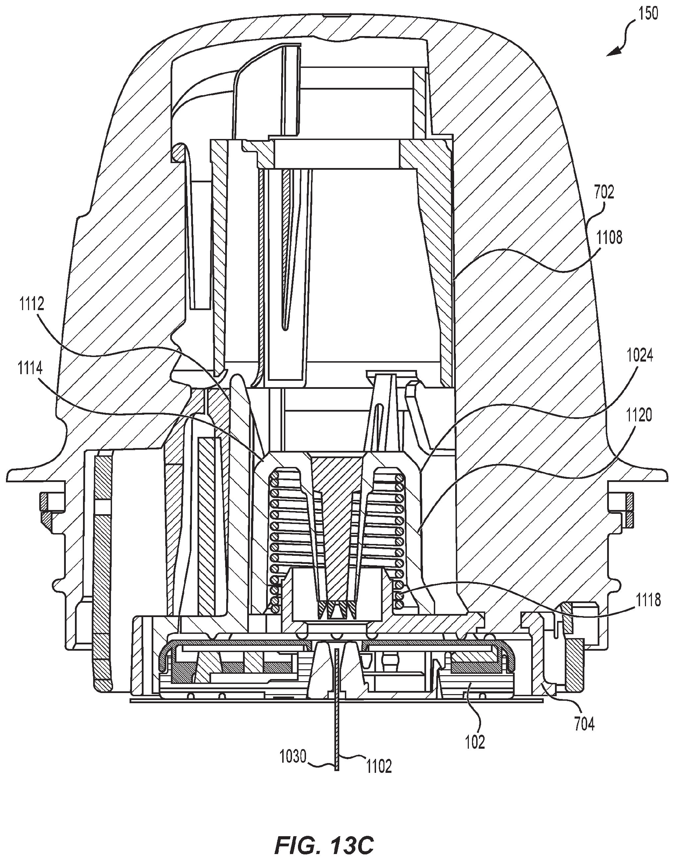

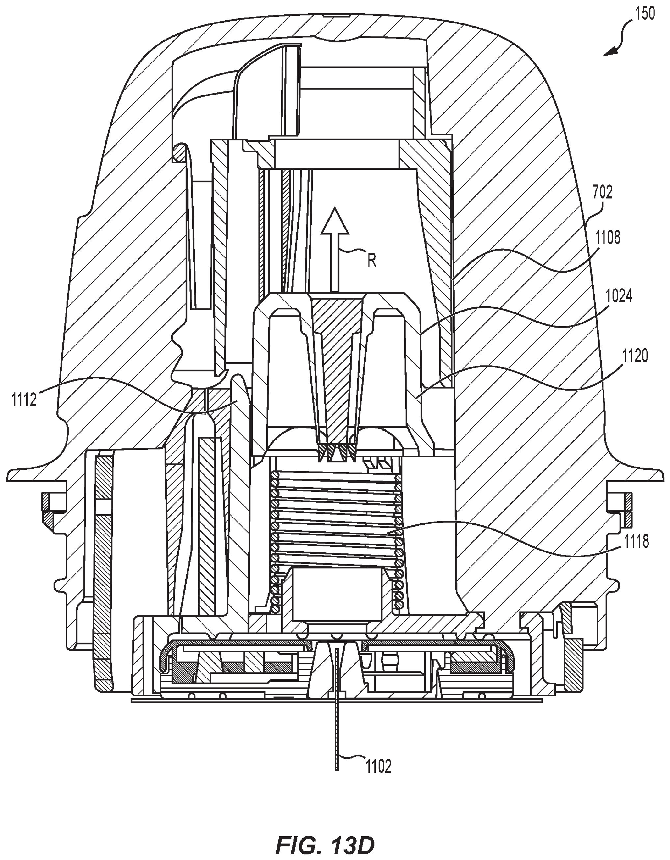

In FIG. 13C, sensor 1102 and sharp 1030 have reached full insertion depth. In so doing, the carrier arms 1112 clear the upper guide section 1108 inner diameter. Then, the compressed force of the coil return spring 1118 drives angled stop surfaces 1114 radially outward, releasing force to drive the sharp carrier 1102 of the sharp retraction assembly 1024 to pull the (slotted or otherwise configured) sharp 1030 out of the user and off of the sensor 1102 as indicated by the arrow R in FIG. 13D.

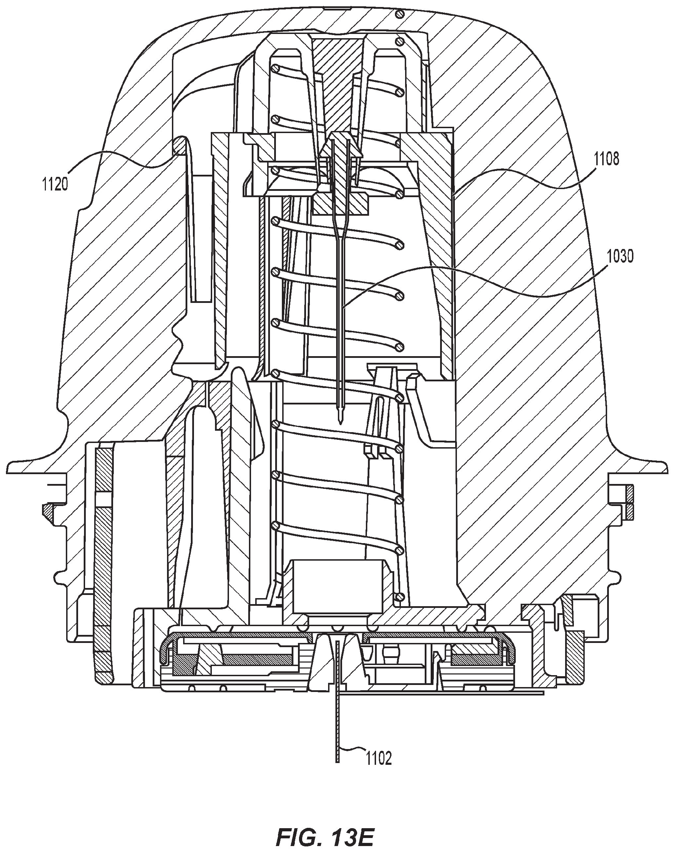

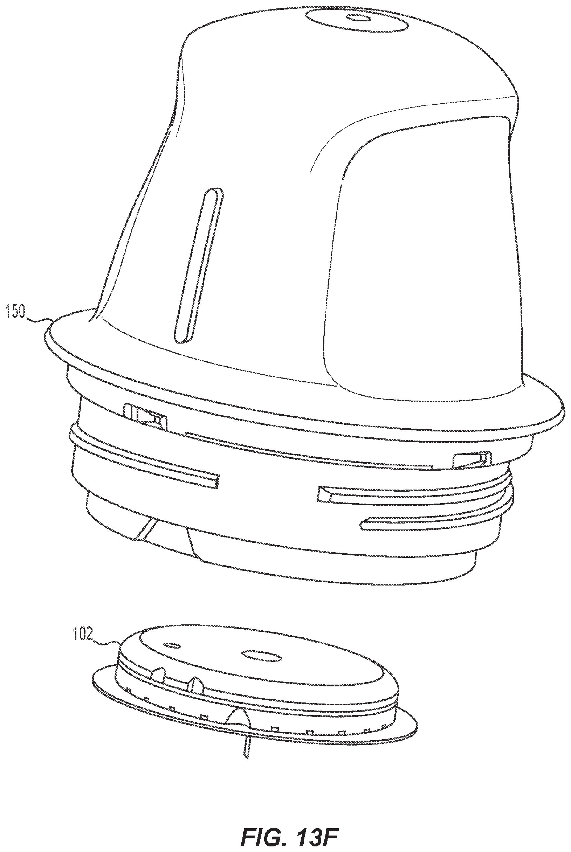

With the sharp 1030 fully retracted as shown in FIG. 13E, the upper guide section 1108 of the sheath 704 is set with a final locking feature 1120. As shown in FIG. 13F, the spent applicator assembly 150 is removed from the insertion site, leaving behind the sensor control device 102, and with the sharp 1030 secured safely inside the applicator assembly 150. The spent applicator assembly 150 is now ready for disposal.

Operation of the applicator 150 when applying the sensor control device 102 is designed to provide the user with a sensation that both the insertion and retraction of the sharp 1030 is performed automatically by the internal mechanisms of the applicator 150. In other words, the disclosed subject matter avoids the user experiencing the sensation that he is manually driving the sharp 1030 into his skin. Thus, once the user applies sufficient force to overcome the resistance from the detent features of the applicator 150, the resulting actions of the applicator 150 are perceived to be an automated response to the applicator being "triggered." The user does not perceive that he is supplying additional force to drive the sharp 1030 to pierce his skin despite that all the driving force is provided by the user and no additional biasing/driving apparatus are used to insert the sharp 1030. As detailed above in FIG. 13C, the retraction of the sharp 1030 is automated by the coil return spring 1118 of the applicator 150.

With respect to any of the applicator examples described herein, as well as any of the components thereof, including but not limited to the sharp, sharp module and sensor module examples, those of skill in the art will understand that said examples can be dimensioned and configured for use with sensors configured to sense an analyte level in a bodily fluid in the epidermis, dermis, or subcutaneous tissue of a subject. In some examples, for example, sharps and distal portions of analyte sensors disclosed herein can both be dimensioned and configured to be positioned at a particular end-depth (i.e., the furthest point of penetration in a tissue or layer of the subject's body, e.g., in the epidermis, dermis, or subcutaneous tissue). With respect to some applicator examples, those of skill in the art will appreciate that certain examples of sharps can be dimensioned and configured to be positioned at a different end-depth in the subject's body relative to the final end-depth of the analyte sensor. In some examples, for example, a sharp can be positioned at a first end-depth in the subject's epidermis prior to retraction, while a distal portion of an analyte sensor can be positioned at a second end-depth in the subject's dermis. In other examples, a sharp can be positioned at a first end-depth in the subject's dermis prior to retraction, while a distal portion of an analyte sensor can be positioned at a second end-depth in the subject's subcutaneous tissue. In still other examples, a sharp can be positioned at a first end-depth prior to retraction and the analyte sensor can be positioned at a second end-depth, wherein the first end-depth and second end-depths are both in the same layer or tissue of the subject's body.

Additionally, with respect to any of the applicator examples described herein, those of skill in the art will understand that an analyte sensor, as well as one or more structural components coupled thereto, including but not limited to one or more spring-mechanisms, can be disposed within the applicator in an off-center position relative to one or more axes of the applicator. In some applicator examples, for example, an analyte sensor and a spring mechanism can be disposed in a first off-center position relative to an axis of the applicator on a first side of the applicator, and the sensor electronics can be disposed in a second off-center position relative to the axis of the applicator on a second side of the applicator. In other applicator examples, the analyte sensor, spring mechanism, and sensor electronics can be disposed in an off-center position relative to an axis of the applicator on the same side. Those of skill in the art will appreciate that other permutations and configurations in which any or all of the analyte sensor, spring mechanism, sensor electronics, and other components of the applicator are disposed in a centered or off-centered position relative to one or more axes of the applicator are possible and fully within the scope of the present disclosure.

Additional details of suitable devices, systems, methods, components and the operation thereof along with related features are set forth in International Publication No. WO2018/136898 to Rao et. al., International Publication No. WO2019/236850 to Thomas et. al., International Publication No. WO2019/236859 to Thomas et. al., International Publication No. WO2019/236876 to Thomas et. al., and U.S. Patent Publication No. 2020/0196919, filed June 6, 2019, each of which is incorporated by reference in its entirety herein. Further details regarding examples of applicators, their components, and variants thereof, are described in U.S. Patent Publication Nos. 2013/0150691, 2016/0331283, and 2018/0235520, all of which are incorporated by reference herein in their entireties and for all purposes. Further details regarding examples of sharp modules, sharps, their components, and variants thereof, are described in U.S. Patent Publication No. 2014/0171771, which is incorporated by reference herein in its entirety and for all purposes.

Biochemical sensors can be described by one or more sensing characteristics. A common sensing characteristic is referred to as the biochemical sensor's sensitivity, which is a measure of the sensor's responsiveness to the concentration of the chemical or composition it is designed to detect. For electrochemical sensors, this response can be in the form of an electrical current (amperometric) or electrical charge (coulometric). For other types of sensors, the response can be in a different form, such as a photonic intensity (e.g., optical light). The sensitivity of a biochemical analyte sensor can vary depending on a number of factors, including whether the sensor is in an *in vitro* state or an *in vivo* state.

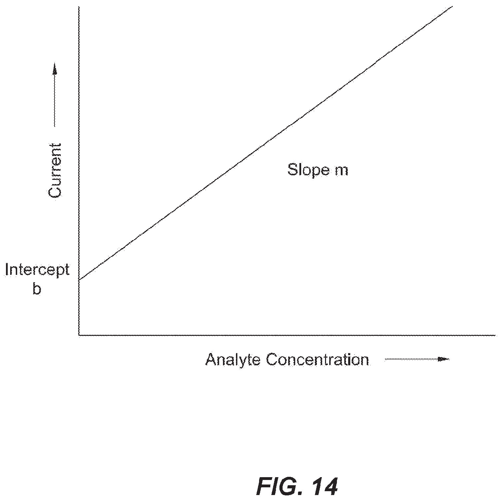

FIG. 14 is a graph depicting the *in vitro* sensitivity of an amperometric analyte sensor. The *in vitro* sensitivity can be obtained by *in vitro* testing the sensor at various analyte concentrations and then performing a regression (e.g., linear or non-linear) or other curve fitting on the resulting data. In this example, the analyte sensor's sensitivity is linear, or substantially linear, and can be modeled according to the equation y=mx+b, where y is the sensor's electrical output current, x is the analyte level (or concentration), m is the slope of the sensitivity and b is the intercept of the sensitivity, where the intercept generally corresponds to a background signal (e.g., noise). For sensors with a linear or substantially linear response, the analyte level that corresponds to a given current can be determined from the slope and intercept of the sensitivity. Sensors with a non-linear sensitivity require additional information to determine the analyte level resulting from the sensor's output current, and those of ordinary skill in the art are familiar with manners by which to model non-linear sensitivities. In some examples of *in vivo* sensors, the *in vitro* sensitivity can be the same as the *in vivo* sensitivity, but in other examples a transfer (or conversion) function is used to translate the *in vitro* sensitivity into the *in vivo* sensitivity that is applicable to the sensor's intended *in vivo* use.

Calibration is a technique for improving or maintaining accuracy by adjusting a sensor's measured output to reduce the differences with the sensor's expected output. One or more parameters that describe the sensor's sensing characteristics, like its sensitivity, are established for use in the calibration adjustment.

Certain *in vivo* analyte monitoring systems require calibration to occur after implantation of the sensor into the user or patient, either by user interaction or by the system itself in an automated fashion. For example, when user interaction is required, the user performs an *in vitro* measurement (e.g., a blood glucose (BG) measurement using a finger stick and an *in vitro* test strip) and enters this into the system, while the analyte sensor is implanted. The system then compares the *in vitro* measurement with the *in vivo* signal and, using the differential, determines an estimate of the sensor's *in vivo* sensitivity. The *in vivo* sensitivity can then be used in an algorithmic process to transform the data collected with the sensor to a value that indicates the user's analyte level. This and other processes that require user action to perform calibration are referred to as "user calibration." Systems can require user calibration due to instability of the sensor's sensitivity, such that the sensitivity drifts or changes over time. Thus, multiple user calibrations (e.g., according to a periodic (e.g., daily) schedule, variable schedule, or on an as-needed basis) can be required to maintain accuracy. While the examples described herein can incorporate a degree of user calibration for a particular implementation, generally this is not preferred as it requires the user to perform a painful or otherwise burdensome BG measurement, and can introduce user error.

Some *in vivo* analyte monitoring systems can regularly adjust the calibration parameters through the use of automated measurements of characteristics of the sensor made by the system itself (e.g., processing circuitry executing software). The repeated adjustment of the sensor's sensitivity based on a variable measured by the system (and not the user) is referred to generally as "system" (or automated) calibration, and can be performed with user calibration, such as an early BG measurement, or without user calibration. Like the case with repeated user calibrations, repeated system calibrations are typically necessitated by drift in the sensor's sensitivity over time. Thus, while the examples described herein can be used with a degree of automated system calibration, preferably the sensor's sensitivity is relatively stable over time such that post-implantation calibration is not required.

Some *in vivo* analyte monitoring systems operate with a sensor that is factory calibrated. Factory calibration refers to the determination or estimation of the one or more calibration parameters prior to distribution to the user or healthcare professional (HCP). The calibration parameter can be determined by the sensor manufacturer (or the manufacturer of the other components of the sensor control device if the two entities are different). Many *in vivo* sensor manufacturing processes fabricate the sensors in groups or batches referred to as production lots, manufacturing stage lots, or simply lots. A single lot can include thousands of sensors.

Sensors can include a calibration code or parameter which can be derived or determined during one or more sensor manufacturing processes and coded or programmed, as part of the manufacturing process, in the data processing device of the analyte monitoring system or provided on the sensor itself, for example, as a bar code, a laser tag, an RFID tag, or other machine readable information provided on the sensor. User calibration during *in vivo* use of the sensor can be obviated, or the frequency of *in vivo* calibrations during sensor wear can be reduced if the code is provided to a receiver (or other data processing device). In examples where the calibration code or parameter is provided on the sensor itself, prior to or at the start of the sensor use, the calibration code or parameter can be automatically transmitted or provided to the data processing device in the analyte monitoring system.

Some *in vivo* analyte monitoring system operate with a sensor that can be one or more of factory calibrated, system calibrated, and/or user calibrated. For example, the sensor can be provided with a calibration code or parameter which can allow for factory calibration. If the information is provided to a receiver (for example, entered by a user), the sensor can operate as a factory calibrated sensor. If the information is not provided to a receiver, the sensor can operate as a user calibrated sensor and/or a system calibrated sensor.

In a further aspect, programming or executable instructions can be provided or stored in the data processing device of the analyte monitoring system, and/or the receiver/controller unit, to provide a time varying adjustment algorithm to the *in vivo* sensor during use. For example, based on a retrospective statistical analysis of analyte sensors used *in vivo* and the corresponding glucose level feedback, a predetermined or analytical curve or a database can be generated which is time based, and configured to provide additional adjustment to the one or more *in vivo* sensor parameters to compensate for potential sensor drift in stability profile, or other factors.

In accordance with the disclosed subject matter, the analyte monitoring system can be configured to compensate or adjust for the sensor sensitivity based on a sensor drift profile. A time varying parameter β(t) can be defined or determined based on analysis of sensor behavior during *in vivo* use, and a time varying drift profile can be determined. In some aspects, the compensation or adjustment to the sensor sensitivity can be programmed in the receiver unit, the controller or data processor of the analyte monitoring system such that the compensation or the adjustment or both can be performed automatically and/or iteratively when sensor data is received from the analyte sensor. In accordance with the disclosed subject matter, the adjustment or compensation algorithm can be initiated or executed by the user (rather than self-initiating or executing) such that the adjustment or the compensation to the analyte sensor sensitivity profile is performed or executed upon user initiation or activation of the corresponding function or routine, or upon the user entering the sensor calibration code.

In accordance with the disclosed subject matter, each sensor in the sensor lot (in some instances not including sample sensors used for *in vitro* testing) can be examined non-destructively to determine or measure its characteristics such as membrane thickness at one or more points of the sensor, and other characteristics including physical characteristics such as the surface area/volume of the active area can be measured or determined. Such measurement or determination can be performed in an automated manner using, for example, optical scanners or other suitable measurement devices or systems, and the determined sensor characteristics for each sensor in the sensor lot is compared to the corresponding mean values based on the sample sensors for possible correction of the calibration parameter or code assigned to each sensor. For example, for a calibration parameter defined as the sensor sensitivity, the sensitivity is approximately inversely proportional to the membrane thickness, such that, for example, a sensor having a measured membrane thickness of approximately 4% greater than the mean membrane thickness for the sampled sensors from the same sensor lot as the sensor, the sensitivity assigned to that sensor in one example is the mean sensitivity determined from the sampled sensors divided by 1.04. Likewise, since the sensitivity is approximately proportional to active area of the sensor, a sensor having measured active area of approximately 3% lower than the mean active area for the sampled sensors from the same sensor lot, the sensitivity assigned to that sensor is the mean sensitivity multiplied by 0.97. The assigned sensitivity can be determined from the mean sensitivity from the sampled sensors, by multiple successive adjustments for each examination or measurement of the sensor. In some examples, examination or measurement of each sensor can additionally include measurement of membrane consistency or texture in addition to the membrane thickness and/or surface are or volume of the active sensing area.

Additional information regarding sensor calibration is provided in U.S. Publication No. 2010/00230285 and U.S. Publication No. 2019/0274598, each of which is incorporated by reference herein in its entirety.

The storage memory 5030 of the sensor control device 102 can include the software blocks related to communication protocols of the communication module. For example, the storage memory 5030 can include a BLE services software block with functions to provide interfaces to make the BLE module 5041 available to the computing hardware of the sensor control device 102. These software functions can include a BLE logical interface and interface parser. BLE services offered by the communication module 5040 can include the generic access profile service, the generic attribute service, generic access service, device information service, data transmission services, and security services. The data transmission service can be a primary service used for transmitting data such as sensor control data, sensor status data, analyte measurement data (historical and current), and event log data. The sensor status data can include error data, current time active, and software state. The analyte measurement data can include information such as current and historical raw measurement values, current and historical values after processing using an appropriate algorithm or model, projections and trends of measurement levels, comparisons of other values to patient-specific averages, calls to action as determined by the algorithms or models and other similar types of data.

According to aspects of the disclosed subject matter, and as embodied herein, a sensor control device 102 can be configured to communicate with multiple devices concurrently by adapting the features of a communication protocol or medium supported by the hardware and radios of the sensor control device 102. As an example, the BLE module 5041 of the communication module 5040 can be provided with software or firmware to enable multiple concurrent connections between the sensor control device 102 as a central device and the other devices as peripheral devices, or as a peripheral device where another device is a central device.

Connections, and ensuing communication sessions, between two devices using a communication protocol such as BLE can be characterized by a similar physical channel operated between the two devices (e.g., a sensor control device 102 and data receiving device 120). The physical channel can include a single channel or a series of channels, including for example and without limitation using an agreed upon series of channels determined by a common clock and channel- or frequency-hopping sequence. Communication sessions can use a similar amount of the available communication spectrum, and multiple such communication sessions can exist in proximity. In some example, each collection of devices in a communication session uses a different physical channel or series of channels, to manage interference of devices in the same proximity.

For purpose of illustration and not limitation, reference is made to an example of a procedure for a sensor-receiver connection for use with the disclosed subject matter. First, the sensor control device 102 repeatedly advertises its connection information to its environment in a search for a data receiving device 120. The sensor control device 102 can repeat advertising on a regular basis until a connection established. The data receiving device 120 detects the advertising packet and scans and filters for the sensor control device 102 to connect to through the data provided in the advertising packet. Next, data receiving device 120 sends a scan request command and the sensor control device 102 responds with a scan response packet providing additional details. Then, the data receiving device 120 sends a connection request using the Bluetooth device address associated with the data receiving device 120. The data receiving device 120 can also continuously request to establish a connection to a sensor control device 102 with a specific Bluetooth device address. Then, the devices establish an initial connection allowing them to begin to exchange data. The devices begin a process to initialize data exchange services and perform a mutual authentication procedure.

During a first connection between the sensor control device 102 and data receiving device 120, the data receiving device 120 can initialize a service, characteristic, and attribute discovery procedure. The data receiving device 120 can evaluate these features of the sensor control device 102 and store them for use during subsequent connections. Next, the devices enable a notification for a customized security service used for mutual authentication of the sensor control device 102 and data receiving device 120. The mutual authentication procedure can be automated and require no user interaction. Following the successful completion of the mutual authentication procedure, the sensor control device 102 sends a connection parameter update to request the data receiving device 120 to use connection parameter settings preferred by the sensor control device 102 and configured to maximum longevity.

The data receiving device 120 then performs sensor control procedures to backfill historical data, current data, event log, and factory data. As an example, for each type of data, the data receiving device 120 sends a request to initiate a backfill process. The request can specify a range of records defined based on, for example, the measurement value, timestamp, or similar, as appropriate. The sensor control device 102 responds with requested data until all previously unsent data in the memory of the sensor control device 102 is delivered to the data receiving device 120. The sensor control device 102 can respond to a backfill request from the data receiving device 120 that all data has already been sent. Once backfill is completed, the data receiving device 120 can notify sensor control device 102 that it is ready to receive regular measurement readings. The sensor control device 102 can send readings across multiple notifications result on a repeating basis. As embodied herein, the multiple notifications can be redundant notifications to ensure that data is transmitted correctly. Alternatively, multiple notifications can make up a single payload.

For purpose of illustration and not limitation, reference is made to an example of a procedure to send a shutdown command to the sensor control device 102. The shutdown operation is executed if the sensor control device 102 is in, for example, an error state, insertion failed state, or sensor expired state. If the sensor control device 102 is not in those states, the sensor control device 102 can log the command and execute the shutdown when sensor control device 102 transitions into the error state or sensor expired state. The data receiving device 120 sends a properly formatted shutdown command to the sensor control device 102. If the sensor control device 102 is actively processing another command, the sensor control device 102 will respond with a standard error response indicating that the sensor control device 102 is busy. Otherwise, the sensor control device 102 sends a response as the command is received. Additionally, the sensor control device 102 sends a success notification through the sensor control characteristic to acknowledge the sensor control device 102 has received the command. The sensor control device 102 registers the shutdown command. At the next appropriate opportunity (e.g., depending on the current sensor state, as described herein), the sensor control device 102 will shut down.

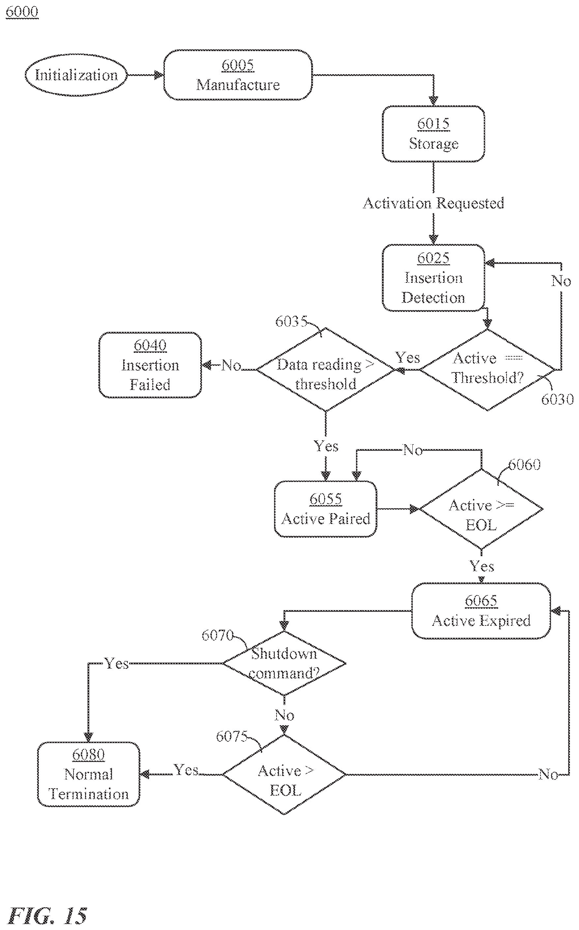

For purpose of illustration and not limitation, reference is made to the example of a high-level depiction of a state machine representation 6000 of the actions that can be taken by the sensor control device 102 as shown in FIG. 15. After initialization, the sensor enters state 6005, which relates to the manufacture of the sensor control device 102. In the manufacture state 6005 the sensor control device 102 can be configured for operation, for example, the storage memory 5030 can be written. At various times while in state 6005, the sensor control device 102 checks for a received command to go to the storage state 6015. Upon entry to the storage state 6015, the sensor performs a software integrity check. While in the storage state 6015, the sensor can also receive an activation request command before advancing to the insertion detection state 6025.

Upon entry to state 6025, the sensor control device 102 can store information relating to devices authenticated to communicate with the sensor as set during activation or initialize algorithms related to conducting and interpreting measurements from the sensing hardware 5060. The sensor control device 102 can also initialize a lifecycle timer, responsible for maintaining an active count of the time of operation of the sensor control device 102 and begin communication with authenticated devices to transmit recorded data. While in the insertion detection state 6025, the sensor can enter state 6030, where the sensor control device 102 checks whether the time of operation is equal to a predetermined threshold. This time of operation threshold can correspond to a timeout function for determining whether an insertion has been successful. If the time of operation has reached the threshold, the sensor control device 102 advances to state 6035, in which the sensor control device 102 checks whether the average data reading is greater than a threshold amount corresponding to an expected data reading volume for triggering detection of a successful insertion. If the data reading volume is lower than the threshold while in state 6035, the sensor advances to state 6040, corresponding to a failed insertion. If the data reading volume satisfies the threshold, the sensor advances to the active paired state 6055.

The active paired state 6055 of the sensor control device 102 reflects the state while the sensor control device 102 is operating as normal by recording measurements, processing the measurements, and reporting them as appropriate. While in the active paired state 6055, the sensor control device 102 sends measurement results or attempts to establish a connection with a receiving device 120. The sensor control device 102 also increments the time of operation. Once the sensor control device 102 reaches a predetermined threshold time of operation (e.g., once the time of operation reaches a predetermined threshold), the sensor control device 102 transitions to the active expired state 6065. The active expired state 6065 of the sensor control device 102 reflects the state while the sensor control device 102 has operated for its maximum predetermined amount of time.

While in the active expired state 6065, the sensor control device 102 can generally perform operations relating to winding down operation and ensuring that the collected measurements have been securely transmitted to receiving devices as needed. For example, while in the active expired state 6065, the sensor control device 102 can transmit collected data and, if no connection is available, can increase efforts to discover authenticated devices nearby and establish and connection therewith. While in the active expired state 6065, the sensor control device 102 can receive a shutdown command at state 6070. If no shutdown command is received, the sensor control device 102 can also, at state 6075, check if the time of operation has exceeded a final operation threshold. The final operation threshold can be based on the battery life of the sensor control device 102. The normal termination state 6080 corresponds to the final operations of the sensor control device 102 and ultimately shutting down the sensor control device 102.

Before a sensor is activated, the ASIC 5000 resides in a low power storage mode state. The activation process can begin, for example, when an incoming RF field (e.g., NFC field) drives the voltage of the power supply to the ASIC 5000 above a reset threshold, which causes the sensor control device 102 to enter a wake-up state. While in the wake-up state, the ASIC 5000 enters an activation sequence state. The ASIC 5000 then wakes the communication module 5040. The communication module 5040 is initialized, triggering a power on self-test. The power on self-test can include the ASIC 5000 communicating with the communication module 5040 using a prescribed sequence of reading and writing data to verify the memory and one-time programmable memory are not corrupted.

When the ASIC 5000 enters the measurement mode for the first time, an insertion detection sequence is performed to verify that the sensor control device 102 has been properly installed onto the patient's body before a proper measurement can take place. First, the sensor control device 102 interprets a command to activate the measurement configuration process, causing the ASIC 5000 to enter measurement command mode. The sensor control device 102 then temporarily enters the measurement lifecycle state to run a number of consecutive measurements to test whether the insertion has been successful. The communication module 5040 or ASIC 5000 evaluates the measurement results to determine insertion success. When insertion is deemed successful, the sensor control device 102 enters a measurement state, in which the sensor control device 102 begins taking regular measurements using sensing hardware 5060. If the sensor control device 102 determines that the insertion was not successful, sensor control device 102 is triggered into an insertion failure mode, in which the ASIC 5000 is commanded back to storage mode while the communication module 5040 disables itself.

FIG. 1B further illustrates an example operating environment for providing over-the-air ("OTA") updates for use with the techniques described herein. An operator of the analyte monitoring system 100 can bundle updates for the data receiving device 120 or sensor control device 102 into updates for an application executing on the multi-purpose device 130. Using available communication channels between the data receiving device 120, the multi-purpose device 130, and the sensor control device 102, the multi-purpose device 130 can receive regular updates for the data receiving device 120 or sensor control device 102 and initiate installation of the updates on the data receiving device 120 or sensor control device 102. The multi-purpose device 130 acts as an installation or update platform for the data receiving device 120 or sensor control device 102 because the application that enables the multi-purpose device 130 to communicate with an sensor control device 102, data receiving device 120 and/or remote application server 155 can update software or firmware on a data receiving device 120 or sensor control device 102 without wide-area networking capabilities.

As embodied herein, a remote application server 155 operated by the manufacturer of the sensor control device 102 and/or the operator of the analyte monitoring system 100 can provide software and firmware updates to the devices of the analyte monitoring system 100. In some examples, the remote application server 155 can provides the updated software and firmware to a user device 145 or directly to a multi-purpose device. As embodied herein, the remote application server 155 can also provide application software updates to an application storefront server 160 using interfaces provided by the application storefront. The multi-purpose device 130 can contact the application storefront server 160 periodically to download and install the updates.

After the multi-purpose device 130 downloads an application update including a firmware or software update for a data receiving device 120 or sensor control device 102, the data receiving device 120 or sensor control device 102 and multi-purpose device 130 establish a connection. The multi-purpose device 130 determines that a firmware or software update is available for the data receiving device 120 or sensor control device 102. The multi-purpose device 130 can prepare the software or firmware update for delivery to the data receiving device 120 or sensor control device 102. As an example, the multi-purpose device 130 can compress or segment the data associated with the software or firmware update, can encrypt or decrypt the firmware or software update, or can perform an integrity check of the firmware or software update. The multi-purpose device 130 sends the data for the firmware or software update to the data receiving device 120 or sensor control device 102. The multi-purpose device 130 can also send a command to the data receiving device 120 or sensor control device 102 to initiate the update. Additionally or alternatively, the multi-purpose device 130 can provide a notification to the user of the multi-purpose device 130 and include instructions for facilitating the update, such as instructions to keep the data receiving device 120 and the multi-purpose device 130 connected to a power source and in close proximity until the update is complete.

The data receiving device 120 or sensor control device 102 receives the data for the update and the command to initiate the update from the multi-purpose device 130. The data receiving device 120 can then install the firmware or software update. To install the update, the data receiving device 120 or sensor control device 102 can place or restart itself in a so-called "safe" mode with limited operational capabilities. Once the update is completed, the data receiving device 120 or sensor control device 102 re-enters or resets into a standard operational mode. The data receiving device 120 or sensor control device 102 can perform one or more self-tests to determine that the firmware or software update was installed successfully. The multi-purpose device 130 can receive the notification of the successful update. The multi-purpose device 130 can then report a confirmation of the successful update to the remote application server 155.

In some examples, the storage memory 5030 of the sensor control device 102 includes one-time programmable (OTP) memory. The term OTP memory can refer to memory that includes access restrictions and security to facilitate writing to particular addresses or segments in the memory a predetermined number of times. The memory 5030 can be prearranged into multiple pre-allocated memory blocks or containers. The containers are pre-allocated into a fixed size. If storage memory 5030 is one-time programming memory, the containers can be considered to be in a non-programmable state. Additional containers which have not yet been written to can be placed into a programmable or writable state. Containerizing the storage memory 5030 in this fashion can improve the transportability of code and data to be written to the storage memory 5030. Updating the software of a device (e.g., the sensor device described herein) stored in an OTP memory can be performed by superseding only the code in a particular previously-written container or containers with updated code written to a new container or containers, rather than replacing the entire code in the memory. In a second example, the memory is not prearranged. Instead, the space allocated for data is dynamically allocated or determined as needed. Incremental updates can be issued, as containers of varying sizes can be defined where updates are anticipated.

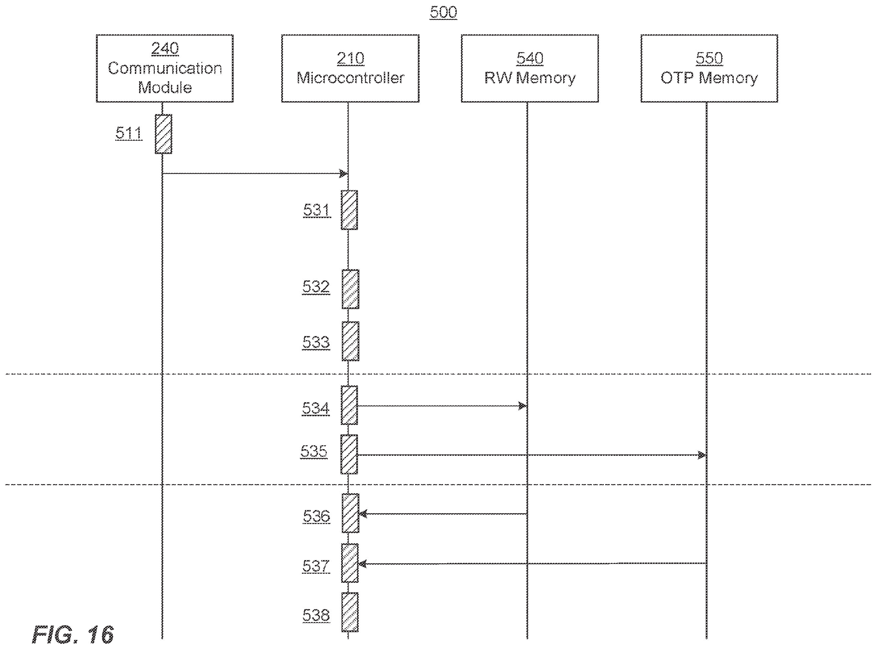

FIG. 16 is a diagram illustrating an example operational and data flow for over-the-air (OTA) programming of a storage memory 5030 in a sensor control device 102 as well as use of the memory after the OTA programming in execution of processes by the sensor device 110 according to the disclosed subject matter. In the example OTA programming 500 illustrated in FIG. 5, a request is sent from an external device (e.g., the data receiving device 130) to initiate OTA programming (or re-programming). At 511, a communication module 5040 of a sensor device 110 receives an OTA programming command. The communication module 5040 sends the OTA programming command to the microcontroller 5010 of the sensor device 110.

At 531, after receiving the OTA programming command, the microcontroller 5010 validates the OTA programming command. The microcontroller 5010 can determine, for example, whether the OTA programming command is signed with an appropriate digital signature token. Upon determining that the OTA programming command is valid, the microcontroller 5010 can set the sensor device into an OTA programming mode. At 532, the microcontroller 5010 can validate the OTA programming data. At 533, The microcontroller 5010 can reset the sensor device 110 to re-initialize the sensor device 110 in a programming state. Once the sensor device 110 has transitioned into the OTA programming state, the microcontroller 5010 can begin to write data to the rewriteable memory 540 (e.g., memory 5020) of the sensor device at 534 and write data to the OTP memory 550 of the sensor device at 535 (e.g., storage memory 5030). The data written by the microcontroller 5010 can be based on the validated OTA programming data. The microcontroller 5010 can write data to cause one or more programming blocks or regions of the OTP memory 550 to be marked invalid or inaccessible. The data written to the free or unused portion of the OTP memory can be used to replace invalidated or inaccessible programming blocks of the OTP memory 550. After the microcontroller 5010 writes the data to the respective memories at 534 and 535, the microcontroller 5010 can perform one or more software integrity checks to ensure that errors were not introduced into the programming blocks during the writing process. Once the microcontroller 5010 is able to determine that the data has been written without errors, the microcontroller 5010 can resume standard operations of the sensor device.

In execution mode, at 536, the microcontroller 5010 can retrieve a programming manifest or profile from the rewriteable memory 540. The programming manifest or profile can include a listing of the valid software programming blocks and can include a guide to program execution for the sensor control device 102. By following the programming manifest or profile, the microcontroller 5010 can determine which memory blocks of the OTP memory 550 are appropriate to execute and avoid execution of out-of-date or invalidated programming blocks or reference to out-of-date data. At 537, the microcontroller 5010 can selectively retrieve memory blocks from the OTP memory 550. At 538, the microcontroller 5010 can use the retrieved memory blocks, by executing programming code stored or using variable stored in the memory.

As embodied herein a first layer of security for communications between the sensor control device 102 and other devices can be established based on security protocols specified by and integrated in the communication protocols used for the communication. Another layer of security can be based on communication protocols that necessitate close proximity of communicating devices. Furthermore certain packets and/or certain data included within packets can be encrypted while other packets and/or data within packets is otherwise encrypted or not encrypted. Additionally or alternatively, application layer encryption can be used with one or more block ciphers or stream ciphers to establish mutual authentication and communication encryption with other devices in the analyte monitoring system 100.

The ASIC 5000 of the sensor control device 102 can be configured to dynamically generate authentication and encryption keys using data retained within the storage memory 5030. The storage memory 5030 can also be pre-programmed with a set of valid authentication and encryption keys to use with particular classes of devices. The ASIC 5000 can be further configured to perform authentication procedures with other devices using received data and apply the generated key to sensitive data prior to transmitting the sensitive data. The generated key can be unique to the sensor control device 102, unique to a pair of devices, unique to a communication session between an sensor control device 102 and other device, unique to a message sent during a communication session, or unique to a block of data contained within a message.

Both the sensor control device 102 and a data receiving device 120 can ensure the authorization of the other party in a communication session to, for example, issue a command or receive data. In some examples, identity authentication can be performed through two features. First, the party asserting its identity provides a validated certificate signed by the manufacturer of the device or the operator of the analyte monitoring system 100. Second, authentication can be enforced through the use of public keys and private keys, and shared secrets derived therefrom, established by the devices of the analyte monitoring system 100 or established by the operator of the analyte monitoring system 100. To confirm the identity of the other party, the party can provide proof that the party has control of its private key.

The manufacturer of the sensor control device 102, data receiving device 120, or provider of the application for multi-purpose device 130 can provide information and programming necessary for the devices to securely communicate through secured programming and updates. For example, the manufacturer can provide information that can be used to generate encryption keys for each device, including secured root keys for the sensor control device 102 and optionally for the data receiving device 120 that can be used in combination with device-specific information and operational data (e.g., entropy-based random values) to generate encryption values unique to the device, session, or data transmission as need.

Analyte data associated with a user is sensitive data at least in part because this information can be used for a variety of purposes, including for health monitoring and medication dosing decisions. In addition to user data, the analyte monitoring system 100 can enforce security hardening against efforts by outside parties to reverse-engineering. Communication connections can be encrypted using a device-unique or session-unique encryption key. Encrypted communications or unencrypted communications between any two devices can be verified with transmission integrity checks built into the communications. Sensor control device 102 operations can be protected from tampering by restricting access to read and write functions to the memory 5020 via a communication interface. The sensor can be configured to grant access only to known or "trusted" devices, provided in a "whitelist" or only to devices that can provide a predetermined code associated with the manufacturer or an otherwise authenticated user. A whitelist can represent an exclusive range, meaning that no connection identifiers besides those included in the whitelist will be used, or a preferred range, in which the whitelist is searched first, but other devices can still be used. The sensor control device 102 can further deny and shut down connection requests if the requestor cannot complete a login procedure over a communication interface within a predetermined period of time (e.g., within four seconds). These characteristics safeguard against specific denial of service attacks, and in particular against denial of service attacks on a BLE interface.

As embodied herein, the analyte monitoring system 100 can employ periodic key rotation to further reduce the likelihood of key compromise and exploitation. A key rotation strategy employed by the analyte monitoring system 100 can be designed to support backward compatibility of field-deployed or distributed devices. As an example, the analyte monitoring system 100 can employ keys for downstream devices (e.g., devices that are in the field or cannot be feasibly provided updates) that are designed to be compatible with multiple generations of keys used by upstream devices.

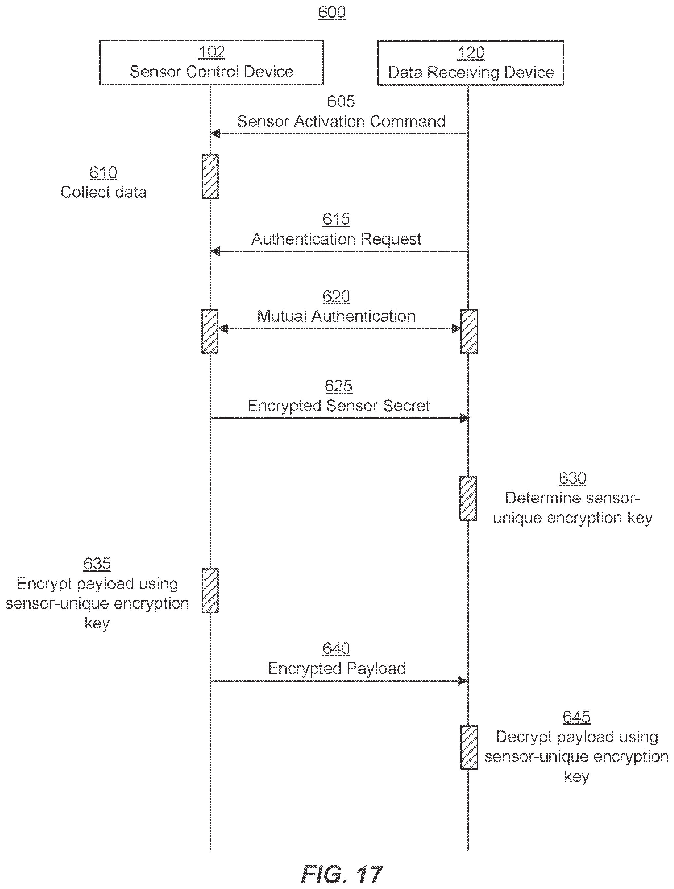

For purpose of illustration and not limitation, reference is made to the example of a message sequence diagram 600 for use with the disclosed subject matter as shown in FIG. 17 and demonstrating an example exchange of data between a pair of devices, particularly a sensor control device 102 and a data receiving device 120. The data receiving device 120 can, as embodied herein, be a data receiving device 120 or a multi-purpose device 130. A 605, the data receiving device 120 can transmit a sensor activation command 605 to the sensor control device 102, for example via a short-range communication protocol. The sensor control device 102 can, prior to 605 be in a primarily dormant state, preserving its battery until full activation is needed. After activation during 610, the sensor control device 102 can collect data or perform other operations as appropriate to the sensing hardware 5060 of the sensor control device 102. At 615 the data receiving device 120 can initiate an authentication request command 615. In response to the authentication request command 615, both the sensor control device 102 and data receiving device 120 can engage in a mutual authentication process 620. The mutual authentication process 620 can involve the transfer of data, including challenge parameters that allow the sensor control device 102 and data receiving device 120 to ensure that the other device is sufficiently capable of adhering to an agreed-upon security framework described herein. Mutual authentication can be based on mechanisms for authentication of two or more entities to each other with or without on-line trusted third parties to verify establishment of a secret key via challenge-response. Mutual authentication can be performed using two-, three-, four-, or five-pass authentication, or similar versions thereof.

Following a successful mutual authentication process 620, at 625 the sensor control device 102 can provide the data receiving device 120 with a sensor secret 625. The sensor secret can contain sensor-unique values and be derived from random values generated during manufacture. The sensor secret can be encrypted prior to or during transmission to prevent third-parties from accessing the secret. The sensor secret 625 can be encrypted via one or more of the keys generated by or in response to the mutual authentication process 620. At 630, the data receiving device 120 can derive a sensor-unique encryption key from the sensor secret. The sensor-unique encryption key can further be session-unique. As such, the sensor-unique encryption key can be determined by each device without being transmitted between the sensor control device 102 or data receiving device 120. At 635, the sensor control device 102 can encrypt data to be included in payload. At 640, the sensor control device 102 can transmit the encrypted payload 640 to the data receiving device 120 using the communication link established between the appropriate communication models of the sensor control device 102 and data receiving device 120. At 645, the data receiving device 120 can decrypt the payload using the sensor-unique encryption key derived at 630. Following 645, the sensor control device 102 can deliver additional (including newly collected) data and the data receiving device 120 can process the received data appropriately.

As discussed herein, the sensor control device 102 can be a device with restricted processing power, battery supply, and storage. The encryption techniques used by the sensor control device 102 (e.g., the cipher algorithm or the choice of implementation of the algorithm) can be selected based at least in part on these restrictions. The data receiving device 120 can be a more powerful device with fewer restrictions of this nature. Therefore, the data receiving device 120 can employ more sophisticated, computationally intense encryption techniques, such as cipher algorithms and implementations.

The sensor control device 102 can be configured to alter its discoverability behavior to attempt to increase the probability of the receiving device receiving an appropriate data packet and/or provide an acknowledgement signal or otherwise reduce restrictions that can be causing an inability to receive an acknowledgement signal. Altering the discoverability behavior of the sensor control device 102 can include, for example and without limitation, altering the frequency at which connection data is included in a data packet, altering how frequently data packets are transmitted generally, lengthening or shortening the broadcast window for data packets, altering the amount of time that the sensor control device 102 listens for acknowledgement or scan signals after broadcasting, including directed transmissions to one or more devices (e.g., through one or more attempted transmissions) that have previously communicated with the sensor control device 102 and/or to one or more devices on a whitelist, altering a transmission power associated with the communication module when broadcasting the data packets (e.g., to increase the range of the broadcast or decrease energy consumed and extend the life of the battery of the analyte sensor), altering the rate of preparing and broadcasting data packets, or a combination of one or more other alterations. Additionally, or alternatively, the receiving device can similarly adjust parameters relating to the listening behavior of the device to increase the likelihood of receiving a data packet including connection data.

As embodied herein, the sensor control device 102 can be configured to broadcast data packets using two types of windows. The first window refers to the rate at which the sensor control device 102 is configured to operate the communication hardware. The second window refers to the rate at which the sensor control device 102 is configured to be actively transmitting data packets (e.g., broadcasting). As an example, the first window can indicate that the sensor control device 102 operates the communication hardware to send and/or receive data packets (including connection data) during the first 2 seconds of each 60 second period. The second window can indicate that, during each 2 second window, the sensor control device 102 transmits a data packet every 60 milliseconds. The rest of the time during the 2 second window, the sensor control device 102 is scanning. The sensor control device 102 can lengthen or shorten either window to modify the discoverability behavior of the sensor control device 102.

In some examples, the discoverability behavior of the analyte sensor can be stored in a discoverability profile, and alterations can be made based on one or more factors, such as the status of the sensor control device 102 and/or by applying rules based on the status of the sensor control device 102. For example, when the battery level of the sensor control device 102 is below a certain amount, the rules can cause the sensor control device 102 to decrease the power consumed by the broadcast process. As another example, configuration settings associated with broadcasting or otherwise transmitting packets can be adjusted based on the ambient temperature, the temperature of the sensor control device 102, or the temperature of certain components of communication hardware of the sensor control device 102. In addition to modifying the transmission power, other parameters associated with the transmission capabilities or processes of the communication hardware of the sensor control device 102 can be modified, including, but not limited to, transmission rate, frequency, and timing. As another example, when the analyte data indicates that the subject is, or is about to be, experiencing a negative health event, the rules can cause the sensor control device 102 to increase its discoverability to alert the receiving device of the negative health event.

As embodied herein, certain calibration features for the sensing hardware 5060 of the sensor control device 102 can be adjusted based on external or interval environment features as well as to compensate for the decay of the sensing hardware 5060 during expended period of disuse (e.g., a "shelf time" prior to use). The calibration features of the sensing hardware 5060 can be autonomously adjusted by the sensor control device 102 (e.g., by operation of the ASIC 5000 to modify features in the memory 5020 or storage 5030) or can be adjusted by other devices of the analyte monitoring system 100.

As an example, sensor sensitivity of the sensing hardware 5060 can be adjusted based on external temperature data or the time since manufacture. When external temperatures are monitored during the storage of the sensors, the disclosed subject matter can adaptively change the compensation to sensor sensitivity over time when the device experiences changing storage conditions. For purpose of illustration not limitations, adaptive sensitivity adjustment can be performed in an "active" storage mode where the sensor control device 102 wakes up periodically to measure temperature. These features can save the battery of the analyte device and extend the lifespan of the analyte sensors. At each temperature measurement, the sensor control device 102 can calculate a sensitivity adjustment for that time period based on the measured temperature. Then, the temperature-weighted adjustments can be accumulated over the active storage mode period to calculate a total sensor sensitivity adjustment value at the end of the active storage mode (e.g., at insertion). Similarly, at insertion, the sensor control device 102 can determine the time difference between manufacture of the sensor control device 102 (which can be written to the storage 5030 of the ASIC 5000) or the sensing hardware 5060 and modify sensor sensitivity or other calibration features according to one or more known decay rates or formulas.

Additionally, for purpose of illustration and not limitation, as embodied herein, sensor sensitivity adjustments can account for other sensor conditions, such as sensor drift. Sensor sensitivity adjustments can be hardcoded into the sensor control device 102 during manufacture, for example in the case of sensor drift, based on an estimate of how much an average sensor would drift. Sensor control device 102 can use a calibration function that has time-varying functions for sensor offset and gain, which can account for drift over a wear period of the sensor. Thus, sensor control device 102 can utilize a function used to transform an interstitial current to interstitial glucose utilizing device-dependent functions describing sensor control device 102 drift over time, and which can represent sensor sensitivity, and can be device specific, combined with a baseline of the glucose profile. Such functions to account for sensor sensitivity and drift can improve sensor control device 102 accuracy over a wear period and without involving user calibration.

The sensor control device 102 detects raw measurement values from sensing hardware 5060. On-sensor processing can be performed, such as by one or more models trained to interpret the raw measurement values. Models can be machine learned models trained off-device to detect, predict, or interpret the raw measurement values to detect, predict, or interpret the levels of one or more analytes. Additional trained models can operate on the output of the machine learning models trained to interact with raw measurement values. As an example, models can be used to detect, predict, or recommend events based on the raw measurements and type of analyte(s) detected by the sensing hardware 5060. Events can include, initiation or completion of physical activity, meals, application of medical treatment or medication, emergent health events, and other events of a similar nature.

Models can be provided to the sensor control device 102, data receiving device 120, or multi-purpose device 130 during manufacture or during firmware or software updates. Models can be periodically refined, such as by the manufacturer of the sensor control device 102 or the operator of the analyte monitoring system 100, based on data received from the sensor control device 102 and data receiving devices of an individual user or multiple users collectively. In some examples, the sensor control device 102 includes sufficient computational components to assist with further training or refinement of the machine learned models, such as based on unique features of the user to which the sensor control device 102 is attached. Machine learning models can include, by way of example and not limitation, models trained using or encompassing decision tree analysis, gradient boosting, ada boosting, artificial neural networks or variants thereof, linear discriminant analysis, nearest neighbor analysis, support vector machines, supervised or unsupervised classification, and others. The models can also include algorithmic or rules-based models in addition to machine learned models. Model-based processing can be performed by other devices, including the data receiving device 120 or multi-purpose device 130, upon receiving data from the sensor control device 102 (or other downstream devices).

Data transmitted between the sensor control device 102 and a data receiving device 120 can include raw or processed measurement values. Data transmitted between the sensor control device 102 and data receiving device 120 can further include alarms or notification for display to a user. The data receiving device 120 can display or otherwise convey notifications to the user based on the raw or processed measurement values or can display alarms when received from the sensor control device 102. Alarms that can be triggered for display to the user include alarms based on direct analyte values (e.g., one-time reading exceeding a threshold or failing to satisfy a threshold), analyte value trends (e.g., average reading over a set period of time exceeding a threshold or failing to satisfy a threshold; slope); analyte value predictions (e.g., algorithmic calculation based on analyte values exceeds a threshold or fails to satisfy a threshold), sensor alerts (e.g., suspected malfunction detected), communication alerts (e.g., no communication between sensor control device 102 and data receiving device 120 for a threshold period of time; unknown device attempting or failing to initiate a communication session with the sensor control device 102), reminders (e.g., reminder to charge data receiving device 120; reminder to take a medication or perform other activity), and other alerts of a similar nature. For purpose of illustration and not limitation, as embodied herein, the alarm parameters described herein can be configurable by a user or can be fixed during manufacture, or combinations of user-settable and non-user-settable parameters.

As discussed herein, data receiving devices 120 (e.g., a "receiving devices") are often intentionally designed to limit their cost and interoperability. Data receiving devices 120 often limit processor power and the types of wireless communications hardware included therein. In particular, data receiving devices 120 are often not configured to communicate on a wide area network and so lack WiFi-compatible hardware. Instead, data receiving devices 120 are configured only to communicate wirelessly with sensor control devices 102 to preserve security. While multi-purpose devices 130 (e.g., smartphones, tablets, smartwatches, etc.) are increasing in popularity as secondary options, data receiving devices 120 used by a large percentage of patients who wear analyte sensors.

To communicate data to other user devices 140 or to an application server 155, a physical wired (e.g., USB) connection is established between the data receiving device 120 and the user device 140. The user device 140 can then optionally relay relevant information to the application server 155. Software executing on the user device 140 or the application server 155 can interpret the received data and, for example, generate actionable reports based on the analyte data included therein.

Analyte reports can be generated by, or on behalf of, a patient or HCP from software executing on the HCP's user device 140 (e.g., a computer terminal in their office) or via a remote, web-based application. For reports to be generated based on up-to-date information from a data receiving device 120, the patient must periodically connect the data receiving device 120 to an internet-connected device through a wired connection. However, as described herein, it can be cumbersome and inconvenient to provide the wired connection between the data receiving device 120 and the user device 140. The user device 140 must have a data interface software driver that can retrieve the data from the data receiving device 120 and transfer it to the reporting software. When drivers associated with the data receiving device 120 or the user device 140 are updated, the drivers must be acquired before a secured connection can be established, further delaying report generation. Due to a lack of interest or knowledge, many patients do not go through the trouble of creating an account for web-based reporting software, or uploading their latest data via an internet connected device. Similarly, many HCPs do not or create an HCP account in report generating software. Subsequently, for many patients who use analyte sensors, HCPs are not able to make efficient, optimal therapy decisions based on up-to-date information from the user's sensor control device 102.

This problem can be particularly acute for users who do not regularly connect a data receiving device 120 to their user device 140. This can cause the drivers to facilitate a secured connection between the data receiving device 120 and the user device 140 to be out of date. For example, non-specialist HCPs can wish to review overall trends in the analyte levels of their patients, but do not frequently see patients who use a data receiving device 120. The non-specialist HCP can only use a user device 140 to review data from a data receiving device 120 when certain patients visit. As another example, certain patients can be generally satisfied with using a data receiving device 120 to review day-to-day information from a sensor control device 102 and therefore only connect their data receiving device 120 to a user device 140 when requested by an HCP.

HCPs, particularly non-specialist HCPs face a variety of challenges in accessing their patients' analyte data and particularly reports generated based on analyte data. These challenges are a major barrier to the utility of continuous analyte monitors, such as continuous glucose monitors, in informing treatment decisions for patients. Many analyte reporting tools require both the patient and HCP to each register accounts, each requiring a username and password, with an application server 155 associated with the reporting tool. The patient must then enter account credentials in order for data from their data receiving device 120 to be uploaded to the application server 155 for processing. The patient must also provide permission to the HCP to link their patient record with the HCP account. This requires both parties to remember their account credentials.

To address these barriers, disclosed herein are techniques for enabling transfer of information to assist with report generation from a data receiving device 120 that do not require a physical data connection with the data receiving device 120 or an account (for either the HCP or the patient) for report generation software from an application server 155. Techniques directed to alternative methods and procedures to retrieve analyte data from a data receiving device 120 further simply procedures for primary care providers (PCPs) and other HCPs to view analyte data and analyses based on analyte data without requiring the PCP to create a separate account, remember a username and password, or engage additional communication channels to request patients to provide the data. Described herein are a variety of systems and techniques for enabling HCPs to easily access their patients' analyte reports. Of particular benefit is that the systems and techniques described herein do not require the additional overhead of having to manage new or unique accounts and data storage requirements. This allows for the techniques to be used with and tailored to the specific clinical application or electronic medical record (EMR) system. These techniques can be extended to the application of configuring EMR systems to access analyte data where similar challenges exist, such as requiring a patient to remember and provide access credentials to permit an EMR to access a patient's data on a one-time or repeating basis.

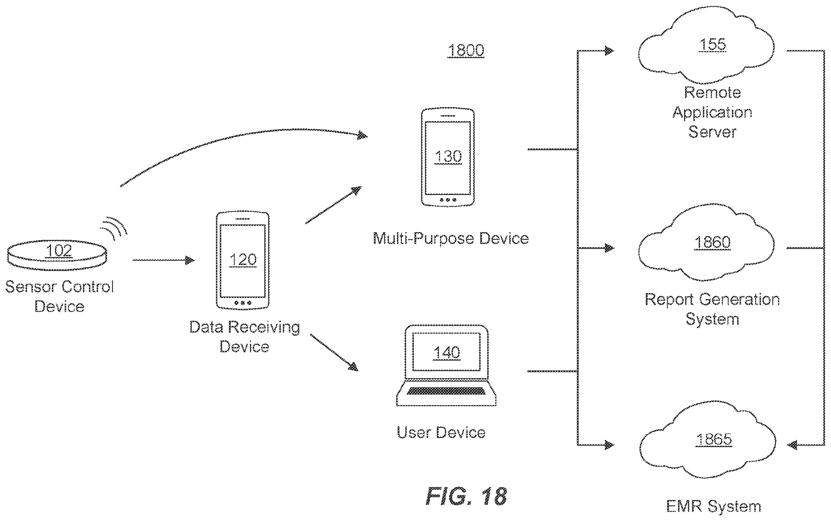

FIG. 18 illustrates an example environment and dataflow for an analyte monitoring system 1800 configured to provide accessible analyte data reports and cross-system integration. The sensor control device 102, configured as described herein, measures levels of one or more analytes of interest of a patient and provides the analyte levels (or signals from which the analyte levels can be derived) to a data receiving device 120. In some examples, the sensor control device 102 can additionally or alternatively provide the analyte levels to a multi-purpose device 130.

The data receiving device 120 is a low-cost proprietary computing device configured to communicate with the sensor control device 102 and provide basic reporting functionality regarding the patient's health. The data receiving device 120 is generally not capable for wireless communication with other generic devices. The data receiving device 120 can be connected to multi-purpose devices 130 or user devices 140 through wired connections or certain limited functionality communication mechanisms.

Multi-purpose devices 130 and user devices 140 include general purpose computing devices. The computing devices are executing software applications or other bundles of executable programming code that are configured to enable the computing devices to communicate with devices within the analyte monitoring system 1800. Multi-purpose devices 130 and user devices 140 can typically perform some processing of analyte data to produce certain forms of reports or recommendations. Multi-purpose devices 130 and user devices 140 can also relay (processed or raw) data from the sensor control device 102 to a variety of remote systems.

The remote systems include, by way of example and not limitation, a remote application server 155 associated with the analyte monitoring system 1800, a report generation system 1860 associated with the analyte monitoring system 1860, and, optionally, an EMR system 1865, which can be integrated with the analyte monitoring system 1860. The remote application server 155 can provide for advanced data processing based on individual patient data or patient data from a larger population. The report generation system 1860 can generate reports based on provided analyte data. The reports can be patient identifying, semi-anonymous, or fully anonymous based on the preferences of the user requesting the generation of the report and the quality of the data. The EMR system 1865 is generally a medical records system administered by or on behalf of an HCP. The EMR system 1865 can, under examples described herein, be integrated with the analyte monitoring system 1800 such that it can automatically receive patient analyte data and reports to facilitate treatment and therapy decisions by HCPs.

As described herein, a multi-purpose device 130 can include a user's smartphone or other device with wide area networking capabilities. The multi-purpose device 130 can have installed on it an application associated with the analyte monitoring system 100 that enables the multi-purpose device 130 to communicate with other devices within the analyte monitoring system 100, including the sensor control device 102 and remote application server 155. The application can provide for a variety of additional features.

According to some examples, the application can provide for a feature to facilitate sharing access to analyte data that the multi-purpose device 130 has uploaded to a remote application server 155 and reports generated based on the analyte data. In a user interface of the application, this feature can be labeled as a "share reports" feature. When the user activates the feature, a unique access code is displayed along with an access URL.

In some examples, the unique access code can be randomly generated on demand and in response to the user's request. In some examples, the application generates the access code, however in other examples, the access code is generated by, for example, the remote application server 155 or report generation system 1860 and provided to the application. When the application generates the access code, the multi-purpose device 130 sends an encrypted message with the access code to the report generation system 1860. In some examples, the encrypted message can include additional information, such as analyte data stored by the multi-purpose device 130 and received from the sensor control device 102. Alternatively, the multi-purpose device 130 can include information to identify the patient associated with the multi-purpose device 130. The report generation system 1860 can use the identifying information to retrieve recent analyte data from the application server 155. The report generation system 1860 stores the access code in association with the analyte data or any pre-generated reports.

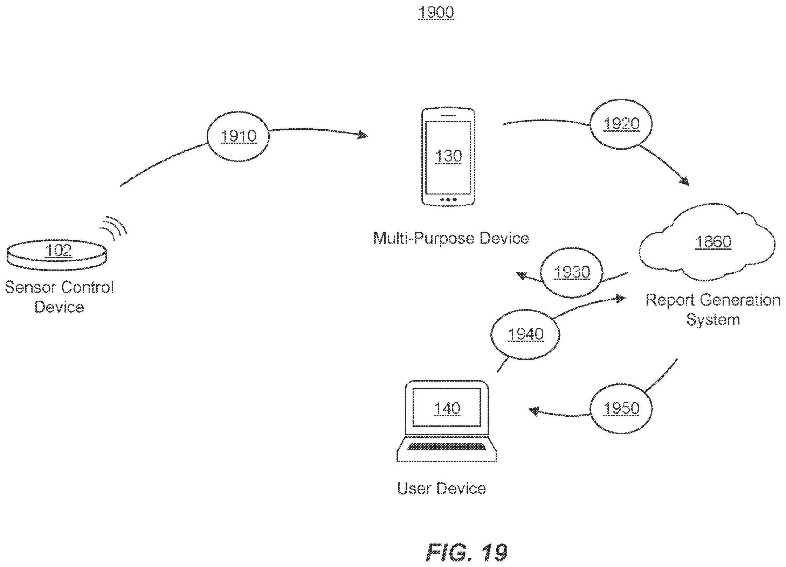

FIG. 19 illustrates an example flow of data between the components of the analyte monitoring system 1900 configured to produce and provide accessible reports. At 1910 the sensor control device 102 provide analyte data to the multi-purpose device 130. As described herein, the sensor control device 102 can provide the data on a continuous, real-time or near-real-time, basis when a connection between the sensor control device 102 and the multi-purpose device 130 are available. Additionally or alternatively, the sensor control device 102 can provide the data on demand from the multi-purpose device 130.

At 1920, the multi-purpose device 130 initiates a request to share reports based on the analyte data with the report generation system 1860. The request can include the latest analyte data from the sensor control device 102 including a predetermined preceding period of time (e.g., 14 days). The amount of data included can be user set, such as by the patient or their HCP. In some examples, the request can, additionally or alternatively, include a reference to analyte data stored in another system, such as a remote application server 155 of the analyte monitoring system 1800.

Based on the analyte data received from the multi-purpose device 130, or retrieved from the remote application server 155 at the request of the multi-purpose device 130, the report generation system 1860 can generate a report for the user. The report can include information such as trends based on the analyte data, trends observed in similar patients or the general population, therapy modification suggestions, and a wide variety of other information. At 1930, the report generation system 1860 can send the report to the multi-purpose device 130 for viewing by the user of the multi-purpose device 130. Additionally or alternatively, the report generation system can provide a unique access code and access URL, which can be used by another user of another user device 140 to view the report. In some examples, the multi-purpose device 130 can generate the access code and provide the access code to the report generation system 1860 during 1920 or during an additional transmission.

At 1940, a user device 140 initiates a request to view the report associated with the analyte data from the sensor control device 102. The user device 140 can navigate, using a web browser or other suitable application, to the access URL. The user of the user device 140 can input the access code to the website, which can be served by the report generation system 1860, a sub-system thereof, or a related website server. The report generation system 1860 can use the access code to identify the analyte data from the sensor control device 102 or pre-generated reports based on the analyte data, if available. The report generation system 1860 can search a database storing the analyte data in association with valid access codes. Upon locating a valid record, at 1950 the report generation system 1860 provides the report to the user device 140 for review.

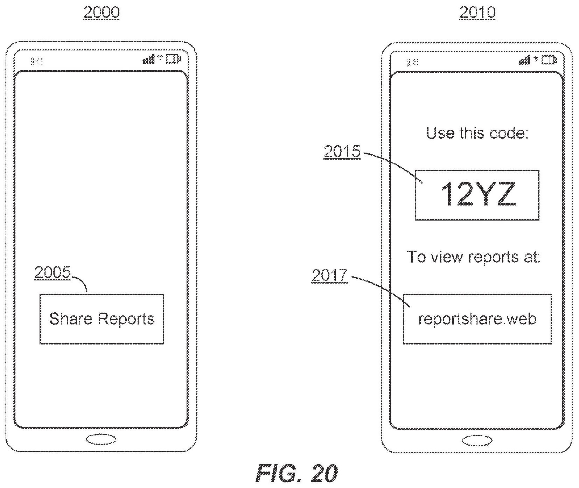

FIG. 20 illustrates example user interfaces of an application executing according to certain embodiments. In particular, FIG. 20 illustrates user interfaces of an application executing on a multi-purpose device 130. Interface 2000 includes a number of options for accessing data and local reports collected by the multi-purpose device 130 from a sensor control device 102. Included among the options is an interactive element 2005 (e.g., an interface button) labeled "Share Reports." When the user selects interactive element 2005, the multi-purpose device performs the routines described above for collecting analyte data associated with a preceding time period and sending the up-to-date analyte data to a report generation system 1860. The multi-purpose device 130 also generates, or receives, an access code associated with their reports. Interface 2010 includes a display element 2015 for the access code, in this case "12YZ." Interface 2010 also includes a display element 2017 instructing a viewer to visit a particular website in order to view the associated reports.

As described herein, in some examples, the multi-purpose device 130 can have a constant connection to a remote application server 155 or the report generation system 1860. When the user interacts with the interactive element 2005, the multi-purpose device 130 can request that data for a relevant period of time be made available. The period of time can be user selected, e.g., based on information included with the request to share reports, or can be automatically determined, e.g., based on the time span of data available about the user. Additionally, in some examples, the remote application server 155 or report generation system 1860 can generate the access code and send the access code to the multi-purpose device 130 to confirm that the report is available.

At a later point, the report generation system 1860 receives a request for a report based on patient data from a user device 140. The request includes an access code. The report generation system 1860 searches its records of patient data and/or reports and identifies the corresponding information based on the access code. The report generation system 1860 can then provide the corresponding report to the user device 140 that has requested the report.

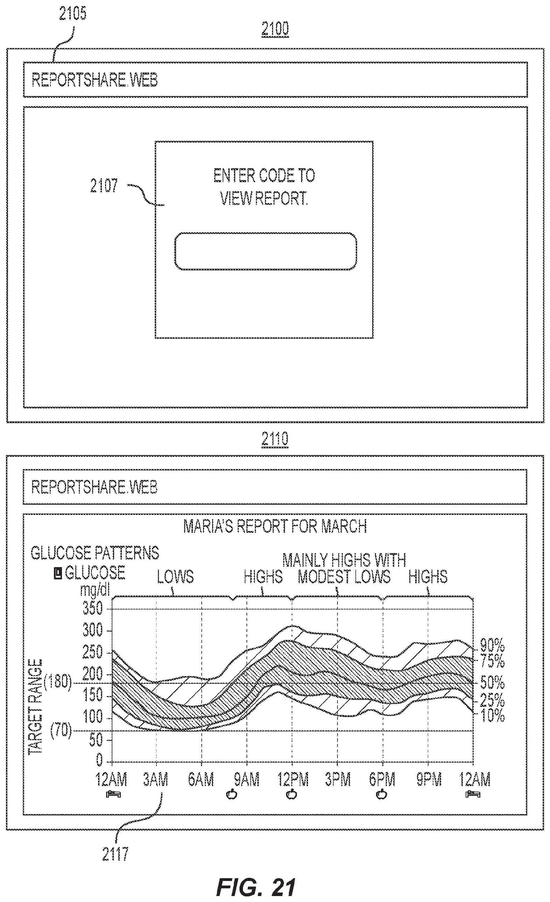

FIG. 21 illustrates example user interfaces of another device according to certain embodiments. In particular, FIG. 21 illustrates user interfaces of an application or web browser executing on a user device 140, which can be used by another user such as an HCP. Interface 2100 includes a web browser executing on the user device 140. As illustrated by the address bar 2105, the user of the user device 140 (e.g., the HCP) has navigated to the website provided in interface 2010. The website includes a user input prompt 2107 requesting the user to enter a code to review the corresponding report. After entering the code, e.g., the code shown in interface 2010, the report generation system 1860 receives the code and searches an associated database for the corresponding analyte data or pre-generated report. If there is no pre-generated report for the corresponding analyte data, then the report can be generated on-demand based on the corresponding analyte data. Interface 2110 illustrates an example presentation of the report 2117 for the user of the multi-purpose device 130. As described herein, the report can include a variety of information and can be presented in a variety of forms based on user preference and the capabilities and features of the user device 140. If there is no report corresponding to the code input by the user, or if the code is no longer valid, interface 2117 can instead show an error indicating that no data is available or that the code has expired.

As described herein, the access code can be limited in terms of the number of uses, the time of use, or other contextual information. As an example, an access code can be set to only be usable for 15 minutes, after which point, the access code will expire. As another example, the access code can be set to only be usable by devices within a specific geographic area or within a specified distance of the multi-purpose device 130 when the user requests to share their reports. When the other user device 140 submits the request including the access code, the approximate location of the user device can be determined and included with the request. Sensible limitations to when and how often reports can be retrieved improve the security of patient data using the techniques described herein.

In some examples, the application executing on the multi-purpose device 130 has an on-going connection with the remote application server 155 or the report generation system 1860. Through this connection the multi-purpose device 130 continuously uploads data received from the sensor control device 102 worn by the user. The data is stored in one or more databases for the user. In some examples, the database can store the analyte data in or associated with an account registered by the patient. For example, the patient can register a username or user identifier and password, while can be required to access the data. As another example, the user can store the analyte data in a de-identified fashion using an anonymous account. In such examples, the remote application server 155 or report generation system 1860 can recognize an identifier for the multi-purpose device 130 (e.g., a serial number, IMEI, MAC address, etc.) or an identifier for the application instance of the application executing on the multi-purpose device 130. The remote application server 155 can associate analyte data from the same recognized identifier in the database. In this example, when the patient requests to share a report, the access code is generated, shared between the multi-purpose device 130 and the repot generation system 1860, and displayed. The analyte data is not sent from the multi-purpose device to the report generation system 1860 on-demand.

As described herein, proprietary data receiving devices 120 (e.g., "receiving devices") can be provided to users in the analyte monitoring system 100. The data receiving devices 120 can be of generally limited functionally, including the hardware and programming to communicate with the sensor control device 120, but often lacking the ability to communicate using a wide area network. Instead, to upload analyte data to an application server 155 for additional storage and processing, the data receiving device 120 can be connected to a user device 140 through a wired connection. The user device 140 can store data for the data receiving device 120 and can further upload the analyte data to the remote application server 155. While the low costs of data receiving devices 120 can make them convenient to provide to users, the inability to easily transfer data stored on the data receiving device 120 to another user, such as an HCP, on demand limits the usefulness of the analyte data viewed by the HCP when making therapy decisions. It would therefore be beneficial to provide for simplified processes to transfer analyte data and other information from a data receiving device 120 to a user device 140 (such as the user device of an HCP in a clinical setting) without a direct wired or wireless connection. It would particularly be beneficial to enable the HCP to view robust reports based on the analyte data, similar to the reports that are available through the above-described "share reports" feature provided through multi-purpose device 130.

Pursuant to the techniques described herein, the software executing on the data receiving device 120 can be modified to provide a "share data" function that can be accessed, for instance, from a setup menu. Rather than upload data to a remote server (because the data receiving device 120 cannot connect with the remote server), when selected by the patient, the share data function causes the data receiving device 120 to generate and display a specially configured matrix barcode (e.g., a Quick Response (QR) code). This matrix barcode is generated to include a URL defining the internet location of a web server associated with a report generation system 1860 that can initiate a request to generate a report based on analyte data provided with the request. The matrix barcode can also include a sequence of encoded analyte data values that can be provided as parameters passed with the URL.

Special encodings, described herein, enable a large amount of data to be included in the matrix barcode that balances the ability of a user to interact with and use the matrix barcode while still providing sufficient data to provide useful information when converted into a report. For example, analyte value readings can represent values for every 15 minutes for a period of 14 days.

Once the data receiving device 120 generates the matrix barcode, another user can use a multi-purpose device 130 to scan the matrix barcode. Many modern smartphones, for example, include a camera that can automatically recognize and decode simple matrix barcodes. The patient or HCP user can use a camera feature native in their smartphone or user device 140 to scan the matrix barcode. The smartphone or user device 140 can direct a web browser to the website server 1860 identified by the encoded URL. The website server 1860 includes a report generation function that decodes the encoded analyte data and generate an analyte report based on these data. The website server 1860 will send the report back to the web browser for display on the multi-purpose device 130 or user device 140. In some examples, no identifying information is encoded within the matrix barcode, meaning that analyte level report security is non-identifiable and protected through requiring physical access to the matrix barcode to be actionable.

If the HCP wishes to view the report on another device, such as a user device 140, the report generation system 1855 can generate an access code, associate the access code with the analyte data in a database, and display the access code on the multi-purpose device 130 along with a URL through which the HCP can access the corresponding reports. As in the examples described previously, using the user device 140, the HCP visits the report viewing website, provides the access code displayed by the multi-purposes data receiving device 130 and receiving the report from the report generation system 1860.

In some examples, for added security, the website server 1860 can generate the access code and include this in the glucose report that is displayed on the multi-purpose device 130 along with another URL identifying another website. The access code can be, for example, a random alphanumeric code. The HCP can access this second website from a web browser on their internet-connected user device 140 or any other internet connected devices by entering the URL into the browser URL field. The second website can also be provided by the same provider as the first website. The second website can request the HCP to enter the access code by causing a code entry field to be displayed on user device 140 web browser. Upon entering the code and indicating submit, the second website will receive the access code and generate analyte report(s) with the patient's data for display on the HCP's user device 140 web browser.

In some examples, the report generation system 1860 can generate the access code for the first and second websites. In some examples, the access code can be generated by the data receiving device 120, e.g., as part of the process for generating the matrix barcode. The access code can be associated with reports (if generated upon receiving the analyte data) or analyte data used for generating reports on demand. The access code can be used on a permanent or semi-permanent basis by the user of the data receiving device 120, such as to facilitate consistent sharing of data. Alternatively, the access code can be temporarily associated with the reports or analyte data. The association can be removed after the expiration of a predetermined or user-provided amount of time, after the report has been accessed a specified number of times (e.g., to facilitate sharing the same report with multiple other users), or upon request by the user of the data receiving device 120 or the user who has accessed the report. The code can take any form, for instance a sequence of alphanumeric digits such as "AB12," or can include other symbols, or pictograms (e.g., emojis).

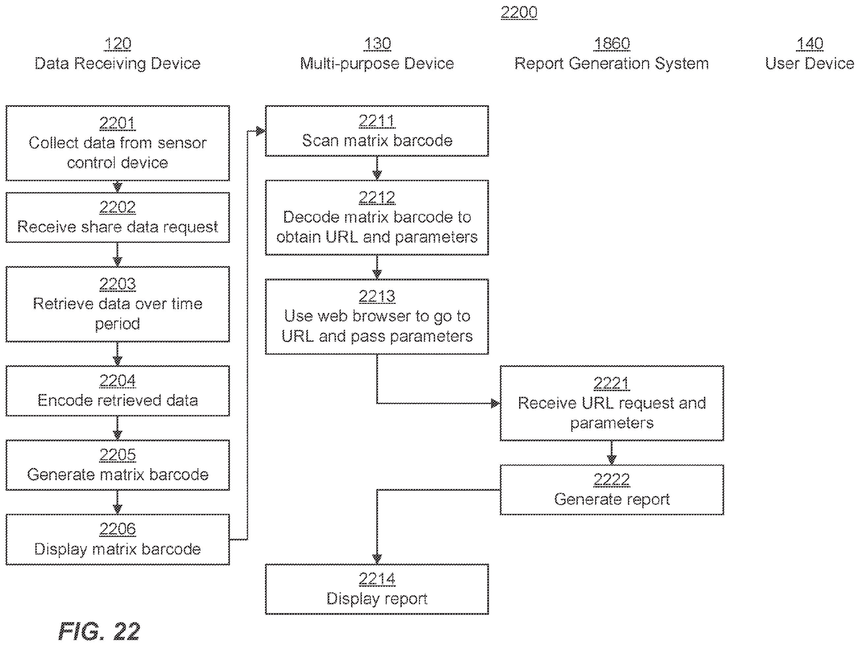

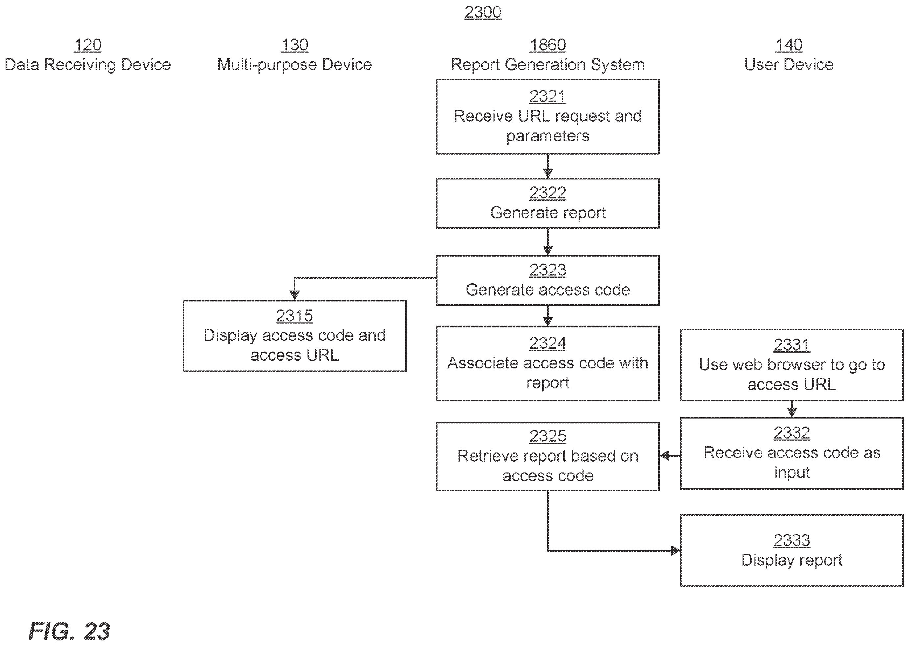

FIGS. 22 and 23 illustrate a first procedure 2200 and a second procedure 2300 for requesting and generating a report based on analyte data provided via a matrix barcode. The process begins at 2201, when the data receiving device 120, also referred to as a receiving device, collects analyte data from a sensor control device 102. The data receiving device 120 can be part of an analyte monitoring system 100, for example, a glucose monitoring system. The data receiving device 120 can comprise a display, one or more processors, and a memory communicatively coupled with the one or more processors. The memory can comprise instructions that, when executed by the one or more processors are configured to cause the one or more processors to perform operations comprising one or more operations of the first procedure 2200, for example operations 2201 to 2206.

At 2202, the data receiving device 120 receives a request to share or offload analyte data. The request can be made, for example, through a user of the data receiving device 120 selecting an interactive element provided on a user interface of the data receiving device 120.

At 2203, the data receiving device 120 retrieves analyte data associated with a particular time period of interest. The data receiving device 120 can be configured to automatically identify data for a predetermined period of time (e.g., the last 14 days, 7 days, 24 hours, etc.). In some examples, the request received at 2202 can specify the time period of interest.

At 2204 the data receiving device 120 encodes the retrieved data. Prior to generating the matrix barcode associated with the analyte data, the analyte data can be encoded. Encoded analyte data can be achieved by formatting the data to be compatible with the parameters provided to the report generation system 1860 so that the report generation system 1860 can generate the report. As an example, the encoded data can be made up of a datestamp and/or timestamp associated with each analyte data value associated with the analyte data. The encoded data can include a single datestamp and/or timestamp followed by a series of analyte data values that are interpreted as being provided at regular intervals (e.g., every 5 minutes, 15 minutes, 30 minutes etc.). This encoding reduces the need to include explicit timing information for each analyte data value when not necessary.

In some examples, other data can be encoded and included in a matrix barcode. As an example, elements to be included in an analyte report can be generated an encoded. Elements to be included in the analyte report can include, for example, a percentile line data calculated from the analyte data or other analyte data metrics. In some examples, the elements to be included can be based on the type of the analyte being tracked. For example, where the analyte is glucose, the encoded data can include a time in hypoglycemic range, a time in hyperglycemic range, and a time of euglycemic range. The encoded data can include derivative values calculated from the analyte data. In some examples, the other data encoded and included in the matrix barcode can include an identifier for the data receiving device 120, an identifier for the patient, an identifier for the software executing on the data receiving device 120, or other ways to uniquely identify the data encoded in the matrix barcode.

When generating matrix barcodes, the complexity of the barcode scales with the density of the data included in the matrix barcode. The more information, particularly the number of bytes, one attempts to include in the matrix barcode, the more complex the barcode becomes. The size of the matrix barcode can be increased to enable more information, but at a certain point, the limit is based on the display resolution of the device displaying the matrix barcode (e.g., the data receiving device 120) and the fidelity of the camera used to scan the matrix barcode. Therefore, additional encodings can be used to improve the balance of information density in the barcode.

One approach to reduce the information density is to reduce the period of time that is covered by the analyte data included with the matrix barcode. Another approach to reduce the information density is to reduce the frequency of the analyte data within the series (e.g., analyte data values corresponding to every 30 minutes instead of every 15 minutes). These solutions are simple and do not require additional computational power or time to use. However, they also reduce the amount of useful data provided to the user who views the ensuing report (e.g., the HCP).

To reduce the information density but maintain parameters such as the duration and frequency of the included data, different forms of data compression can be used. As one example, the dynamic range (e.g., precision) of the analyte data values can be reduced in a context aware fashion. One such data compression approach can be to round the analyte data values. For example, analyte data values can be stored by the data receiving device with precision up to 0.1 mg/dL. Before encoding the data values into a matrix barcode, the data can be rounded or truncated to a precision of up to 1 mg/dL. As another example, analyte data values can be rounded to the nearest 5 mg/dL (or 10 mg/dL or other appropriate values).

In some examples, rounding can be performed to round different levels of precision based on the original value of the analyte data value itself. Specifically, for an analyte where values higher than a threshold are considered more important to monitor than values below the threshold, analyte data values above the threshold can be rounded to a more precise value. The reverse can be true for analyte data values where lower values are considered more important to monitor. Multiple thresholds and multiple levels of rounding precision can be used to scale the rounding precision accordingly. Additionally, the threshold values can be pre-programmed or can be set by the user of the data receiving device 120 or the user who reviews the reports (e.g., an HCP) based on the individual user's need or preferences.

As an example, where the analyte is or correlates to glucose values, it can be preferable to have more resolute glucose values for lower glucose values than for higher glucose values. When decoding the glucose series, approximate resolution to 1 mg/dL can be partially recovered by using spline techniques on the series and rounding the resulting glucose values to the nearest 1 mg/dL.

An example of a multi-threshold rounded scheme is given below:
For glucose values from 40-100: round to nearest 2 mg/dL
For glucose values from 101-180: round to nearest 3 mg/dL
For glucose values from 181-280: round to nearest 4 mg/dL
For glucose values from 281-400: round to nearest 5 mg/dL

When rounding the analyte data used in the matrix barcode metrics calculated by the report generation system 1860 for inclusion in the report are calculated using the rounded data. The values of metrics included in reports calculated using analyte values with a higher precision can differ from values of metrics calculated using analyte values with a rounded precision. Therefore, the value of the metrics shown in a report can differ slightly from the values that are presented on the data receiving device 120. This can cause the user or HCP to question the accuracy of the data, be distracted by the difference, or even to change therapy recommendations based on the less accurate data. To address this issue, the input data to the matrix barcode can further include metrics calculated based on the original analyte data (e.g., with full resolution) to be used in subsequently generated reports. The report generation system 1860 can use these metrics instead of recalculating them from the rounded glucose data. This has the added advantage of allowing the report generation system 1860 to execute more efficiently because it does not need to calculate the metrics before the report can be generated.

The report generation system 1860 that receives the web request based on the encoded data will interpret the data as necessary. In some examples, the web request (and therefore the matrix barcode) can include an identifier for the encoding. The identifier can be, for example, a version number that allows the report generation system 1860 to select the appropriate decoding procedure based the version number. Including the identifier provides for added flexibility for data receiving device 120 programming, would allow for multiple types of encodings to be used simultaneously, and would allow for this procedure to be used even if the user of the data receiving device 120 has not update the software or firmware executing thereon. The type of encoding can further be selected based on the type of analyte data, the density of the data included in the request, or user preference.

Returning to the procedure 2200 of FIG. 22, the procedure 2200 continues at 2205, when the data receiving device 120 generates the matrix barcode. The matrix barcode can be generated using available algorithms or approaches (e.g., the QR code) or can be generated by a proprietary algorithm used by the analyte monitoring system 1800. While a proprietary algorithm can be used to improve the security of the process and can enable additional features with the matrix barcode (e.g., including more data), it will also limit interoperability and the convenience afforded by using a more common approach.

At 2206, the data receiving device 120 displays the matrix barcode. The data receiving device 120 can further display instructions for using the matrix barcode to request and retrieve the report.

At 2211, the matrix barcode is scanned. As an example, a multi-purpose device 130 can scan the matrix barcode. The multi-purpose device 130 can be part of an analyte monitoring system 100, for example, a glucose monitoring system. The multi-purpose device 130 can comprise a display, one or more processors, a camera (or other hardware to scan a matrix barcode) and a memory communicatively coupled with the one or more processors. The memory can comprise instructions that, when executed by the one or more processors are configured to cause the one or more processors to perform operations comprising one or more operations of the first procedure 2200, for example operations 2211, 2212, 2213, and 2214.

Another user device 140 having suitable hardware (e.g., a camera) and software to decode the matrix barcode can also be used. Many camera applications of modern smartphones include the capability to easily recognize and scan matrix barcodes.

At 2212, the matrix barcode is decoded to obtain the URL and parameters to be used with the URL. As with scanning matrix barcodes, many modern smartphones include the capability to decode and interpret commonly available matrix barcodes. If a proprietary algorithm is used to generate the matrix barcode, the multi-purpose device 130 can be provided instructions to download or execute programming code to decode the matrix barcode. For example, an application associated with the analyte monitoring system 1800 can be downloaded and executed on the multi-purpose device. Generally speaking, decode the matrix barcode includes identifying the data encoded within the matrix barcode. For example, where the matrix barcode includes the access URL and analyte data, decoding the matrix barcode involves converting that information into plaintext or another format suitable for interpretation by the multi-purpose device 130 as it determines how to handle the data.

At 2213, the URL and parameters are used to generate and send a web request. For example a web browser executing on the data receiving device 120 can be used. Having decoded the matrix barcode, the access URL and analyte data to be provided as parameters for the web request to generate a report are available to the multi-purpose device 130. The multi-purpose device 130 can construct the web request using standard mechanisms. The multi-purpose device 130 initiates the request to the report generation system 1860.

At 2221, the report generation system 1860 receives the web request based on the URL and parameters. The report generation system 1860 can be part of an analyte monitoring system 100, for example, a glucose monitoring system. The report generation system can be or include a server or server computing device comprise networking hardware, one or more processors, and a memory communicatively coupled with the one or more processors. The memory can comprise instructions that, when executed by the one or more processors are configured to cause the one or more processors to perform operations comprising one or more operations of the first procedure 2200, for example operations 2221 and 2222. At 2222, the report generation system 1860 generates the report. As discussed herein, the report generation system 1860 can interpret the parameters passed by the multi-purpose device 130 to discern the analyte values that will be used in the report. The report can take on a variety of forms, including display of individual analyte data values, trend information (e.g., short and long-term extrema, time in range, rate of change trends, etc.), treatment summaries, therapy recommendations, and other suitable information. The content of the report is based on the data available to the report generation system 1860. Wherein the data is generally of low quality or anonymous, the report will necessarily be more limited than when the data is high quality and personalized to the patient. The report generation system 1860 can then send the report back to the multi-purpose device 130. As an example, the report can be provided as a formatted webpage or transportable document (e.g., a PDF).

At 2214, the report is displayed by the multi-purpose device 130.

FIG. 23 illustrates a related procedure 2300 for requesting and generating a report based on analyte data provided via a matrix barcode. In particular, operation 2321 is an alternative version of operation 2221 illustrated in FIG. 22. Operations 2201-2206 and 2211-2213 illustrated in FIG. 22 can be similarly performed prior to 23, and are excluded only for the sake of providing a concise explanation of the procedure 2300.

At 2321, the report generation system 1860 receives the web request (e.g., "a first web request") based on the URL and parameters. The report generation system 1860 can be part of an analyte monitoring system 100, for example, a glucose monitoring system. The report generation system can be or include a server or server computing device comprise networking hardware, one or more processors, and a memory communicatively coupled with the one or more processors. The memory can comprise instructions that, when executed by the one or more processors are configured to cause the one or more processors to perform operations comprising one or more operations of the procedure 2300, for example operations 2321-2325. The first web request includes a request to enable limited access to additional user devices 140. At 2322, the report generation system 1860 generates the report based on the received parameters. The report generation system 1860 uses processes described herein to generate the report based on available analyte data.

At 2223, the report generation system 1860 generates an access code for the report. As described herein, an access code can be associated with the analyte data or the report, once generated, to limit access of the report only to individuals with the access code. Additionally, the report can be used to share the report and analyte data with users and systems without requiring a connection directly between the multi-purpose device 130 and the other systems. Instead, the report generation system 1860 uses the access code to validate access to the report and provide the report to the other system on behalf of the patient.

At 2224, the report generation system 1860 associates the access code with the report. For example, the report generation system 1860 can store the access code in a database with the report. In examples in which the report is generated on demand to display, the database can instead store the analyte data passed as parameters to the report generation system 1860 in association with the access code. As described above, the access code can be a temporary access code that is deleted after certain conditions have transpired such as a number of uses, passage of a specified amount of time, etc.

After the report generation system 1860 generates the access code, the report generation system 1860 can send the access code to the multi-purpose device 130 that initiated the web request at 2213. For example, the access code can be presented as a response to the web request.

At 2315, the access code can be displayed by the multi-purpose device 130. The multi-purpose device 130 can be part of an analyte monitoring system 100, for example, a glucose monitoring system. The multi-purpose device 130 can comprise a display, one or more processors, a camera (or other hardware to scan a matrix barcode) and a memory communicatively coupled with the one or more processors. The memory can comprise instructions that, when executed by the one or more processors are configured to cause the one or more processors to perform operations comprising one or more operations of the procedure 2300, for example operation 2315. Additionally, the multi-purpose device 130 can be displayed with an access URL through which the report can be accessed. Note that in some examples, the multi-purpose purpose device 130 can generate the access code itself and include the access code in the request to generate the report issued to the report generation system 1860.

At 2331, another user device 140 can use a web browser to visit the access URL. The user device 140 can be part of an analyte monitoring system 100, for example, a glucose monitoring system. The user device 140 can comprise a display, one or more processors, and a memory communicatively coupled with the one or more processors. The memory can comprise instructions that, when executed by the one or more processors are configured to cause the one or more processors to perform operations comprising one or more operations of the procedure 2300, for example operations 2331-2333. A webpage can be displayed requesting the user of the user device 140 to enter the access code. The access URL can be publicly available and any web browser can be used to visit the access URL because without an access code, visitors to the access URL cannot retrieve patient data. Additionally, in examples where the data included in reports shared through the mechanisms described herein include only anonymized or de-identified data, individual patient information is not at risk. If a patient has allowed for personal information to be included, the report can be further protected through passwords or other contextual information limiting access to the report. This can reduce the efficacy of brute force approaches to guess access codes and retrieve patient information.

At 2232, the user device 140 can receive the access code as input to the webpage. The user device 140 can send a second web request to the report generation system 1860 (or another related server) with the access code.

At 2225, the report generation system 1860 can retrieve the report based on the access code and the second web request. After receiving the access code, the report generation system 1860 can search the associated database using the access code and can identify the report accordingly. If the access code cannot be located or has expired, the report generation system 1860 can send a message to the user device 140 indicating that an error has occurred and identify the source of the error. If the access code is located and valid, in response to the second web request, the report generation system 1860 can send the report to the user device 140.

At 2333, the user device displays the report. The report can be displayed in the web browser or using an application configured to display the report. In some examples, the report can be best viewed using a proprietary application associated with the analyte monitoring system 1800, which can be installed on the user device 140.

Although the above examples have been described in the context of a data receiving device 120, similar techniques can be used with multi-purpose devices 130 configured to receive and process data from second control devices 102. Even though multi-purpose devices 130 can include hardware to connect to wide area networks (including the internet), users can lack have time or motivation to ensure that the remote application server 155 has up-to-date information before visiting an HCP. Similarly, users can not have accounts set up through the remote application server 155 or enabled to share analyte data with an HCP. Additionally, the multi-purpose device 130 cannot have access to the wide area network (e.g., not have cellular service) when visiting an HCP to upload the most up-to-date information. Examples described herein preclude the need for patients and/or HCPs to initiate and maintain an account with the report generation software or maintain consistent connection to the remote application server 155 or report generation system 1860.

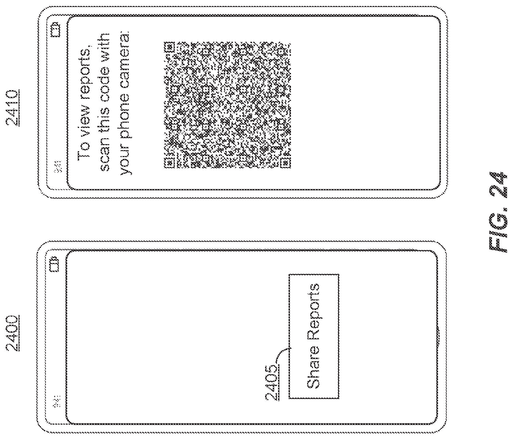

FIG. 24 illustrates example user interfaces of an application executing according to certain examples. In particular, FIG. 24 illustrates user interfaces of an application executing on a data receiving device 120 without wide area networking capabilities. Interface 2400 includes a number of options for accessing data and local reports collected by the data receiving device 120 from a sensor control device 102. Included among the options is an interactive element 2405 (e.g., an interface button) labeled "Share Reports." When the user selects interactive element 2405, the data receiving device 120 performs routines, including those described herein, for collecting and encoding analyte data associated with a preceding time period. The data receiving device 120 also generates a matrix barcode including the encoded analyte data and a URL to cause a multi-purpose device 130 to upload the encoded analyte data to a repot generation system 1860. The data receiving device 120 then displays the matrix barcode in an interface, such as interface 2410, which provides instructions for how to initiate the upload.

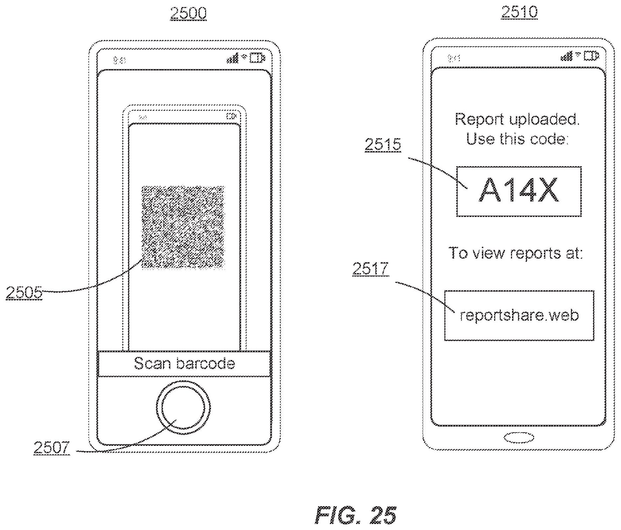

FIG. 25 illustrates example interface of an application executing according to certain examples. In particular, Fig. 25 illustrates user interfaces of applications executing on a multi-purpose device 130. Interface 2500 corresponds to a camera application of the multi-purpose device 130. The user has positioned the multi-purpose device 130 so that the data receiving device 120, while showing interface 2410, is within view of the camera of the multi-purpose device 130. Using the interactive element 2507, the user of the multi-purpose device 130 scans the matrix barcode shown in the viewfinder. After the multi-purpose device 130 scans the matrix barcode, the multi-purpose device 130 decodes the matrix barcode to interpret the URL and analyte data encoded therein. The multi-purpose device 130 then initiates a web request based on the URL to upload the analyte data to the report generation system 1860. The report generation system 1860 stores the analyte data in a database and generates an access code associated with the analyte data. The report generation system 1860 provides the access code and an access URL to the multi-purpose device 130. Interface 2510 illustrates the interface shown after the user has successfully uploaded the analyte data. The interface 2510 includes a display element with the access code 2515 provided by the report generation system 1860 and a display element with an access URL. If a user visits the access URL and provides the access code, the user device 140 used to visit the access URL can provide interfaces such as those illustrated in FIG. 21.

Electronic Medical Record (EMR) systems are used by many HCPs. The EMR system (e.g., EMR system 1865) stores data relating to a patient and the patient's interactions with an HCP. In some examples, it would be beneficial to incorporate systems and techniques, including those described herein, for generating a report relating to a patient's analyte data. In particular, it would be beneficial to simplify the processes used by the HCP and by the EMR system 1865 itself to access the patient's analyte data. For example, an analyte reporting application as described herein can be included as part of the EMR system. The analyte reporting application can be any software program established on the HCPs computer or established on a server that is accessible to the HCP and, in some example, is not necessarily an EMR.

In many systems, patient data can only be accessed by an EMR system 1865 for storage and processing with explicit permission from the patient. The patient must log into their account with a remote application server 155, identify the correct HCP who needs to see their analyte data, and ensure that the data provided is up to date. In some cases, the HCP must also log into an account with the remote application, identify the correct patient, and issue a request before the patient can authorize sharing data. An HCP can also need to remind a patient to approve a sharing request. This process is slow and error-prone - whether users forget their account credentials, identify the incorrect patient or HCP, or commit other errors - and generally inefficiently uses time when an HCP is considering a patient's medical records and treatment options. Attempts have been made to automate some parts of this process. For example, EMR systems 1865 and remote application servers 155 can attempt to match information like patient name, unique identifier, or contact information to generate a request to authorize data sharing. However, this requires patients to ensure that the information provided to their HCP and the remote application server 155 to be the same, any misspellings or, e.g., alternative contact information, can revert the patient back to a fully manual process. Even when matching is successful, the patient still must log into their account with the remote application server 155 to authorize the sharing request. Moreover, previous attempts to automate data sharing do not account for patients who did not have an account with the remote application server 155.

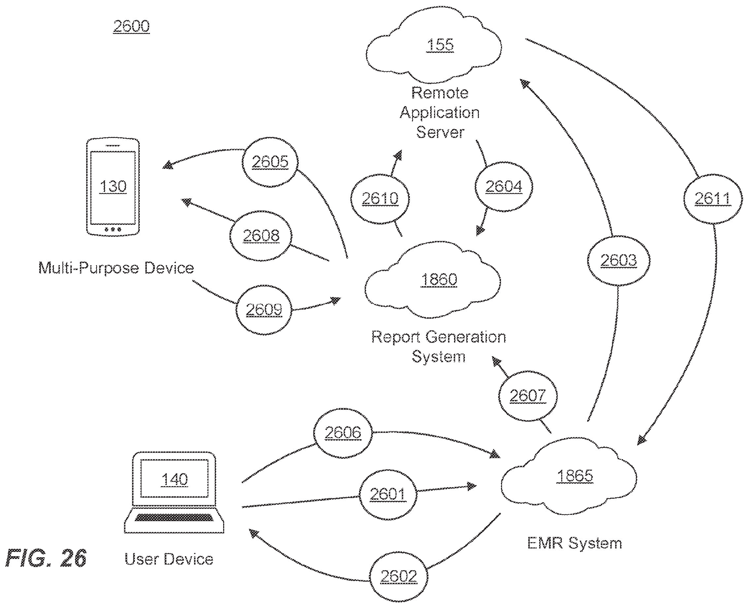

FIG. 26 illustrates an example flow of data between the components of the analyte monitoring system 2600 configured to facilitate a persistent connection or association between the remote application server 155 and EMR system 1865. The illustrated data flow begins at 2601, in which the HCP, using a user device 140 requests a report from the EMR system 1865 based on or using the analyte data, from remote application server 155, of a patient. The HCP can also initiate a request to review the latest analyte data of the patient or otherwise create a request that requests an interface between the EMR system 1865 and the remote application server 155.

Upon receiving the request, the EMR system 1865 determines that it does not have access to the analyte data of the patient stored on the remote application server 155. The request can include a unique identifier for the patient (e.g., a patient identification number or other unique representation). In some examples, the EMR system 1865 can use the unique identifier for the patient to query a database associated with the EMR system 1865 and stored data on patients who have integrated their data from the remote application server 155 with the EMR system 1865. In some examples, the EMR system 1865 can query the remote application server 155 with a request for data and reports associated with the patient identifier. When the integration between the remote application server 155 and EMR system 1865 has not been completed for a particular patient, the EMR system 1865 will receive a rejection to the request to access the data and reports.

Upon determining that the EMR system 1865 is not integrated with the remote application server data for the particular patient, at 2602, the EMR system 1865 responds that the HCP does not have access to the request information for the patient. The user device 140 can display an error message indicating that access has not been granted to the HCP and/or EMR system 1865. In some examples, the EMR system 1865 can cause the user device 140 to display instructions to the HCP for how to request access and/or integrate the EMR system 1865 and remote application server 155. As an example, the user device 140 can display, through the EMR system application, a prompt for the HCP to enter an access code associated with the patient data.

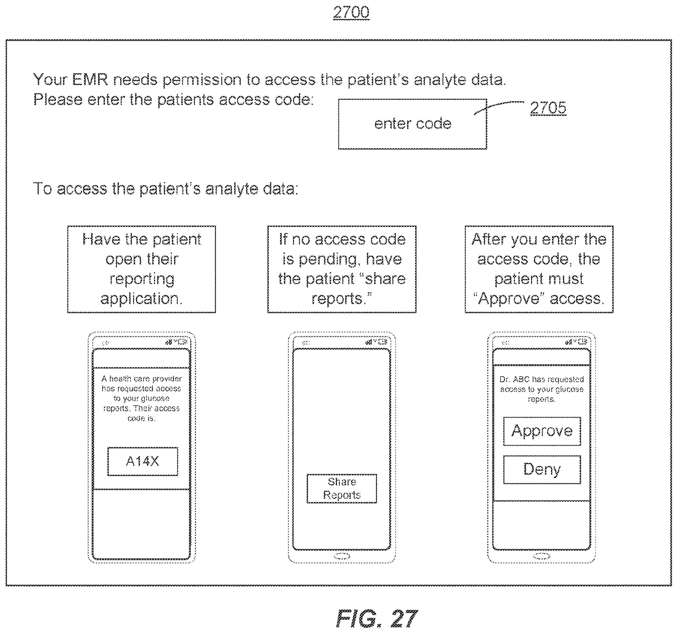

FIG. 27 illustrates an example interface of the user device 140 executing an application associated with the EMR system 1865 showing instructions on how to request access to the analyte data of a particular patient. The interface includes a user input field 2705 through which the HCP can input an access code provided by the patient.

Returning to the dataflow in FIG. 26, at 2603, the EMR system 1865 initiates a request to receive patient data associated with the patient identified by the HCP. The request can include the unique patient identifier and other information for the remote application server 155 to identify which patient is of interest to the HCP. The remote application server 155 can perform validation and integrity checks on the request to reduce the opportunity for a malicious user to spam authorization from the patient.

At 2604, upon receiving the request from the EMR system 1865, the remote application server 155 can request the report generation system 1860 to initialize the integration process on behalf of the EMR system 1865. As described herein, the integration process includes generating an access code, providing the access code via the multi-purpose device 130 of the patient, and allowing the user device 140 to provide the access code to the repot generation system 1860. Therefore, the report generation system 1860 can generate a request to the multi-purpose device 130. The request can include an indication that an HCP has requested access to the analyte data of the patient associated with the multi-purpose device 130. If available, e.g., if provided by the EMR system 1865, the request can identify the HCP. The request can also include an access code generated by the report generation system 1860. The EMR system 1865 provides the request to the multi-purpose device 130 at 2605.

Upon receiving the request from the report generation system 1860, the multi-purpose device 130 can display the access code. An application associated with the remote application server 155 can launch into foreground execution by the multi-purpose device 130 and inform the user of the multi-purpose device 130 (e.g., the patient), that an HCP has requested access to their analyte data. The application can display the access code and provide instructions for the patient to display the access code to their HCP. In some examples, the multi-purpose device 130 can first confirm that the patient wishes to share their analyte data and reports with the HCP before displaying the access code. In some examples, the multi-purpose device 130 can generate the access code itself, in which case the multi-purpose device 130 also provides the access code to the report generation system 1860 (not illustrated).

In some examples, the patient initiates the process to generate an access code, rather than the process being initiated by the report generation system 1860 on behalf of the HCP. As discussed herein, the patient can use a "share reports" feature to generate an access code shared between the multi-purpose device 130 and the report generation system 1860. The report generation system 1860 can use the access code, as discussed below, to identify a patient's records.

The patient provides the access code to the HCP. The HCP enters the access code into the provided user input on the application executing on the user device 140 and associated with the user EMR system 1865. At 2606, the user device 140 provides the entered access code to the EMR system 1865.

At 2607, the EMR system 1865 provides the access code to the report generation system 1860. For the sake of brevity, this is shown as a direct transmission between the EMR system 1865 and the report generation system 1860, although it should be understood that the access code can be provided by the EMR system 1865 to the report generation system 1860 via the remote application server 155. Similarly, in some examples, the user device 140 can have or establish a connection to the report generation system 1860 and the user device can provide the access code to the report generation system 1860 through said connection. As an example, the multi-purpose device 130 can, in addition to displaying the access code, display an access URL associated with the report generation system 1860. The HCP can use the user device 140 to navigate to the website accessible via the access URL and provide the access code to the report generation system 1860 through the website.

Upon receiving the access code, the report generation system validates and verifies the access code from the user device 140 with the access code generated by the report generation system 1860 (or by the multi-purpose device 130 and provided to the report generation system 1860). Validating the access code can include determining that the access code has not expired and does not have other restrictions limiting the availability of the access code to permit analyte data integrations. If the access code is invalid, the report generation system 1860 can optionally notify the user device 140 (e.g., through the remote application server 155 or EMR system 1865 as an intermediary system) that the access code that has been provided is not valid or that some other error has occurred. The report generation system 1860 can also notify the multi-purpose device 130 of an error.

If the access code is valid, the report generation system 1860 can perform an additional access validation with the multi-purpose device 130. In some examples, the process illustrated in dataflow 2600 can be used to create a continuous or permanent integration of the patient's analyte data with the EMR system 1865. Because this can include highly sensitive information, the analyte monitoring system 1800 can request the patient to validate the request, even after the HCP has provided an access code. This acts as a second factor for authenticating access to the patient data. At 2608, the report generation system 1860 transmits the validation request to the multi-purpose device 130.

The multi-purpose device 130 receives the validation request and displays the request to the patient. As an example, the validation request can be displayed as a pop-up or push notification on the multi-purpose device 130. The notification can include a user interface with a prompt asking the user to validate the access request made by the HCP. The notification can include information associated with the HCP and the EMR system 1865 provided when the EMR system 1865 provided the access code to the report generation system 1860. At 2609, the multi-purpose device 130 provides the patient's response to the prompt to the report generation system 1860.

If the patient denies access through the prompt, the report generation system 1860 can deny access to the EMR system 1865. The report generation system 1860 can inform the EMR system 1865 that access to the patient's analyte data was not granted. In some examples, the report generation system 1860 can measure the number of requests for access for a particular patient or made by a particular EMR system 1865 or HCP over a period of time and determine to block further access requests if abuse of the system is suspected.

If the patient affirmative responded to the validation request, at 2610, the report generation system 1860 informs the remote application server 155 that access has been authorized. The report generation system 1860 can notify the remote application server 155 associated with the analyte monitoring system 1800 that a valid access code has been received and that the multi-purpose device 130 has verified the request.

At 2611, the remote application server 155 grants access to the requested patient analyte data and reports and provides the requested data and reports to the EMR system 1865. As described, the integration of data can enable to the HCP to retrieve the latest available analyte data for the patient using the HMR system 1865. For example, as a sensor control device 102 worn by the patient provides data to the multi-purpose device 130 and the data is uploaded to the remote application server 155, the data can be shared with or accessed by the HCP through the EMR system 1865. The patient can control which data is shared, such as enabling only a one-time sharing of data or sharing data for a particular time range or for a particular period of time. Of particular benefit is that this system can proceed without any user (e.g., the patient or HCP) being required to remember access credentials for any of the systems being used. Additionally, the identity of the patient and HCP are verified to each other multiple times throughout the process, which helps to ensure that the correct data about the correct patient is being shared to the correct HCP.

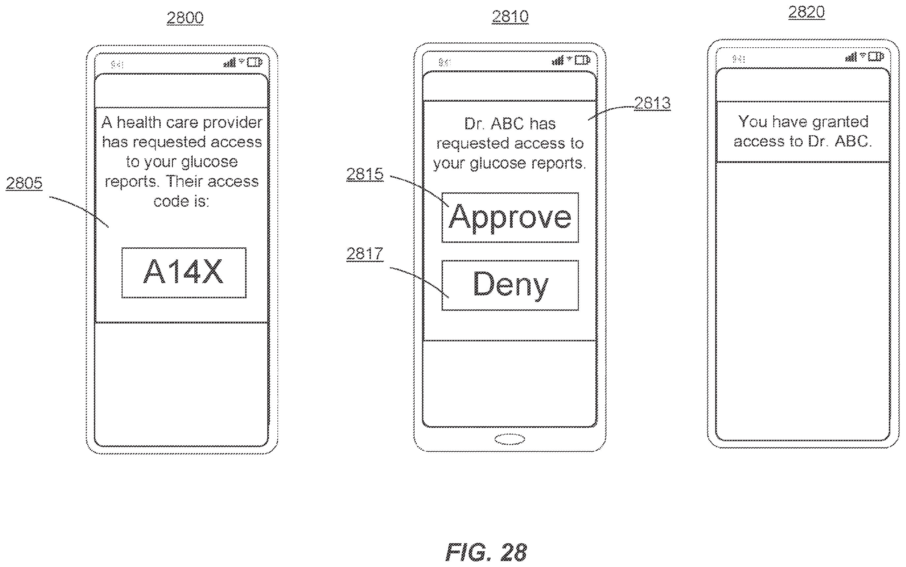

FIG. 28 illustrates user interfaces that can be displayed on a multi-purpose device according to examples disclosed herein. In particular, FIG. 28 illustrates detailed views of user interfaces 2800-2820 that can be used to facilitate the integration of analyte data from the remote application server 155 being shared with an EMR system 1865. User interface 2800 includes a notification 2805 from the application associated with the analyte monitoring system 1800 executing on the multi-purpose device 130 indicating that an HCP has requested access to the patient's analyte data. The notification 2805 includes an access code that will be used by the HCP as described above. Interface 2810 illustrates a notification 2813 from the application associated with the analyte monitoring system 1800 executing on the multi-purpose device 130 indicating that a specific HCP has entered an access code. The notification 2813 includes an interactive element 2815 to approve or authorize the access and an interactive element 2817 to deny the access. Interface 2820 illustrates a notification confirming that access has been granted.

Once the connection or association between the EMR system 1865 and the remote application server 155 is established, information entered by the HCP, e.g., based on the reports from the report generation system 1860, can be shared back to the remote application server 155 as appropriate. For example, therapy modifications or requests for further monitoring performance can be made by the HCP through the EMR system 1865. This can be particularly beneficial where the HCP is not comfortable using native applications provided by the analyte monitoring system 1800 or otherwise prefers their own EMR system 1865 for its convenience. The further information from the HCP can also be shared with the patient through their own access to the remote application server 155 or other applications associated with the analyte monitoring system 1800. Additionally, because analyte data associated with the patient is centralized by the remote application server 155 but can come from sensor control devices 102 via a variety of pathways (e.g., through a data receiving device 120, multi-purpose device 130, or user device 140), the data can be shared with the EMR system 1865 regardless of the source. The patient does not need to remember to perform additional processes to ensure that their HCP is provided with up-to-date data to assist with their health monitoring.

In some examples, the integration between the remote application server 155 and the EMR system 1865 can be limited based on system settings or user preference. For example, when granting access, the patient can specify that only analyte data of a certain type or for a specified period of time will be shared with the EMR system 1865. Requests to access data stored by the remote application server 155 and exceeding the granted authorization can be denied by the remote application server 155 on behalf of the patient or can be converted into a request for additional access on behalf of the HCP.

Some examples can facilitate data sharing between the patient and the EMR system 1865 even when the patient does not have an account with the remote application server 155. For example, when the patient requests to "share reports," the remote application server 155 can generate a limited-use account that is associated with the application instances of the application associated with the remote application server 155. Once the authorization process is completed, the remote application server 155 can share the data associated with the limited-use account. The patient can be provided the option to create a full account to maintain the connection or association to the EMR system 1865. The patient can also cause the account to be deleted, in which case the data sharing is a one-time operation.

Note that, although the remote application server 155 and the report generation system 1860 are described as separate entities, this is merely for the purpose of providing a clear explanation of the functionalities of various components. In some examples, the remote application server 155 and report generation system 1860 can be functions of the same overall system.

In some examples, the connection or association between the remote application server 155 and the EMR system 1865 can be facilitated using a matrix barcode rather than requiring entry of an access code at the user device 140. In this example, the user device 140, upon receiving confirmation from the EMR system 1865 that the HCP does not yet have access to the request patient data, displays a matrix barcode. The matrix barcode can be configured to initiate a web request to enable access of a specific user or system (e.g., the HCP and EMR system 1865) to the patients analyte data stored by the remote application server.

The patient can use a camera of their multi-purpose device 130 to scan the matrix barcode displayed on the user device 140. The matrix barcode can include instructions to enable authorization for the EMR system 1865 to receive access to the patients' analyte data via the remote application server 155. When the matrix barcode is scanned, the multi-purpose device 130 can extract details about the authorization request encoded within the matrix barcode and use this information to begin an authorization procedure, such as that described herein. In some examples, the matrix barcode scanning functionality can be bundled into an application associated with the analyte monitoring system 1800 executing on the multi-purpose device 130 (e.g., that enables the multi-purpose device 130 to communicate with the sensor control device 102). Scanning the matrix barcode can cause a prompt to allow access to be displayed within the application with information about the type of access that will be granted, who and what system are requesting access, and mechanisms to control access. Upon the patient's affirmative response, access is granted to the EMR system 1865, which can be notified by the remote application server 155.

As discussed herein, if the patient affirmatively initiates the request to generate an access code, e.g., through activating a "share reports" feature of the application executing on the multi-purpose device 130, interface 2800 can merely confirm that recent analyte data has been provided to the report generation system 1860 and provide the access code.

As discussed herein, data receiving devices 120 are lower cost mechanisms for a patient to interact with an analyte monitoring system 1800. Data receiving devices 120 can communicate with a sensor control device 120, but cannot communicate with a remote application server 150 directly. Instead, the data receiving device 120 is connected to a user device 140 through a wired connection and the analyte data is uploaded by the user device 140 to the remote application server. For many patients, data receiving devices are the primary method of monitoring their analyte levels on a day-to-day basis.

In some examples, even the data receiving device 120 can be used to grant access to allow a EMR system 1865 to access analyte data stored by the data receiving device 120 or continuously uploaded to the remote application server 150. The procedure described above, e.g., with respect to at least FIG. 26 can be modified to use the data receiving device.

As an example, when the HCP uses the user device 140 to access the analyte data of the patient (e.g., at 2600), the EMR system 1865 can cause an application interface to be displayed on the user device 140 providing instructions to enable access. The application interface can also include a user input field to enter an access code (such as in the interface 2700 of FIG. 27). The instructions can instruct the HCP to ask the patient to use their data receiving device 120 to activate a "share reports" feature. This share reports feature can be similar to the share reports feature using data receiving devices 120 discussed above (e.g., with respect to FIGS. 22-23). The share reports feature causes the data receiving device 120 to generate a matrix barcode encoded with a URL and encoded analyte data as parameters for the URL. The instructions can further instruct the HCP or a patient to scan the matrix barcode using a suitable application (e.g., a camera application executing on a smartphone or other multi-purpose device 130). The matrix barcode can be further encoded with access credentials and other user identifying information to facilitate persistent connection or association between the EMR system 1855 and the remote application server 155.

Upon scanning the matrix barcode, the encoded analyte data is provided to the report generation system 1860, which generates an access code and access URL. The report generation system 1860 provides the access code and access URL to the device that scanned the matrix barcode (e.g., as shown in FIG. 25). The HCP can enter the access code to the user input field presented in the application associated with the EMR system 1865. The EMR system 1865 then relays the access code to the report generation system 1860 (e.g., as in 2607). Upon validating the access code, the remote application server 155 can grant access to some or all of the analyte data provided by the multi-purpose device 130 to the report generation system 1860. This access can be limited to one-time access or continual access per system settings or user preferences of the patient. As the patient uploads additional analyte data, the data can also be shared with the EMR system 1865 as described herein without having to validate access on each occurrence.

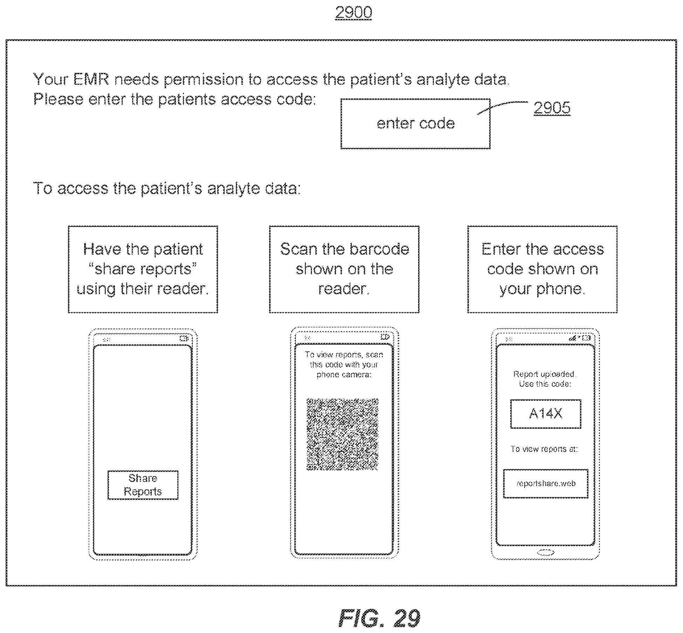

FIG. 29 illustrates an example user interface for granting access according to embodiments herein. In particular, FIG. 29 illustrates an example user interface 2900 that can be used by an application associated with an EMR system 1865 and executing on the user device of an HCP requesting access to a patient's analyte data. The user interface 2900 includes a user input field 2905 for the HCP to enter an access code provided by a patient. The user interface 2900 also includes instructions for the HCP to follow to generate the access code when the patient uses a data receiving device 120. The instructions, as outlined above, require the HCP to scan a matrix barcode generated by the data receiving device 120 using another device, such as a smartphone, and enter the access code that will be displayed in the user input field 2905.

This procedure can be further modified to minimize the amount of manual user inputs required to grant authorization. In this example, after the HCP or patient scans the matrix barcode, the URL causes accesses a web application to be displayed on the device that scanned the matrix barcode. The web application includes a camera function and provides instructions for the user to scan an authorization matrix barcode. The authorization matrix barcode is provided in the instructions shown by application associated with the EMR system 1865 and executing on the user device after the HCP has requested access. The authorization matrix barcode includes the information needed for the web application executing on the other device to send the analyte data, reports, or data access credentials to the report generation system 1860 or the remote application server 155 to facilitate access.

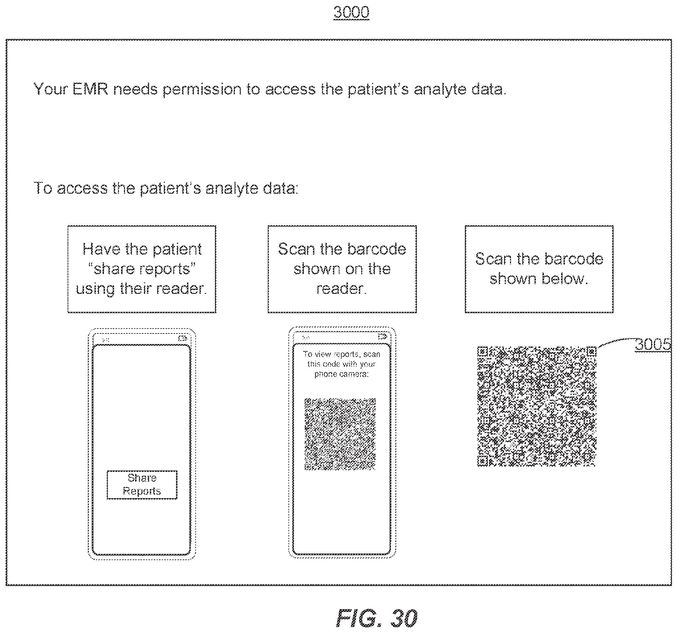

FIG. 30 illustrates an example user interface for granting access according to embodiments herein. In particular, FIG. 30 illustrates an example user interface 3000 that can be used by an application associated with an EMR system 1865 and executing on the user device of an HCP requesting access to a patient's analyte data. The user interface 3000 includes instructions for the HCP to follow to request an access code to be generated. For example, the instructions can enable the EMR system 1865 to receive data when the patient uses a data receiving device 120. The instructions, as outlined above, ask the HCP to scan a matrix barcode generated by the data receiving device 120 using another device, such as a smartphone, and then scan a second matrix barcode 3005 provided in the user interface 3000. In some examples, the instructions can be modified to ask the HCP to scan a matrix barcode 3005 provided in the user interface 3000 first to initiate the process. In an unillustrated example, the instructions in the user interface 3000 can additionally or alternatively ask the patient to use a camera or scanning function of an application executing on their multi-purpose device 130 to scan a matrix barcode displayed in the user interface 3000. In some examples, the application is associated with the analyte monitoring system 100. In some examples, the application is a camera or scanning application. The matrix barcode can, for example, include a URL, deeplink, or other instructions to cause the multi-purpose device 130 to initiate a request to establish or authorize a connection or association between their analyte data stored by the analyte monitoring system 100 and patient data stored by the EMR system 1865 according to techniques described herein.

The HCP can access data retrieved from and based on a sensor control device 102 in web- or server-based applications. For example, a patient can transfer data from a sensor control device 102 to a data receiving device 120 or multi-purpose device 130. The data can then be relayed to a remote application server 155 to be processed. In addition to providing standardized reports based on the analyte data, applications executing on or made available by the remote application server 155 can provide guided interpretation of the data. For example, where the analyte of interest includes or is derived from glucose levels, the application executing on the remote application server 155 can provide interpretation of metrics such as an ambulatory glucose profile (AGP) by identifying glucose patterns or areas that need attention. The guided interpretation can include details such as therapy and lifestyle guidance.

Therapy and lifestyle guidance can include assessment of the effect of a patient's current therapy on their health goals, such as their glycemic control. However, this guidance requires knowledge of the patient's current therapy which is not always readily available to the remote application server 155. For example, the remote application server 155 can be operated by the provider of the analyte monitoring system 100, while the information about a patient's current therapy can be housed by a third party (e.g., an HCP system). It would be beneficial to provide for integration of information such as a patient's current therapy within the tools provided by the remote application server 155 to provide, to the HCP and to the patient, more effective therapy and lifestyle guidance, and to provide a comprehensive view of the patient's health and health management.

The HCP can enter therapy information directly into the application executing on the remote application server 155. For example, the HCP can be asked to enter information such as medication names, doses, frequencies, and schedule. Taking, again, glucose management as one example, if the therapy includes insulin, then the HCP can be asked to enter context-specific information for the insulin regimen which can include dose amount, schedule, insulin to carbohydrate ratio, correction factor, and target glucose. If the patient is on insulin pump therapy, then the HCP can enter the basal rates and schedule.

In another example, the HCP can enter the therapy information into their electronic medical records (EMR) system 1865. The therapy information can be retrieved from the EMR system 1865 and imported into the remote application server 155, or can otherwise be accessed on demand by the remote application server 155. In particular, aspects of the remote application server 155 can be integrated with the EMR system 1865.

In some examples, therapy information stored by the EMR system 1865 can be stored in a well-structured format (e.g., a "first structured format"). As an example, the therapy information can be stored in a database or table with labeled headers and consistent use and entry enforcement provisions. A database of the remote application server 155 can store similarly data in another well-structured format (e.g., a "second structure format"). In such examples, therapy information can be extracted from the EMR system 1865 after a mapping between fields of the EMR system 1865 databases and the remote application server 155, e.g., a mapping between the first structured format and the second structured format.

In some examples, the therapy information stored by the EMR system 1865 can be included in the form of structure-free data or free text notes. Because there is no straightforward mapping available, additional processing on the therapy information can be required to identify the relevant information and determine how to use the therapy information in the context of the remote application server 155. As an example, free text can be interpreted using keyword association, natural language processing, or pattern matching techniques to extract the relevant information for addition to the remote application server 155. The extracted information can be mapped to a structure format used by the database of the remote application server 155.

To encourage the use of well-structured formats, the remote application server 155 can provide a structured data entry interface. As an example, the remote application server 155 can provide an insulin regimen table with dose amount, schedule, insulin to carbohydrate ratio, correction factor, and target glucose, or a table to enter insulin pump therapy parameters. Similar interface options can be provided for other analytes of interest. Once the data is entered into the remote application server 155, the remote application server 155 can use its integration with the EMR system 1865 to convert the data into a structured format usable by the EMR system 1865. For example, a reverse mapping to the EMR system 1865 can be determined. Alternatively, the structured data from the remote application server 155 can be converted into a format that can be added to the free text fields provided by the EMR system 1865. This will encourage data to be saved in a structured format, even in the free text fields of the EMR system 1865, so that the data can be easily extracted during subsequent imports.

The connection or association and reporting techniques described herein are addressed to a traditional order-based system for electronic medical reports. However, these techniques would also apply to an application-based EMR system that can be developed, for example, using the Smart-on-FHIR framework. Additionally or alternatively, these procedures can also work with an intermediary that bridges EMR systems to an external data provider.

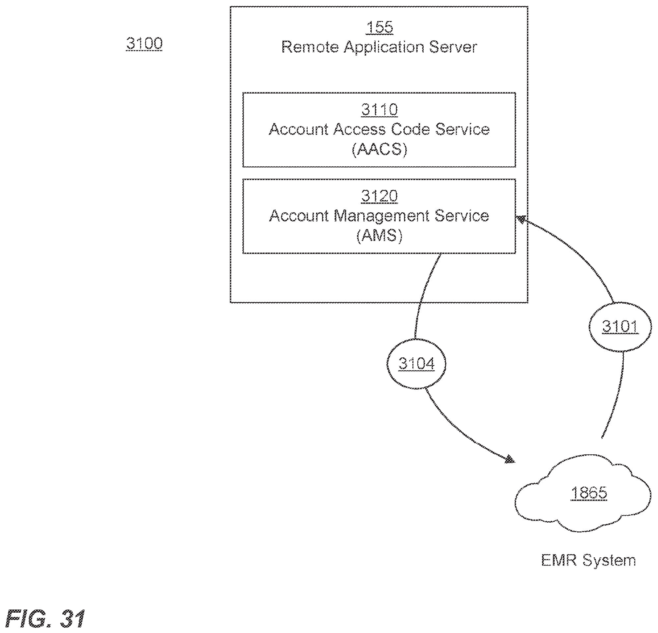

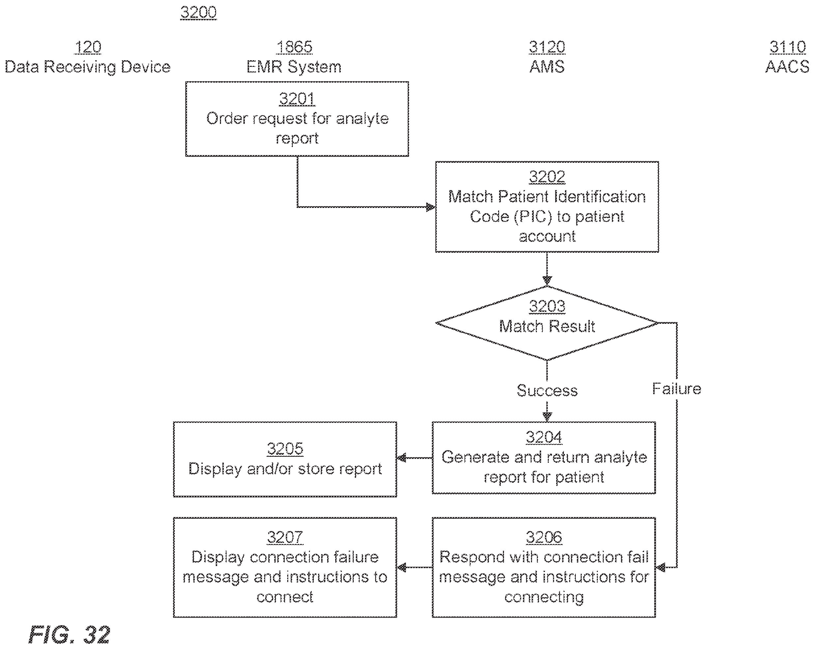

FIG. 31 illustrates an example dataflow for an analyte monitoring system according to certain embodiments. In the illustrated dataflow 3100, the remote application server 155 includes two additional components: an account access code service (AACS) 3110 and an account management service (AMS) 3120. The EMR system 1865 has multiple patient accounts corresponding to multiple users. The remote application server 155 similarly has accounts corresponding to each of the existing patient accounts stored by the EMR system 1865. The remote application server 155 manages the patients' analyte data and generates reports using these data. In the example illustrated in FIG. 31 and FIG. 32, the remote application server 155 and EMR system 1865 store a common unique identifier for each patient. One example common unique identifier is a Patient Identification Code (PIC) stored by both the remote application server 155 and EMR system 1865 for a given patient. Note that the unique identifier, such as the PIC can be originally generated by either system and can take different forms. For example, the PIC can be a patient's medical record number in the EMR system 1865. As another example, the PIC can be a global unique identification code (GUID) generated by either system during the process of establishing the connection or association.

When an order is placed at the EMR system (e.g., by a health care professional) for an analyte report, the request 3101 is sent electronically to the remote application server 155. The request 3101 includes this unique identifier for the patient. Upon receiving the request, the remote application server 155 using the account management service 3120, determines the patient account associated with the received unique identifier. The remote application server 155, through the account management service 3120 returns, at 3104, one or more reports based on analyte data associated with the patent's account stored by the remote application server 155 to the EMR system 1865. The EMR system 1865 also associates the one or more reports with the patient account in the EMR system 1865.

FIG. 32 illustrates a procedure for requesting and generating a report according to certain embodiments. The procedure 3200 illustrated in FIG. 32 corresponds to the dataflow 3100 illustrated in FIG. 31. At 3201, the EMR system 1865 orders a request for an analyte report. The EMR system 1865 can be in communication with or a part of an analyte monitoring system 100, for example, a glucose monitoring system. The EMR system 1865 can be or include a server or server computing device comprise networking hardware, one or more processors, and a memory communicatively coupled with the one or more processors. The memory can comprise instructions that, when executed by the one or more processors are configured to cause the one or more processors to perform operations comprising one or more operations of the procedure 3200, for example operations 3201, 3205, and 3207.

At 3202, the AMS 3120 attempts to match a patient identification code (PIC) to an existing patient account stored by the EMR system 1865. The AMS 3120 can be a part of an analyte monitoring system 100, for example, a glucose monitoring system. The AMS 3120 can be or include a server or server computing device comprise networking hardware, one or more processors, and a memory communicatively coupled with the one or more processors. The memory can comprise instructions that, when executed by the one or more processors are configured to cause the one or more processors to perform operations comprising one or more operations of the procedure 3200, for example operations 3202, 3203, 3204, and 3206. At 3203, the AMS 3120 determines whether the AMS 3120 is able to identify a match. In this case, a match between the PIC indicates a connection or association between patient accounts in the EMR system 1865 and the remote application server 155. If at 3203 the AMS 3120 finds a successful match, then the procedure proceeds to 3204.

At 3204, the AMS 3120 (or other component of the remote application server 155) generates a report based on analyte data associated with the patient identified with the PIC. At 3204, the AMS 3120 sends the generated report to the EMR system 1865. At 3205, the EMR system 1865 displays and/or stores the generated report. In some examples, the AMS sends 3120 and/or the EMR system 1865 displays a confirmation of the association or connection of the analyte data associated with the patient and the data associated with the patient and stored by the EMR system 1865. If at 3203, the AMS 3120 is not able to find a successful match, then the procedure proceeds to 3206. At 3206, the AMS 3120 responds to the EMR system 1865 with a message indicating that the AMS system 3120 was not able to connect the received PIC with an existing patient account. At 3207, the EMR system 1865 displays the connection or association failure message and instructions to establish the connection or association.

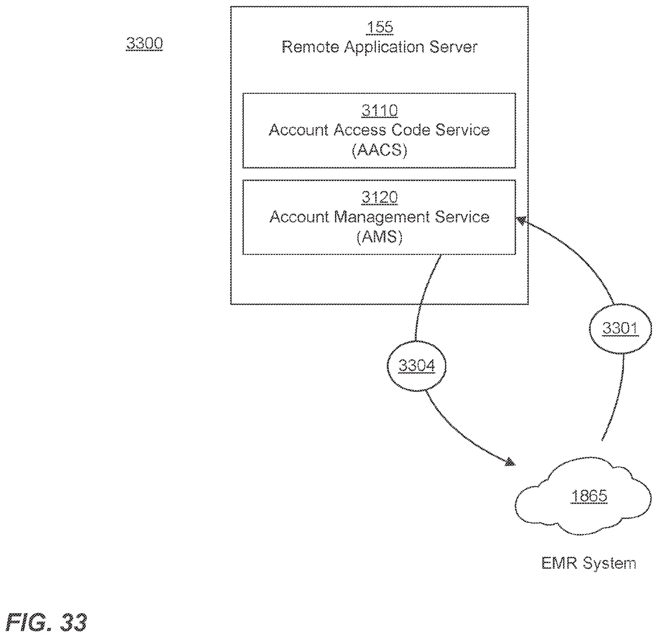

FIG. 33 illustrates an example dataflow for an analyte monitoring system according to certain embodiments. In particular, the example dataflow 3300 relates to a procedure for establishing a connection or association that uses patient identification information and other fields of information commonly stored by the remote application server 155 and EMR system 1865. In particular, the ideal patient identification information is information common to both systems. Such patient identification information can include, by way of example and not limitation, first name, last name, date of birth, email address, phone number, insurance identification, and the like. The EMR system 1865 places an order 3301 as a request to establish a connection or association. The order 3301 includes the patient information identification. The remote application server 155 receives the order including the patient identification information. The AMS 3120 (or other component of the remote application server 155) identifies the received patient identification information in the order and attempts to match them with the corresponding fields in its database. If the AMS 3120 finds a match, then it returns a response 3304. The response 3304 can include a Patient Identification Code (PIC). The EMR system 1865 stores this PIC to establish the connection or association. In some examples, the EMR system 1865 can generate a PIC for its own patient records. The EMR system 1865 can provide the PIC in its request 3301. Upon finding a successful match of the patient information included in the request 3301, the remote application server 1865 can store the PIC, establishing the connection or association. Subsequent requests for reports issued by the EMR system 1865 can include this PIC (whether generated by the EMR system 1865 or the remote application server 155), as described herein. In some examples, the AMS sends 3120 and/or the EMR system 1865 displays a confirmation of the association or connection of the analyte data associated with the patient and the data associated with the patient and stored by the EMR system 1865.

One advantage of this procedure for establishing a connection or association is that it does not require affirmative action from the patient. However, in some examples, these fields associated with the patient are not identically recorded in both the EMR system 1865 and remote application server 155. For example, one system can have a different spelling for a patient's name, additional or different names (e.g., include a middle name or middle initial; include a patient's maiden name), out of date email or phone number, at the like. In such a scenario, the connection or association will fail. If the AMS 3120 cannot match the information received with the request 3301 with information stored in its own database, then the response 3304 from the AMS 3120 will include a connection or association failure message. The connection or association failure message can also include instructions for alternative ways to establish a connection or association, as described herein.

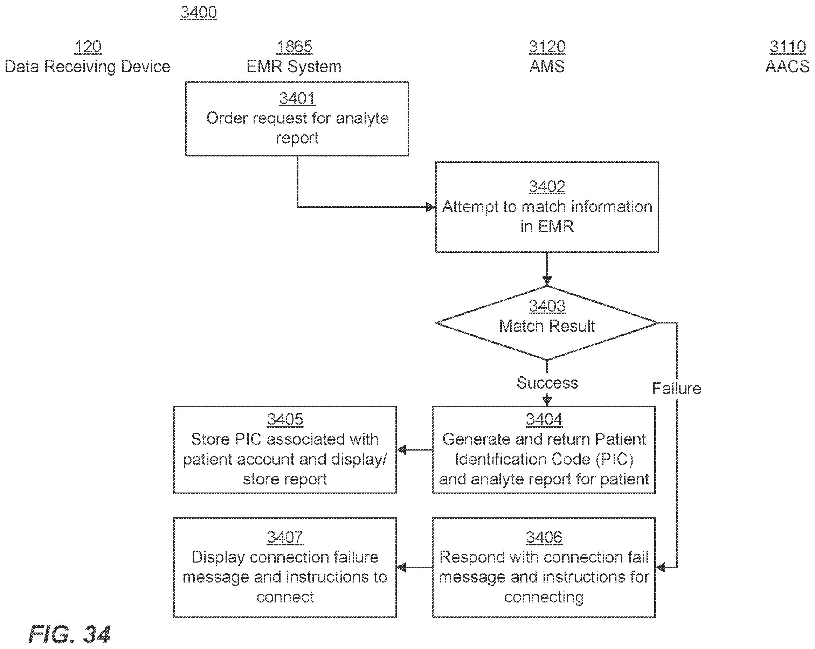

FIG. 34 illustrates a procedure 3400 for requesting and generating a report according to certain embodiments. The procedure 3400 illustrated in FIG. 34 corresponds to the dataflow 3300 illustrated in FIG. 33. At 3401, the EMR system 1865 submits a request for an analyte report to the remote application server 155. The EMR system 1865 can be in communication with or a part of an analyte monitoring system 100, for example, a glucose monitoring system. The EMR system 1865 can be or include a server or server computing device comprise networking hardware, one or more processors, and a memory communicatively coupled with the one or more processors. The memory can comprise instructions that, when executed by the one or more processors are configured to cause the one or more processors to perform operations comprising one or more operations of the procedure 3400, for example operations 3401, 3405, and 3407. As described herein, the request includes patient information from one or more patient information fields of the EMR system 1865 in lieu of a PIC.

At 3402, the AMS 3120 attempts to match the received patient information with information stored by the remote application server 155. The AMS 3120 can be a part of an analyte monitoring system 100, for example, a glucose monitoring system. The AMS 3120 can be or include a server or server computing device comprise networking hardware, one or more processors, and a memory communicatively coupled with the one or more processors. The memory can comprise instructions that, when executed by the one or more processors are configured to cause the one or more processors to perform operations comprising one or more operations of the procedure 3400, for example operations 3402, 3403, 3404, and 3406.

At 3403, the AMS 3120 determines whether the AMS 3120 is able to identify a match to the information provided by the EMR system 1865. In this case, a match indicates that a patient account with the analyte monitoring system has already been established and that it is therefore appropriate to link that information to the patient account represented by the ERM system 1865. If at 3403 the AMS 3120 finds a successful match, then the procedure proceeds to 3404. At 3404, the AMS 3120 (or other component of the remote application server 155) generates a PIC and further generates a report based on analyte data associated with the patient identified based on the provided information. At 3404, the AMS 3120 sends the PIC and generated report to the EMR system 1865. At 3405, the EMR system 1865 stores the PIC associated with the patient account in the EMR system 1865 and displays and/or stores the generated report. If at 3403, the AMS 3120 is not able to find a successful match, then the procedure proceeds to 3406. At 3406, the AMS 3120 responds to the EMR system 1865 with a message indicating that the AMS system 3120 was not able to identify an existing patient account with the provided information. At 3407, the EMR system 1865 displays the connection failure message and instructions to establish the connection.

Once the connection or association is established between patient data stored by the EMR system 1865 and associated with a particular patient and patient data stored by the AMS 3120 (or other component of the remote application server 155 of the analyte monitoring system 100), data can be exchanged between the EMR system 1865 and AMS 3120 to keep the patient data stored by each system up to date. For example, when the AMS 3120, or other component of the remote application server 155 of the analyte monitoring system 100 determines that new or updated analyte data, data derived from the analyte data, or the like, is available for the patient record associated with a patient, the AMS 3120 can send the new or updated data to the EMR system 1865 for display and/or storage in association with the patent records. Similarly, when the EMR system 1865 determines that new or updated patient records data, including diagnoses, prognoses, treatments, medication regimens, other therapeutic regimens, and like, is available for the patient record associated with a patient, the EMR system 1865 can send the new or updated data to the AMS 3120 for storage or analysis in association with the patent records. The updated data be provided, for example, simultaneously with the determination that new data is available or upon analysis during a regularly schedule batch update. Similarly, the patient data records between the AMS 3120 and EMR system 1865 can be synchronized (i.e., updated data between the AMS 3120 and EMR system 1865 can be exchanged) on a periodic or recurring basis. In some examples, the AMS 3120 can be configured to request updated data from the EMR system 1865 or the EMR system 1865 can be configured to request updated data from the AMS 3120. For example, as described herein, the remote application server 155 can include or communicate with a report generation server, such that the analyte monitoring system 100 is capable of generating reports based on a patient's data. When the AMS 3120 receives a request for such as report, the AMS 3120 can first request updated patient data from the EMR system 1865. The EMR system 1865 can store, for example, therapeutic data about the patient or a patient's prescribed medication regimen, which can be material to the generation of the report. The EMR system 1865 can respond to the request with the latest medication regimen (or other related data). If, in response to a request for new or updated patient data, the responding system (e.g., if the AMS 3120 initiates the request, the EMR system 1865 would be the responding system, and vice versa) determines that no new patient data is available, then the responding system can respond with a message indicating that no new data associated with the particular patient is available.

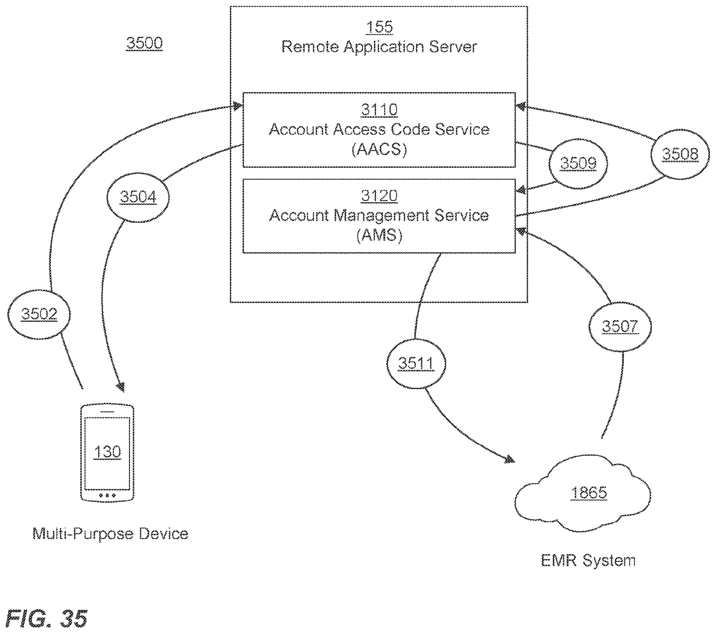

FIG. 35 illustrates an example dataflow for an analyte monitoring system. The dataflow 3500 corresponds to a procedure for establishing a connection or association between patient data stored in a remote application server 155 and EMR system 1865 and optionally requesting analyte reports based on that data according to some examples. The particular procedure illustrated in FIG. 35 involves affirmative action from the patient to confirm approval for the EMR system 1865 to access data associated with the patient in the remote application server 155. As illustrated, the procedure involves a multi-purpose device 130 owned or used by the patient. As described herein, the multi-purpose device 130 can be embodied as a smartphone executing an application that is associated with the patient's account in the remote application server 155. In some examples, this application also interacts with the patient's analyte sensor and send analyte data detected by the analyte sensor to the patient's account in the remote application server 155. That is, the multi-purpose device 130 can be the vector through which the patient sends analyte data to the remote application server 155. In other examples, the application can be separate from the application that interacts with the patient's analyte sensor and is instead a dedicated application for authenticating access and connection or association requests between the EMR system 1865 and the remote application server 155.

As illustrated, the remote application server 155 includes an account access code server (AACS) 3110. The AACS 3110, along with the AMS 3120, is one of many services operating in or accessible to the remote application server 155. The actual software architecture of the remote application server 155 can include many additional services, or many instances of the same service, executing in parallel with the illustrated AACS 3110 and AMS 3120. The AACS 3110 is configured to generate unique access codes on request from the user (via the multi-purpose device 130), the EMR system 1865, or other services and components of the remote application server 155. The AACS 3110 can also be configured to validate access codes, as described herein, when received from the same.

In some examples, the access code can be a globally unique identifier (GUID) or a code that is at least unique within the context of the remote application server 155. In some examples, the access code can include a relatively long series of characters to facilitate this uniqueness. In some examples, the access code is only valid for a limited amount of time, such as 60 minutes, 30 minutes, 20 minutes, 10 minutes, 5 minutes, etc. Because the access code is only valid for a limited period of time, the length and complexity of the code can be reduced while still maintaining the uniqueness of the code (e.g., during the prescribed period of time). As an example, with a short period of validity, the access code can be reduced to a code that is easily remembered and typed by the patient. In some examples, the access code can be reduced to a six-digit numeric code or a four-digit alphanumeric code, among other possible lengths and constructions. A short access code is permissible because there is expected to only be a small number of patient accounts seeking connections during the allotted time period and codes can be reused after their validity time period has expired.

The dataflow 3500 initiates with the multi-purpose device 130 being configured to display a user interface element (e.g., a button or a menu item) that initiates the connection or association process. Upon receiving a selection of the user interface element, the multi-purpose device 130 sends a request 3502 to the AACS 3110 for an access code. The AACS 3110 generates the access code upon receiving the request 3502 and associates the generated access code with the patient account associated with the multi-purpose device 130. As an example, the remote application server 155 can uniquely associate the multi-purpose device 130 with a particular patient account or set of patient records. Additionally or alternatively, the remote application server 155 can associate an account logged into the application executing on the multi-purpose device 130 with the patient account or database records, or can associate a particular unique identifier with both the multi-purpose device 130 and specific patient records in the remote application server 155. Other associations are also possible in other examples.

The AACS sends a response 3504 to the multi-purpose device 130 including the access code. The multi-purpose device 130 displays the access code. The access code is then entered into a requested field on the EMR system 1865, for example in a comment field of the order or in a dedicated field of the EMR system 1865 user interface. The EMR system 1865 can receive the access code as a result of the patient showing or sending the displayed access code to their HCP. The EMR system 1865 sends a request 3507 to the remote application server 155 including the access code. As an example, the request 3507 can be an order for an analyte report (in which case the first order request will also be used to establish a connection or association). As another example, the request 3507 can be a request for connection or association only (e.g., not including a request for an analyte report, which can be separately requested later). The AMS 3120 receives the request 3507 on behalf of the remote application server 155 and sends the access code received in the request 3507 as a validation request 3508 to the AACS 3110.

The AACS 3110 determines if the access code is valid. For example, the AACS 3110 can determine if the access code matches one that has been previously generated, whether the access code is still within its validity time period, and whether the access code is associated with the patient account records for which access is being requested in the request 3507. If the access code is valid, the AACS 3110 sends a response 3509 to the AMS 3120 with an approval for access to the patient account records and the account identification information. The AMS 3120 sends a response 3511 to the EMR system 1865 with a PIC. As described herein, the PIC is to be retained as a shared identifier for a particular patient's records in both the remote application server 155 and the EMR system 1865. The EMR system 1865 stores this PIC and associates it with the patient's EMR account. In some examples, the EMR system 1865 generates the PIC and include the PIC in the request 3507. The remote application server 155 can store the PIC with the patient's account in AMS 3120. If the request 3507 includes an analyte report request, the AMS 3120 can also respond with one or more reports generated from analyte data associated with the patient, as described herein. If the AACS 3110 determines that the access code included in the request 3507 (and related to the AACS 3110 by the AMS 3120) is invalid, then the AACS 3110 response 3509 to the AMS 3120 is a disapproval for access. The AMS 3120 response 3511 to the EMR system 1865 includes a connection or association failure message as described herein.

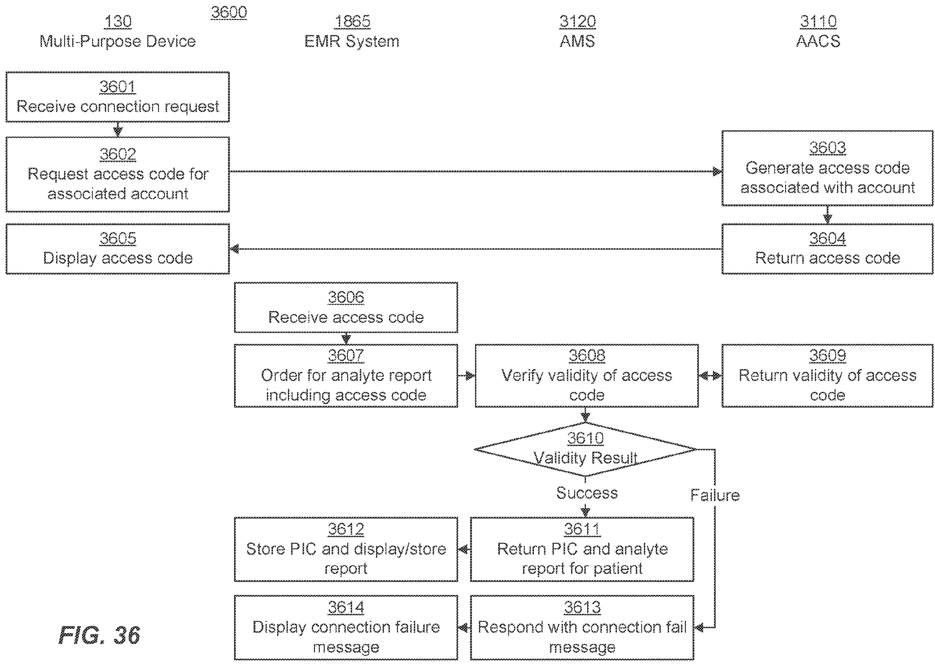

FIG. 36 illustrates a procedure for requesting and generating a report according to certain embodiments. The procedure 3600 illustrated in FIG. 36 corresponds to the dataflow 3500 illustrated in FIG. 35. At 3601, the multi-purpose device 130 receives a request to establish a connection or association between the patient record in an EMR system 1865 and the remote application server 155. The multi-purpose device 130 can be part of an analyte monitoring system 100, for example, a glucose monitoring system. The multi-purpose device 130 can comprise a display, one or more processors, a camera (or other hardware to scan a matrix barcode) and a memory communicatively coupled with the one or more processors. The memory can comprise instructions that, when executed by the one or more processors are configured to cause the one or more processors to perform operations comprising one or more operations of the procedure 3600, for example operations 3601, 3602, and 3605. At 3602, the multi-purpose device 130 generates and sends a request for an access code for the account associated with the multi-purpose device 130. As another example, the multi-purpose device 130 generates a request for an access code for the account associated with the instance of a specific application executing on the multi-purpose device 130 or with an account logged into the instance of the application. The request is sent to the AACS 3110.

At 3603, the AACS 3110 generates the access code associated with the appropriate account. The AACS 3110 can be a part of an analyte monitoring system 100, for example, a glucose monitoring system. The AACS 3110 can be or include a server or server computing device comprise networking hardware, one or more processors, and a memory communicatively coupled with the one or more processors. The memory can comprise instructions that, when executed by the one or more processors are configured to cause the one or more processors to perform operations comprising one or more operations of the procedure 3600, for example operations 3603, 3604, and 3609. At 3604, the AACS 3110 returns the access code to the multi-purpose device 130. An example of displaying the access code and URL is shown and described in FIG. 20 and in FIG. 25 with respect to an example in which the user scans a matrix barcode displayed by a data receiving device 120. At 3605, the multi-purpose device 130 displays the received access code. The patient can then display the access code to the HCP operating the EMR system 1865.

At 3606, the EMR system 1865 receives the access code. The EMR system 1865 can be in communication with or a part of an analyte monitoring system 100, for example, a glucose monitoring system. The EMR system 1865 can be or include a server or server computing device comprise networking hardware, one or more processors, and a memory communicatively coupled with the one or more processors. The memory can comprise instructions that, when executed by the one or more processors are configured to cause the one or more processors to perform operations comprising one or more operations of the procedure 3600, for example operations 3606, 3607, 3612, and 3614. For example, the HCP can enter the access code to the EMR system 1865. At 3607, the EMR system 1865 generates an order for an analyte report corresponding to the patient's analyte data stored by the remote application server 155. At 3608 and 3609 the AMS 3120 and 3110 (respectively) coordinate to verify the access code received from the EMR system 1865 with the access code generated by the AACS 3310.

At 3610, the AMS 3120 evaluates the validity of the result of 3608. The AMS 3120 can be a part of an analyte monitoring system 100, for example, a glucose monitoring system. The AMS 3120 can be or include a server or server computing device comprise networking hardware, one or more processors, and a memory communicatively coupled with the one or more processors. The memory can comprise instructions that, when executed by the one or more processors are configured to cause the one or more processors to perform operations comprising one or more operations of the procedure 3600, for example operations 3608, 3610, 3611, and 3613. If at 3610 the AMS 3120 finds a valid result, then the procedure proceeds to 3611. At 3611, the AMS 3120 (or other component of the remote application server 155) generates a report based on analyte data associated with the patient identified with the PIC. At 3611, the AMS 3120 sends the generated report to the EMR system 1865. In some examples, the AMS sends 3120 and/or the EMR system 1865 displays a confirmation of the association or connection of the analyte data associated with the patient and the data associated with the patient and stored by the EMR system 1865. At 3612, the EMR system 1865 displays and/or stores the generated report. If at 3610, the AMS 3120 finds an invalid result, then the procedure proceeds to 3613. At 3613, the AMS 3120 responds to the EMR system 1865 with a message indicating that the AMS system 3120 was not able to connect the received PIC with an existing patient account. At 3614, the EMR system 1865 displays the connection or association failure message and instructions to establish the connection or association.

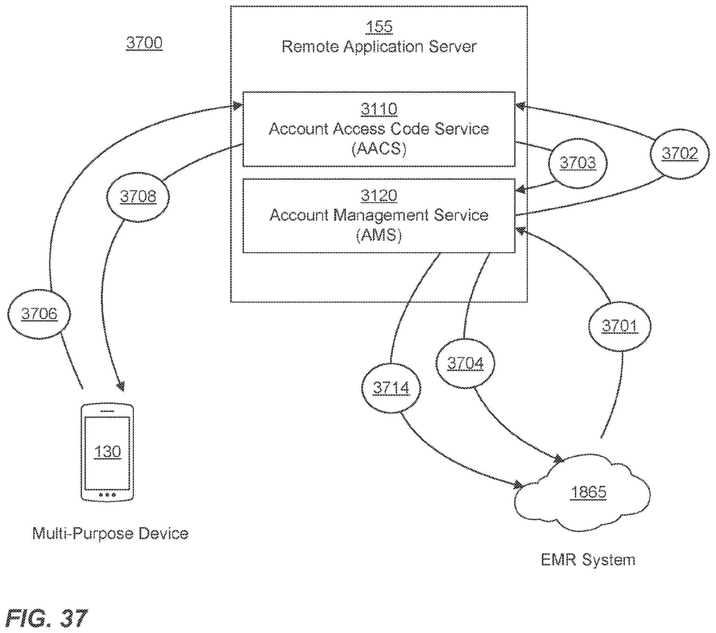

FIG. 37 illustrates an example dataflow for an analyte monitoring system. The dataflow 3700 corresponds to a procedure for establishing a connection or association between patient data stored in a remote application server 155 and EMR system 1865 and optionally requesting analyte reports based on that data according to some examples. In the illustrated dataflow 3700, the connection or association request is initiated from an order request 3701 sent by the EMR system 1865. In some examples, the order request 3701 can include patient identifying information, such as patient name, date of birth, and contact information such as an email address. The AMS 3120 attempts to find a match in its account database based on the patient information. If the AMS 3120 fails to find a match, then AMS 3120 sends a message to the contact information (e.g., email address) provided by the EMR system 1865. In some examples, the AMS 3120 does not perform matching and instead, if the connection or association hasn't been established, sends the message to the contact information provided by the EMR system 1865. For patients with a multi-purpose device 130, the patient can receive the message on their multi-purpose device 130. The message contains instructions for the patient to click on a link in the message that will open a webpage or a specific portion of the application associated with the remote application server 155 and executing on the multi-purpose device 130. The webpage is configured to display an access code entry field along with instructions for the patient.

The patient can then use the multi-purpose device 130 to send a request 3702 to the AACS 3110 to generate an access code. The AACS 3110 generates an access code as described herein and sends the access code back to the multi-purpose device 130 in a response 3703 for display. When the patient enters the access code into the webpage, the webpage sends the access code to the AMS 3120, which will include it in an access request 3705 to the AACS 3110 on behalf of the ERM system 1865. If the AACS determines that the access code is valid, it will respond 3706 to the AMS with access approval and the account information for the patient. The AMS the establishes the connection or association as described herein and optionally responds 3707 to the EMR system 1865 with a PIC, analyte report, or connection or association failure message according to the techniques described herein.

In this example, the patient and HCP do not necessarily need to be in near-real-time contact for the EMR system 1865 to gain access, but still retains the security benefits of the access code-based approach. As an example, in the dataflow 3500, the patient grants access to the EMR system 1865 during or just prior to a medical appointment. The HCP can ensure the patient follows through with their required actions because the process will require them to be in near-real-time contact. In contrast, in the dataflow 3700, the patient locates the requests the access code from the AACS 3110 and enters the access code into the webpage relatively asynchronously with their interactions with an HCP. As an example, the HCP can request access prior to a medical appointment and the patient can complete the process at home, maximizing time for the HCP to interact with the patient during the appointment. In some examples, various components can interact automatically. For example, the webpage to receive the access code can automatically open the application associated with the patient account on the remote application server 155 and brings it to the foreground on the screen of the application that initiates the request 3706 to generate the access code. As another example, when the access code is returned to the application, it can provide a simplified user interface to copy the access code into the entry field on the webpage.

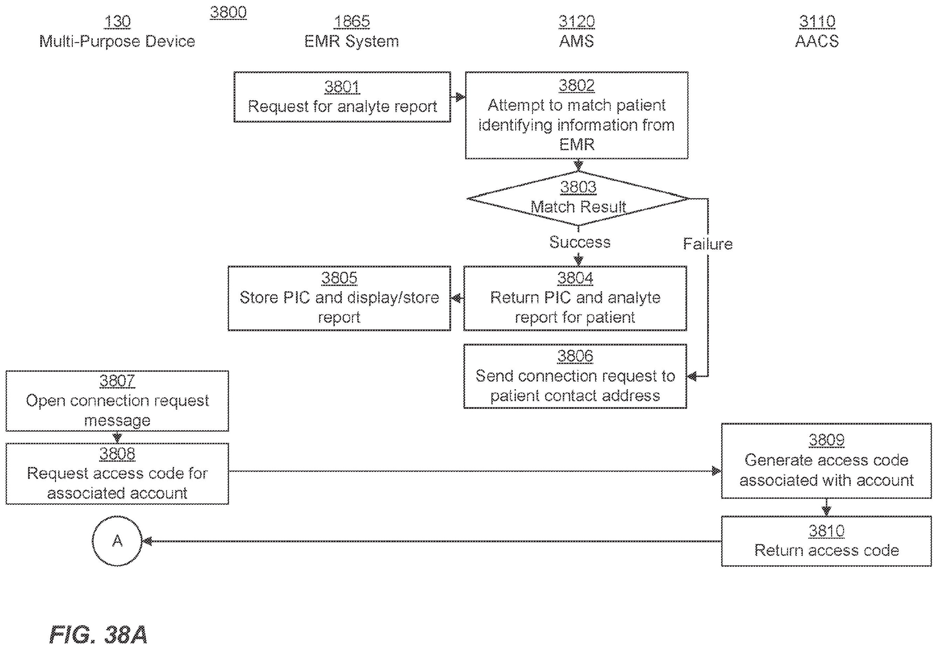

FIGS. 38A-38B illustrate a procedure for requesting and generating a report according to certain embodiments. The procedure 3800 illustrated in FIGS. 38A-38B corresponds to the dataflow 3700 illustrated in FIG. 37. At 3801, the EMR system 1865 receives a request for an analyte report based on a patient record in the EMR system 1865. The EMR system 1865 can be in communication with or a part of an analyte monitoring system 100, for example, a glucose monitoring system. The EMR system 1865 can be or include a server or server computing device comprise networking hardware, one or more processors, and a memory communicatively coupled with the one or more processors. The memory can comprise instructions that, when executed by the one or more processors are configured to cause the one or more processors to perform operations comprising one or more operations of the procedure 3800, for example operations 3801, 3805, 3816, and 3818. As described herein, in some examples the request can instead be a request just to establish a connection or association (e.g., to generate a PIC to be shared by the EMR system 1865 and AMS 3120 or to request the AMS 3120 to use a PIC included in the request). The EMR system 1865 generates and sends the request to the AMS 3120. As described herein, the request includes patient identifying information (e.g., name, contact information, etc.) but does not include a PIC as one has not yet been stored by the EMR system 1865.

At 3802, the AMS 3120 attempts to match the received patient identifying information with corresponding information stored in a patient account at the remote application server 155. The AMS 3120 can be a part of an analyte monitoring system 100, for example, a glucose monitoring system. The AMS 3120 can be or include a server or server computing device comprise networking hardware, one or more processors, and a memory communicatively coupled with the one or more processors. The memory can comprise instructions that, when executed by the one or more processors are configured to cause the one or more processors to perform operations comprising one or more operations of the procedure 3800, for example operations 3802, 3803, 3804, 3806, 3813, 3814, 3815, and 3817. At 3803, the AMS 3120 evaluates the result of the match attempt. If the attempt is successful, then an existing patient record is available. At 3120, the AMS 3120 generates a new PIC for the patient data and/or retrieves an existing PIC and generates an analyte report based on the analyte data associated with identified patient account. The AMS 3120 sends the analyte report and PIC to the EMR system. 1865. At 3805, the EMR system 1865 stores the PIC and displays and/or stores the analyte report.

If, at 3803, the AMS 3120 determines that the attempt to match provided patient data with existing data is unsuccessful, then, at 3806, the AMS 3120 sends a connection or association request to the patient. As an example, the AMS 3120 can access existing contact information for the patient from its patient records (e.g., email address, phone number, etc.) or as provided by the EMR system 1865. At 3807, having received the connection or association request message, the multi-purpose device 130 opens the connection or association request message and displays the message to the patient. The multi-purpose device 130 can be part of an analyte monitoring system 100, for example, a glucose monitoring system. The multi-purpose device 130 can comprise a display, one or more processors, a camera (or other hardware to scan a matrix barcode) and a memory communicatively coupled with the one or more processors. The memory can comprise instructions that, when executed by the one or more processors are configured to cause the one or more processors to perform operations comprising one or more operations of the procedure 3800, for example operations 3807, 3808, 3811, and 3812. As described, the connection or association request message includes an interface element or other affordance through which the patient can continue the connection or association request process. At 3808, the multi-purpose device 130 generates and sends a request for an access code for the account associated with the multi-purpose device 130. As another example, the multi-purpose device 130 can generate a request for an access code for the account associated with the instance of a specific application executing on the multi-purpose device 130 or with an account logged into the instance of the application. The request is sent to the AACS 3110.

At 3809, the AACS 3110 generates the access code associated with the appropriate account. The AACS 3110 can be a part of an analyte monitoring system 100, for example, a glucose monitoring system. The AACS 3110 can be or include a server or server computing device comprise networking hardware, one or more processors, and a memory communicatively coupled with the one or more processors. The memory can comprise instructions that, when executed by the one or more processors are configured to cause the one or more processors to perform operations comprising one or more operations of the procedure 3800, for example operations 3809, and 3810. At 3810, the AACS 3110 returns the access code to the multi-purpose device 130.

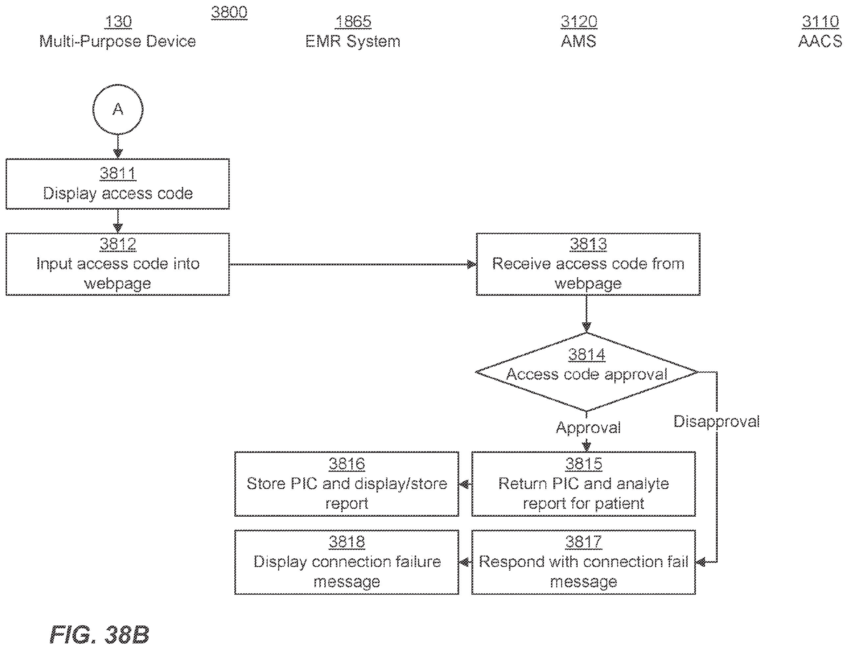

The procedure 3800 continues from placeholder A as illustrated in FIG. 38B. At 3811, the multi-purpose device 130 displays the received access code. The patient, for example, through the connection or association request message can be instructed to visit a specific webpage configured to receive access codes associated with their analyte data and/or to establish a connection or association between their analyte data and their HCP's EMR system 1865. In some examples, the webpage automatically opens after the user submits the request for an access code. In some examples, the webpage can be a destination within an application associated with the analyte monitoring system or EMR system 1865. An example of displaying the access code and URL is shown and described in FIG. 20 and in FIG. 25 with respect to an example in which the user scans a matrix barcode displayed by a data receiving device 120. At 3812, the patient inputs the access code into the webpage.

At 3813, the AMS 3120 receives the access code from the webpage. For example, the webpage can be provided by the analyte monitoring system such that data entered into the webpage is provided automatically to the AMS 3120 along with a request (or a reference to a request) for an analyte report. The AMS 3120 coordinates with the AACS 3110 to evaluate the validity of the request and access code and determine whether to approve the request. At 3814, the AMS 3120 evaluates whether to approve the request for the analyte report based on the access code. If at 3814 the AMS 3120 approves the access code, then the procedure proceeds to 3815. At 3815, the AMS 3120 (or other component of the remote application server 155) generates a report based on analyte data associated with the patient identified with the PIC. At 3815, the AMS 3120 sends the generated report to the EMR system 1865. At 3816, the EMR system 1865 displays and/or stores the generated report. In some examples, the AMS sends 3120 and/or the EMR system 1865 displays a confirmation of the association or connection of the analyte data associated with the patient and the data associated with the patient and stored by the EMR system 1865. If at 3814, the AMS 3120 disapproves the access code, then the procedure proceeds to 3817. At 3817, the AMS 3120 responds to the EMR system 1865 with a message indicating that the AMS system 3120 was not able to connect the received PIC with an existing patient account. At 3818, the EMR system 1865 displays the connection or association failure message and instructions to establish the connection.

In some examples, the analyte monitoring system 100 provides the ability to use a proprietary device, such as a data receiving device 120 that is not connected to the internet. This data receiving device 120 is used to communicate to the analyte sensor. Data are typically uploaded from a data receiving device 120 via a wired connection, e.g. USB, to an Internet-connected user device 140 to via another connection to a multi-purpose device 130. As described herein, in some examples, data can be transferred from the data receiving device 120 to a multi-purpose device 130 using a matrix barcode.

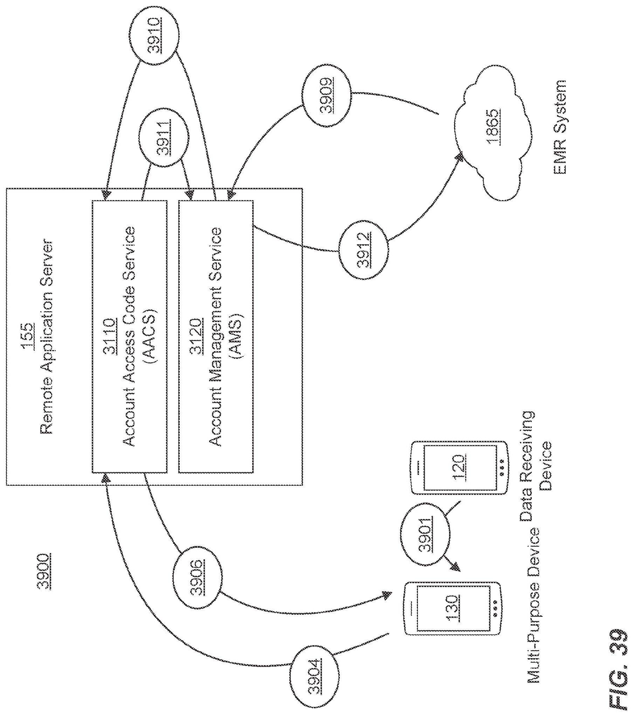

FIG. 39 illustrates an example dataflow for an analyte monitoring system. The dataflow 3900 corresponds to a procedure for establishing a connection or association between patient data stored in a remote application server 155 and EMR system 1865 and optionally requesting analyte reports based on that data according to some examples for a patient using a data receiving device 120. This procedure utilizes a separate multi-purpose device 130 device to facilitate the communication between the data receiving device 120 and the remote application server 155. As an example, the multi-purpose device 130 can be embodied as a smartphone, configured with a camera and a software to scan, interpret, and execute matrix barcodes (e.g., QR codes) as described herein.

The data receiving device 120 software is configured to provide a user interface to the user to initiate the process. When the patient selects this user interface, the data receiving device 120 software generates and displays a matrix barcode which contains a URL address to a webpage that communicates with the AACS 3110 (or other suitable components of the remote application server 155). The user scans 3901 the matrix barcode with the multi-purpose device 130. The multi-purpose device 130 then sends a request 3904 to the AACS 3110 including the data stored in the matrix barcode. As described herein, the data stored in the matrix barcode can include, for example, data receiving device 120 device identifier, glucose data, and/or glucose report calculations for the patient. The multi-purpose device 130 can initiate the request directly (e.g., using programming in the application executing on the multi-purpose device and associated with the remote application server 155). The multi-purpose device 120 can initiate the request indirectly, for example, by executing a URL included in the matrix barcode to access a webpage configured to initiate the request 3904. The AACS 3110 generates an access code, associates it with the patient account associated with the data receiving device 120 (or otherwise associated with the multi-purpose device), and returns the access code in a response 3906. The multi-purpose device displays the access code (e.g., in a user interface of the application or in the webpage).

The patient provides the access code to the HCP (e.g., a doctor, assistant, other clinic personnel). The HCP enters the access code in the EMR system 1865. For example, the HCP can initiate an order requesting an analyte report. The EMR system 1865 sends a request 3909 for the analyte report to the AMS 3120. The request 3909 includes the access code and optionally other identifying information for the patient (e.g., patient name, unique identifier, PIC). The AMS 3120 provides the access code to the AACS 3110 in a verification request 3910. If the AACS 3110 determines that the access code is valid, the AACS3110 provides a response 3911 to the AMS 3120 with an access approval message and information to facilitate retrieving the patient's analyte data and/or an analyte report based on the data. For example, the response 3911 can include the PIC (retrieving from the patient record or if provided in the order request 3909). The AMS 3120 can proceed to establish the connection or association between patient records in the remote application server 155 and the EMR system 1865 as described herein. The AMS 3120 can provide a response 3912 to the EMR system with information confirming the connection or association. The response 3912 can include the requested analyte report. Once the patient accounts maintained by the remote application server 155 and EMR system 1865 are connected, the EMR system can request reports at any time as described herein.

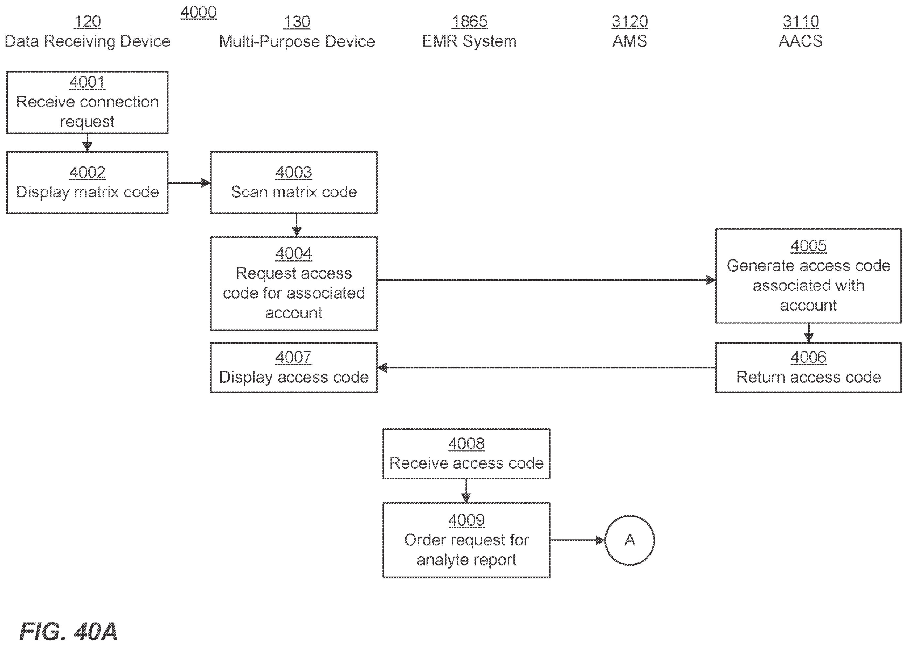

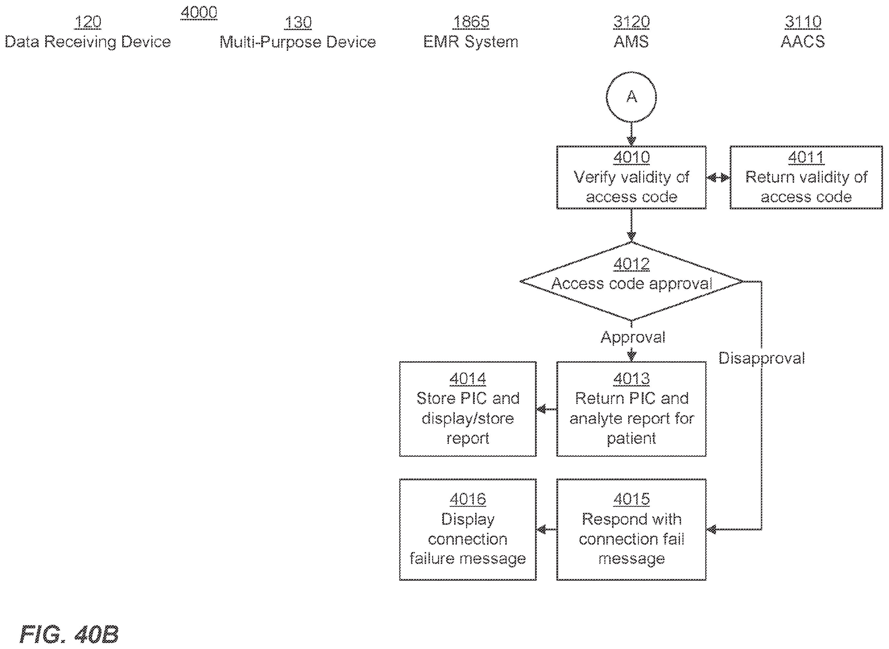

FIGS. 40A-40B illustrate a procedure for requesting and generating a report according to certain embodiments. The procedure 4000 illustrated in FIGS. 40A-40B corresponds to the dataflow 3900 illustrated in FIG. 39. At 4001, the data receiving device 4001 receives a request to enable a connection between their patient data stored by the EMR system 1865 and their analyte data stored by the AMS 3120, including data that can be transferred from the data receiving device 120 to a multi-purpose device 130 and uploaded to the AMS 3120. The data receiving device 120 can be part of an analyte monitoring system 100, for example, a glucose monitoring system. The data receiving device 120 can comprise a display, one or more processors, and a memory communicatively coupled with the one or more processors. The memory can comprise instructions that, when executed by the one or more processors are configured to cause the one or more processors to perform operations comprising one or more operations of the procedure 4000, for example operations 4001 and 4002. In response to receiving the request, the data receiving device 120 generates a matrix barcode containing information to facilitate the connection or association request. As described herein, the matrix barcode can include a URL of a web end point provided by the remote application server 155. The matrix barcode can include instructions or parameters relevant to initiating the connection or association. For example, the matrix barcode can include information identifying the patient or the data receiving device 120. At 4002 the data receiving device 120 displays the matrix barcode within a user interface. The user interface can include instructions for the patient to scan the matrix barcode using a camera of their multi-purpose device 130. An example of a data receiving device 120 displaying a matrix barcode for scanning is illustrated in FIG. 24.

At 4003, the multi-purpose device 130 scans the matrix barcode at the instruction of the patient or another user. The multi-purpose device 130 can be part of an analyte monitoring system 100, for example, a glucose monitoring system. The multi-purpose device 130 can comprise a display, one or more processors, a camera (or other hardware to scan a matrix barcode) and a memory communicatively coupled with the one or more processors. The memory can comprise instructions that, when executed by the one or more processors are configured to cause the one or more processors to perform operations comprising one or more operations of the procedure 4000, for example operations 4003, 4004, and 4007. In some examples, the multi-purpose device includes a camera and software to automatically scan and execute matrix barcodes when detected within the viewfinder of the camera. In some examples, an application associated with the analyte monitoring system and executing on the multi-purpose device 130 includes a scanning function to detect and execute matrix barcodes of the type displayed by the data receiving device 120. As described herein, the multi-purpose device 130 has hardware sufficient to support internet connectivity and is able to connect to the remote application server 155 through one or more networks. At 4004, the multi-purpose device 130 generates and sends a request for an access code for the account associated with the multi-purpose device 130. The request can be generated based on the data encoded within the matrix barcode. In some examples, the request can be generated by the multi-purpose device 130 executing the URL encoded within the matrix barcode. The request is sent to the AACS 3110.

At 4005, the AACS 3110 generates the access code associated with the appropriate account. The AACS 3110 can be a part of an analyte monitoring system 100, for example, a glucose monitoring system. The AACS 3110 can be or include a server or server computing device comprise networking hardware, one or more processors, and a memory communicatively coupled with the one or more processors. The memory can comprise instructions that, when executed by the one or more processors are configured to cause the one or more processors to perform operations comprising one or more operations of the procedure 4000, for example operations 4005, 4006, and 4011. At 4006, the AACS 3110 returns the access code to the multi-purpose device 130. At 4007, the multi-purpose device 130 displays the access code within its user interface. An example of displaying the access code and URL is shown and described in FIG. 20 and in FIG. 25 with respect to an example in which the user scans a matrix barcode displayed by a data receiving device 120.

At 4008, the EMR system 1865 receives the access code. The EMR system 1865 can be in communication with or a part of an analyte monitoring system 100, for example, a glucose monitoring system. The EMR system 1865 can be or include a server or server computing device comprise networking hardware, one or more processors, and a memory communicatively coupled with the one or more processors. The memory can comprise instructions that, when executed by the one or more processors are configured to cause the one or more processors to perform operations comprising one or more operations of the procedure 4000, for example operations 4008, 4009, 4014, and 4016. For example, the patient can provide the access code to an HCP or other personnel operating the EMR system 1865. The HCP can enter the access code into the EMR system 1865, such as in a specified input field or in a comment section associated with the patient's records. The HCP can enter the access code in the context of making a request to the AMS 3120 for analyte data of the patient. At 4009, the EMR system 1865 submits an order request for an analyte report for the patient. The EMR system 1865 can include the access code, retrieved from the multi-purpose device 130 with the order request. An example of a EMR system 1865 requesting an access code to access patient analyte data is illustrated in FIG. 27.

After receiving the order request, at 4010 the AMS 3120 attempts to verify the validity of the access code with the AACS 3110. The AMS 3120 can be a part of an analyte monitoring system 100, for example, a glucose monitoring system. The AMS 3120 can be or include a server or server computing device comprise networking hardware, one or more processors, and a memory communicatively coupled with the one or more processors. The memory can comprise instructions that, when executed by the one or more processors are configured to cause the one or more processors to perform operations comprising one or more operations of the procedure 4000, for example operations 4010, 4012, 4013, and 4015. For example, the AMS 3120 can provide the access code to the AACS with a verification request. The verification request can further include patient identifying information included with the order request. The AACS 3110 can coordinate with the AMS 3120 at 4011 to return a validity determination of the access code. At 4012, the AMS 3120 evaluates the validity of the result of 4010 and 4011. If at 4012 the AMS 3120 approves the access code, then the procedure proceeds to 4013. At 4013, the AMS 3120 (or other component of the remote application server 155) generates a report based on analyte data associated with the patient identified with the PIC. At 4013, the AMS 3120 sends the generated report to the EMR system 1865. At 4014, the EMR system 1865 displays and/or stores the generated report. In some examples, the AMS sends 3120 and/or the EMR system 1865 displays a confirmation of the association or connection of the analyte data associated with the patient and the data associated with the patient and stored by the EMR system 1865. If at 4012, the AMS 3120 disapproves the access code, then the procedure proceeds to 4015. At 4015, the AMS 3120 responds to the EMR system 1865 with a message indicating that the AMS system 3120 was not able to connect the received PIC with an existing patient account. At 4016, the EMR system 1865 displays the connection or association failure message and instructions to establish the connection or association.

As previously described herein, one challenge for patients using a data receiving device 120 is a requirement to physically connect the data receiving device 120 to another user device 140 capable of uploading analyte data collected by the data receiving device 120. Often, the patient installs proprietary software on the user device 140 to facilitate the data transfer between the data receiving device 120 and the user device 140. The proprietary software can also establish the connection or association between the user device 140 and the remote application server 155. Often, patients fail to regularly connect their data receiving device 120 to the user device 140 as the data receiving device 140 includes functionality sufficient for the patient to maintain their health by providing insight into analyte levels measured by the analyte sensor. In this scenario, even if patient accounts maintained by the EMR system 1865 and remote application server 155 are connected, the remote application server 155 does not have sufficient or up-to-date data to provide a report.

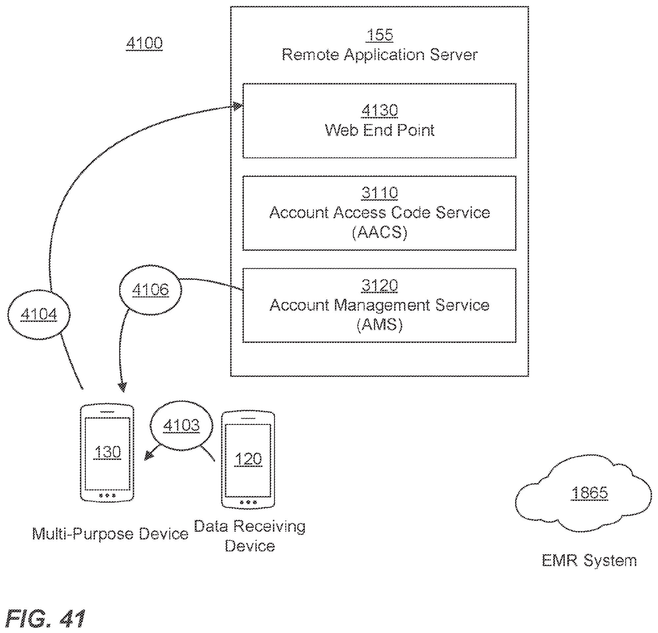

FIG. 41 illustrates an example dataflow for an analyte monitoring system. As an example, FIG. 41 illustrates a dataflow 4100 to facilitate upload of data from data receiving device 120 to remote application server 155 using a multi-purpose device 130 as an intermediary. The data receiving device 120 software is configured with the previously described UI mechanism to facilitate data upload or transfer between the data receiving device 120 and the multi-purpose device 130 via a matrix barcode. As an example, this can take the form of the previously described "share reports" feature. When the patient selects the "share reports" interface element, the data receiving device 120 generates and displays matrix barcode. Embedded within the matrix barcode is analyte data and/or calculations, an identifier for the data receiving device 120 or the patient, and a URL to an available web end point 4130 in remote application server 155 that is configured to accept analyte data. In some examples, as described herein, the web end point 4130 can be provided through a report generation system 1860 associated with the remote application server 155. The patient, or another user, scans the matrix barcode with a multi-purpose device 130 and receives the matrix barcode payload 4103. The multi-purpose device 130 decodes the matrix barcode and executes the URL to send the payload 4104 to the web end point 4130. The remote application server 155 receives the analyte data, finds the patient account associated with the identifier of the data receiving device 120 and stores the data in the patient account. In this example, the patient can perform the procedure at any convenient time because they are not required to physically connect the data receiving device 120 to a specific user device 140 executing a specialized application. In some examples, when the remote application server 155 receives a request from the EMR system 1865 to generate an analyte report, but there is insufficient data to generate the report, the remote application server 155 can respond with the notice to the EMR system 1865 that there is insufficient data. Additionally or alternatively, the remote application server 155 can alert the user (e.g., through a contact message to one or more contact addresses or phone numbers) when insufficient analyte data is available to generate a report. The response to the EMR system 1865 or contact message to the patient can include instructions for performing the upload activity as described.

In some examples, the contact message to the patient can be delivered via one or more communication channels, including but not limited to email messages, short-message service (SMS) messages, automated voice messages or phone calls, third-party microblogging services, or other communication channels selected by the patient to receive contact messages or selected by the HCP or EMR system 1865 to deliver contact messages. In some examples, the contact message can be delivered via an in-application message through an application executing on the multi-purpose device 130. For example, the application can be a messaging application selected by the patient. As another example, the application can be associated with the analyte monitoring system 100 and used by the patient or another user to monitor analyte levels and alerts. In this example, when the message is delivered via in-application messaging, the patient receives the message directly through a trusted application and can easily verify that the message is from their HCP.

In some examples, the patient receives an invitation to connect their data stored by the analyte monitoring system 100 (e.g., within the AMS 3120) to a EMR system 1865 or other health system. The patient receives a contact message from the HCP, EMR system 1865, or a representative thereof. The patient can receive the contact message on a multi-purpose device 130 or a user device 140. As described herein, the contact message can be in the form of an email, SMS message, in-application message, or the like. The contact message invites the patient to connect their patient data to the EMR system 1865. For example, the contact message can include a link or other interactive element. The link can launch a web browser and load a corresponding invite webpage with instructions on how to enable the connection or association.

When the link is selected, a request for the invite webpage is sent to the analyte monitoring system 100, for example to a web end point provided by the analyte monitoring system 100 or another component thereof. The invite webpage is returned to the calling browser which displays instructions to the patient and an entry field to receive a data access code. For example, the webpage can include an input field for receiving an access code and other input to identify the patient's account with the AMS 3120 or EMR system 1865. The other input can include a request for the patient to enter their account credentials (e.g., user ID, username, password) and other verification information (e.g., social security number, insurance identification number, phone number). In some examples, the link includes parameters including the account access credentials for the patient's account such that when launched, patient account identifying information is provided to the webpage automatically, saving the patient the burden of entering in patient information. The analyte monitoring system 100 (e.g., the AACS 3110) retrieves and stores the patient's account credentials from the parameters included in the link. The patient can be instructed to request and generate an access code to associate with the authorize requesting by the HCP.

The patient can generate an access code using any of the techniques described herein. Additionally or alternatively, the software application executing on the multi-purpose device 130 and associated with the analyte monitoring system 100 can include a menu within its UI for generating data access codes separate from report sharing access codes. For example, the patient can be instructed to navigate to a designated page or UI within the software application and interact with a UI element to request and receive a "data access" code. The data access code may be generated by an account access server, such as the AACS 3110, upon receiving a request from the multi-purpose device 130 according to the techniques described herein. The data access code, like the other access codes described herein may be a short (e.g., 4-8 character) easy to read and remember code (e.g., comprising alphanumeric characters) with a limited time of validity. The data access code is unique within the timeframe of its validity. For example, if the time of validity is 15 minutes, then no other data access code matching the data access code is generated for 15 minutes (or more to account for a buffer time period) or until the data access code is used by the patient. After the validity time period and optional buffer time period expire, another data access code matching the previously generated data access code may be generated.

The patient can be instructed to enter the data access code into the appropriate entry field displayed in the web browser or other UI activated when the user selected the link in the contact message. The patient then enters the data access code into the access code field. In some examples, the invite webpage can further include a submit or save button which causes the webpage to send the entered data access code. In some examples, the webpage does not include a save or submit button, instead the text entered into the access code field is submitted automatically when a sufficient amount of input (e.g., input of a specified length or satisfying character requirements) is detected as being entered into the entry field. Upon submitting the data access code, the webpage causes the data access code to be provided to the AACS 3110. The AACS 3110 evaluates the validity of the data access code and confirms that the data access code was generated for the particular patient's data. If the access code is valid (e.g., has not yet expired) and associated with the patient, the AMS 3120 can be instructed to connect to the EMR system 1865 and initiate a connection or association between the patient's data stored by the AMS 3120 and the EMR system 1865. Additionally or alternatively, the contact message can be generated automatically when a request is received by the AMS 3120 and initiated by the EMR system 1865. Upon affirming the validity of the data access code, the AMS 3120 can then match the approval represented by the input of the data access code by the user with a pending connection or association request from the EMR system 1865 and initiate the connection or association. The webpage can then update to display a confirmation that the patient's data has been successfully connected to the EMR system 1865 and made available to their HCP. If no match to the entered data access code is found, or if the data access code is determined to be invalid, then the webpage is updated, or a new webpage is sent to the web browser with a message indicating the failure to connect. This process may also be used to send data and reports to the browser where the access code was entered, according to examples described herein.

Note that even in examples where a separate user device 140 is used to upload data from the data receiving device 120, the remote application server 155 can use the access code techniques described herein to ensure that the data being uploaded is directed to the correct user account. As an example, the software executing on the user device 140 can request the patient to provide login credentials to authenticate themselves to the remote application server 155. To facilitate the login, without the user being required to supply a username and password, the patient can receive an email or other contact message, as described herein. The patient can execute a link provided in the email. The patient then uses the software application executing on the user device 140 or optionally the multi-purpose device 140 to request an access code from the remote application server 155. The user device 140 displays an access code received from the AACS 3110 and displays it to the patient. The patient copies or otherwise enters the access code to the webpage opened via the link in the contact message and submits it to the remote application server 155. The AACS 3110 determines if the access code is valid and if so, stores data received from the user device 140 with the patient account associated with the valid access code. Through this process the patient is not required to enter a lengthy username and password, yet security of the patient's data is maintained.

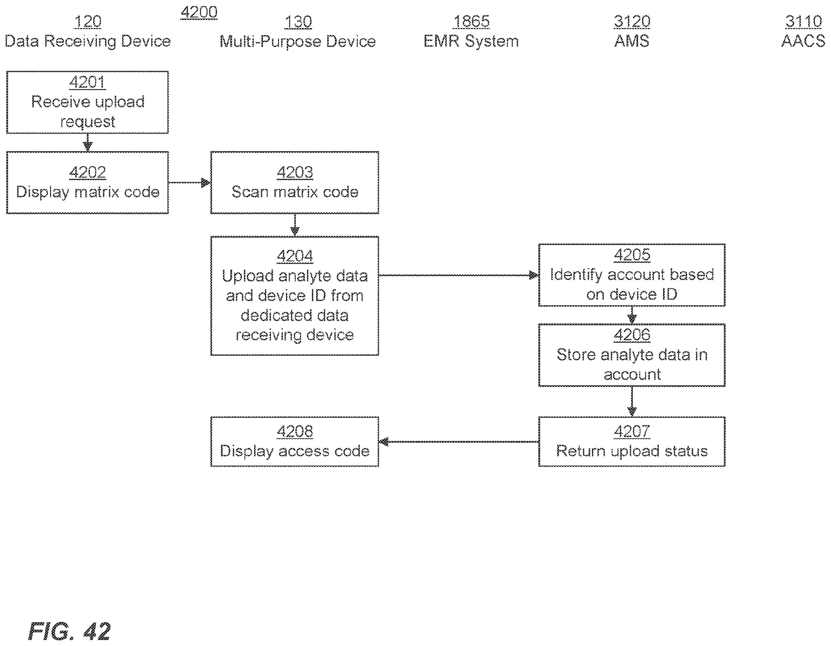

FIG. 42 illustrates a procedure for requesting and generating a report according to certain embodiments. The procedure 4200 illustrated in FIG. 42 corresponds to the dataflow 4100 illustrated in FIG. 41. At 4201, the data receiving device 4001 receives a request to upload analyte data stored by the data receiving device 120 to the AMS 3120. The data receiving device 120 can be part of an analyte monitoring system 100, for example, a glucose monitoring system. The data receiving device 120 can comprise a display, one or more processors, and a memory communicatively coupled with the one or more processors. The memory can comprise instructions that, when executed by the one or more processors are configured to cause the one or more processors to perform operations comprising one or more operations of the first procedure 4200, for example operations 4201 and 4202. In particular, the data receiving device 120 can receive a request for a "share reports" function as described herein. In response to receiving the request, the data receiving device 120 generates a matrix barcode containing information to facilitate the transfer and upload of select data from the data receiving device 120. As described herein, the matrix barcode can include a URL of a web end point provided by the remote application server 155. The matrix barcode can include instructions or parameters relevant to uploading the data. For example, the matrix barcode can include information identifying the patient or the data receiving device 120. Additionally, the matrix barcode can include analyte data encoded for upload according to examples disclosed herein. At 4202 the data receiving device 120 displays the matrix barcode within a user interface. The user interface can include instructions for the patient to scan the matrix barcode using a camera of their multi-purpose device 130. An example of a data receiving device 120 displaying a matrix barcode for scanning is illustrated in FIG. 24.

At 4203, the multi-purpose device 130 scans the matrix barcode at the instruction of the patient or another user. The multi-purpose device 130 can be part of an analyte monitoring system 100, for example, a glucose monitoring system. The multi-purpose device 130 can comprise a display, one or more processors, and a memory communicatively coupled with the one or more processors. The memory can comprise instructions that, when executed by the one or more processors are configured to cause the one or more processors to perform operations comprising one or more operations of the procedure 4200, for example operation 4203, 4204, and 4208. In some examples, the multi-purpose device 130 includes a camera and software to automatically scan and execute matrix barcodes when detected within the viewfinder of the camera. In some examples, an application associated with the analyte monitoring system and executing on the multi-purpose device 130 includes a scanning function to detect and execute matrix barcodes of the type displayed by the data receiving device 120. As described herein, the multi-purpose device 130 has hardware sufficient to support internet connectivity and is able to connect to the remote application server 155 through one or more networks. An example of a multi-purpose device 130 scanning a matrix barcode displayed by a data receiving device 120 and uploading data encoded in the matrix barcode is illustrated in FIG. 25.

At 4204, the multi-purpose device 130 uploads the analyte data received from the data receiving device 120 and additional information to identify the account to associate with the analyte data. As an example, the upload from the multi-purpose device 130 can include a device identifier for the data receiving device 120. In some examples, the upload can be generated by the multi-purpose device 130 executing a URL encoded within the matrix barcode and generated by the data receiving device 120. The uploaded data is provided to the AMS 3120.

At 4205, the AMS 3120 identifies a patient account record based on the additional information received in the upload, such as the device identifier. The AMS 3120 can be a part of an analyte monitoring system 100, for example, a glucose monitoring system. The AMS 3120 can be or include a server or server computing device comprise networking hardware, one or more processors, and a memory communicatively coupled with the one or more processors. The memory can comprise instructions that, when executed by the one or more processors are configured to cause the one or more processors to perform operations comprising one or more operations of the procedure 4200, for example operations 4205 to 4207. In some examples, if no relevant account records can be identified, the AMS 3120 can create new account records for temporary or permanent association with the patient. At 4206, the AMS 3120 stores the received analyte data in the account records for the patient. During or after storage of the received analyte data in the account records, the AMS 3120 can request the AACS 3110 to generate an access code associated with the uploaded data that has been associated with the patient account. In some examples, the access code can be associated with all data associated with the patient account, data relating to a specific date range associated with the data, or other groupings of the data. The AACS 3110 provides the access code to the AMS 3120. At 4207, the AMS 3120 returns an upload status to the multi-purpose device 130. The upload status can indicate whether the data was successfully associated with a patient record or otherwise stored by the AMS 3120. The upload status can further include the access code generated by the AACS 3110 and associated with the patient accounts. At 4208, the multi-purpose device 130 displays the access code within its user interface. The access code can be displayed with instructions to using the access code to retrieve the analyte data and/or reports generated based on the same. As described herein, the access code can be used by the patient to view a report generated based on the records and/or can provide the access code (as well as the access instructions) to their HCP or another user. An example of displaying the access code and URL is shown and described in FIG. 20 and in FIG. 25 with respect to an example in which the user scans a matrix barcode displayed by a data receiving device 120.

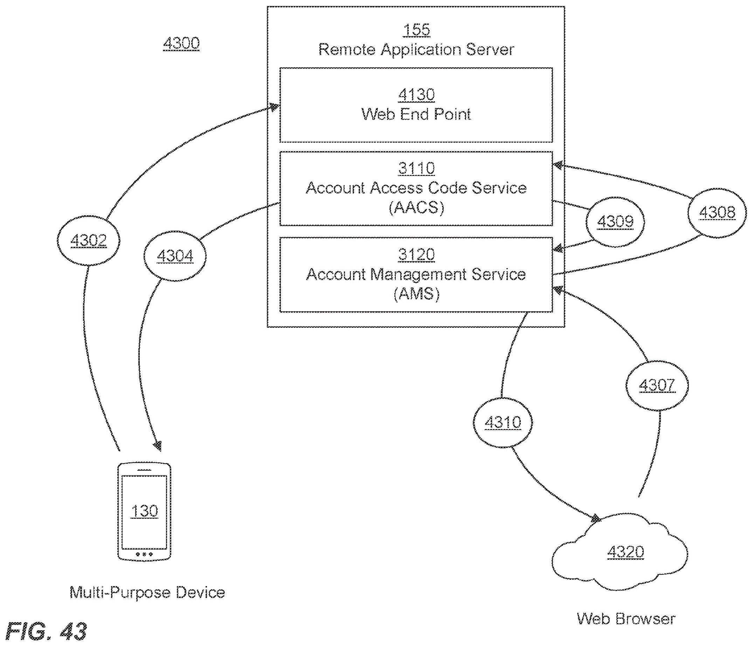

FIG. 43 illustrates an example dataflow for an analyte monitoring system. In particular, FIG. 43 illustrates an example dataflow 4300 wherein integration with an EMR system 1865 is not required. Using a "quick share" function, the patient is able to request time-limited analyte reports on demand. The user can share the generated analyte reports to an HCP or other user. In the dataflow 4300, the application executing on the multi-purpose device 130 is configured with a user interface element that, when selected, sends a data upload 4302 including a selection of analyte data on-demand to the remote application server 155 via a web end point 4130. For example, when instructed, the multi-purpose device 130 can upload data corresponding to the most recent day, three days, five days, week, two weeks, and the like. In some examples, the patient can specify particular data to upload, such as analyte data corresponding to a specified time range.

Upon the remote application server 155 receiving the data upload, the AACS 3110 generates and returns an access code. The AMS 3120 stores the data and associates the data with the access code. The AACS 3110 provides the generated access code with a confirmation of data upload 4304. The multi-purpose device 130 displays the access code, along with instructions to access the data. For example, as described herein, the multi-purpose device 130 displays an URL address that can be entered into a web browser 4320. The webpage, provided by the remote application server is configured to display a field for a user (which can be the patient, a HCP, or another user) to enter the access code. The web browser 4320 causes access code to be sent to the AMS 3120 in a request 4307 for the report. The AMS 3120 validates the access code by providing the access code to the AACS 3110 in a validation request 4308. The AACS 3110 compares the access code from the validation request 4308 to the list of active and valid access codes. The AACS 3110 provides a response 4309 with the validity of the access code. If the access code is valid, the AMS 3120 generates the requested report based on the analyte data associated with the access code and provided via the data upload 4302. The AMS 3120 provides the report in a response 4310 to the web browser 4320. In some examples, the analyte data associated with the access code is discarded after the report is generated.

Note that the "quick share" function can also be implemented where the patient uses a data receiving device 120. For example, the data receiving device 120 software can include a user interface element to enable the user to request an analyte report for sharing on-demand, in a manner similar to that described for the multi-purpose device 130. In some examples, the data receiving device 120 selects the analyte data to be shared or the user selects the analyte data to be shared using the data receiving device 120. The data receiving device 120 generates a matrix barcode include the selected analyte data and URL configured to cause the multi-purpose device 130 to upload the analyte data with a request to use the quick share function as described. In some examples, the data receiving device 120 provides all available analyte data and the multi-purpose device 130 selects which analyte data to upload to the web end point 4130.

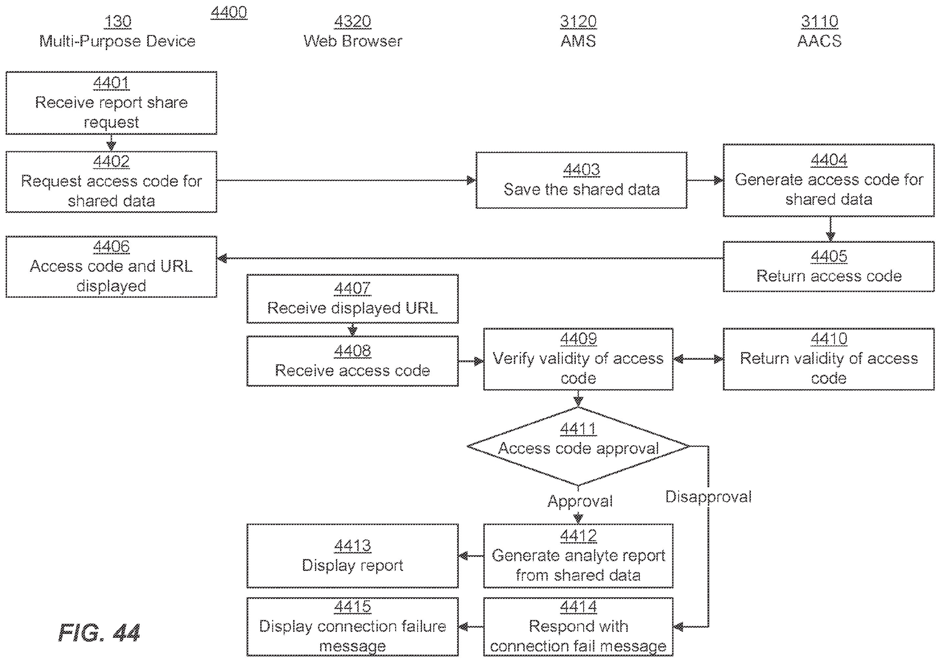

FIG. 44 illustrate a procedure for requesting and generating a report according to certain embodiments. The procedure 4400 illustrated in FIG. 44 corresponds to the dataflow 4300 illustrated in FIG. 43.

At 4401, the multi-purpose device 130 receives a share report request, for example using a "quick share" function provided by the software application executing on the multi-purpose device 130. The multi-purpose device 130 can be part of an analyte monitoring system 100, for example, a glucose monitoring system. The multi-purpose device 130 can comprise a display, one or more processors, a camera (or other hardware to scan a matrix barcode) and a memory communicatively coupled with the one or more processors. The memory can comprise instructions that, when executed by the one or more processors are configured to cause the one or more processors to perform operations comprising one or more operations of the procedure 4400, for example operations 4401, 4402, and 4406. In some examples, the multi-purpose device can initiate the share report request in response to scanning a matrix barcode generated and displayed by a data receiving device 120 to share data stored by the data receiving device 120. At 4402, the multi-purpose device 130 can generate and send a request for an access code to facilitate access to the analyte data or a report generated using the analyte data by the user, an HCP, or other trusted third parties.

At 4403, the AMS 3120 saves the shared data within one or more associated databases maintained by the analyte monitoring system 100. The AMS 3120 can be a part of an analyte monitoring system 100, for example, a glucose monitoring system. The AMS 3120 can be or include a server or server computing device comprise networking hardware, one or more processors, and a memory communicatively coupled with the one or more processors. The memory can comprise instructions that, when executed by the one or more processors are configured to cause the one or more processors to perform operations comprising one or more operations of the procedure 4400, for example operations 4403, 4409, 4411, 4412, and 4414. The AMS 3120 also requests the AACS 3110 to generate an access code to associate with the saved data.

At 4404 the AACS 3110 generates the access code. The AACS 3110 can be a part of an analyte monitoring system 100, for example, a glucose monitoring system. The AACS 3110 can be or include a server or server computing device comprise networking hardware, one or more processors, and a memory communicatively coupled with the one or more processors. The memory can comprise instructions that, when executed by the one or more processors are configured to cause the one or more processors to perform operations comprising one or more operations of the procedure 4400, for example operations 4404, 4405, and 4410. As an example, the shared data can be stored independently of other data uploaded by the multi-purpose device 130 so that sharing the shared data only shares the specific data specified and approved by the patient. As another example, the shared data can be stored in association with a patient's existing account records for further reference, but the access code can only be set as valid for sharing the shared data received by the AMS 3120 from the multi-purpose device. At 4405 the AACS 3110 returns the access code to the multi-purpose device 130. At 4406, the multi-purpose device 130 displays the access code and a URL to access the report based on the shared data as described herein. An example of displaying the access code and URL is shown and described in FIG. 20 and in FIG. 25 with respect to an example in which the user scans a matrix barcode displayed by a data receiving device 120.

At 4407 a web browser 4320 receives a request to access the URL displayed by the multi-purpose device 130. In some examples, the web browser 4320 is executing on the multi-purpose device 130 or another user device 140 of the patient. In some examples, the web browser 4320 is executing on a user device 140 of the patient's HCP or another computing device. In some examples, the web browser 4320 is executing on a user device 140 of a trusted third-party with whom the patient has elected to share a report based on recent analyte data. An example of a webpage to receive an access code and display a corresponding report is illustrated in FIG. 21. At 4408, the web browser 4320 receives the access code. For example, the patient can provide the access code to an HCP or other personnel associated with the HCP. The HCP can enter the access code into the web browser 4320, such as in a specified input field displayed by the web browser 4320. The HCP, or other user, can enter the access code in the context of making a request to review a report based on the shared analyte data. The web browser 4320 submits a web request to the AMS 3120, for example via a web end point 4130 for an analyte report for the patient. The web browser 4320 can include the access code, retrieved from the multi-purpose device 130 with the request.

After receiving the request from the web browser 4320 via web end point 4130, at 4409 the AMS 3120 attempts to verify the validity of the access code with the AACS 3110. For example, the AMS 3120 can provide the access code to the AACS with a verification request. The AACS 3110 can coordinate with the AMS 3120 at 4410 to return a validity determination of the access code. At 4411, the AMS 3120 evaluates the result of 4409 and 4410. If at 4411 the AMS 3120 approves the access code, then the procedure proceeds to 4412. At 4412, the AMS 3120 (or other component of the remote application server 155) generates a report based on the analyte data associated with the patient account records and uploaded by the multi-purpose device 130. At 4413, the AMS 3120 sends the generated report to the web browser 4320. At 4413, the web browser 4320 displays and/or stores the generated report. If at 4411, the AMS 3120 disapproves the access code, then the procedure proceeds to 4414. At 4414, the AMS 3120 responds to the web browser 4320 with a message indicating that the AMS system 3120 was not able to connect the received access code with an existing patient account. At 4415, the web browser 4320 displays the connection or association failure message and instructions to establish the connection or association.

The techniques are described in the context of data received or derived from an analyte sensor. In some examples, the analyte sensor can be a sensor configured, as described herein, to detect a specific analyte, such as glucose, lactate, ketone bodies, and the like. Regardless of the type of analyte being detected, the analyte monitoring system 100 can operate as described to establish, maintain, and share data along connections between servers of the analyte monitoring system 100 and an EMR system 1865. The analyte sensor can, additionally or alternatively include or be replaced with one or more other medical devices that generate, record, or otherwise provide data that would be advantageous to be shared between a dedicated monitoring system (e.g., the analyte monitoring system 100) and an EMR system 1865. The techniques described herein can be applied in a system integrating data from a device generating, recording, or otherwise providing data that would be advantageous to be shared between a monitoring system and an EMR system 1865, particularly where user or patient control of patient accounts are provided by the monitoring system and the EMR system 1865.

Although described in some examples as included a single server, the various systems and servers of the environments and dataflows described herein can include, by way of example and not limitation, one or more servers in a local or remote location, one or more servers in a distributed computing or processing environment (e.g., multiple server connected in the cloud), one or more logical servers representing one or more physical servers each, one or more processes or threads executing on computing hardware such that the named server is operating only part of the time on shared computing hardware, or any other suitable remote computing environment. As an example, the remote application server 155, report generation system 1860, EMR system 1865, AACS 3110, AMS 3120, and the variously described messaging and web servers can all be, include, or be embodied by one or more multiple servers working together or in parallel. Additionally, as described herein, all communication between the various devices, systems, and component described can be encrypted according to one or more encryption methodologies appropriate for the protection of personally identifiable information and/or patient health or medical records.

### The Clauses

1. A server computing device of a glucose monitoring system, the server computing device comprising: one or more processors; and a memory communicatively coupled with the one or more processors, the memory comprising instructions that, when executed by the one or more processors, are configured to cause the one or more processors to perform operations comprising:
   receiving, from an electronic medical records (EMR) system, a request to establish a connection between a set of patient records stored by the EMR system and associated with a patient and a set of patient records stored by the glucose monitoring system and associated with the patient, wherein the request includes patient identifying information;
   comparing the patient identifying information received with the request with patient identifying information stored by the glucose monitoring system;
   in response to determining, based on the comparison, that the set of patient records stored by the glucose monitoring system and associated with the patient exist:
      generating an association between the set of patient records stored by the EMR system and associated with the patient and the set of patient records stored by the glucose monitoring system and associated with the patient; and
      sending, to the EMR system, a confirmation of the association; and
   in response to determining, based on the comparison, that the set of patient records stored by the glucose monitoring system does not exist:
      sending, to the EMR system, a notification that the request to establish the connection cannot been completed.
2. The server computing device of clause 1, wherein the patient identifying information comprises patient name, patient date of birth, patient address, patient email address, patient phone number, or patient health care provider information.
3. The server computing device of any of clauses 1-2, wherein the patient identifying information comprises a patient identification code comprising an identifier unique to the patient in the EMR system.
4. The server computing device of any of clauses 1-3, wherein the instructions are further configured to cause the one or more processors to perform further operations comprising:
   determining that new glucose data associated with the set of patient records stored by the glucose monitoring system and associated with the patient is available; and
   sending, to the EMR system, the new glucose data associated with the set of patient records stored by the glucose monitoring system and associated with the patient.
5. The server computing device of any of clauses 1-4, wherein the instructions are further configured to cause the one or more processors to perform further operations comprising:
   requesting, from the EMR system, patient data associated with the set of patient records stored by the EMR system and associated with the patient; and
   receiving, from the EMR system in lieu of the requested patient data, a message indicating that no new patient data associated with the set of patient records stored by the EMR system and associated with the patient is available.
6. The server computing device of any of clauses 1-5, wherein the instructions are further configured to cause the one or more processors to perform further operations comprising:
   receiving, from the EMR system, a request for new glucose data associated with the set of patient records stored by the glucose monitoring system and associated with the patient;
   determining that no new glucose data associated with the set of patient records stored by the glucose monitoring system and associated with the patient is available; and
   sending, to the EMR system in lieu of the requested patient data, a message indicating that no new glucose data associated with the set of patient records stored by the glucose monitoring system and associated with the patient is available.
7. The server computing device of any of clauses 1-6, wherein the instructions are further configured to cause the one or more processors to perform further operations comprising:
   generating, with an account access code service, an access code associated with the set of patient records stored by the glucose monitoring system and associated with the patient; and
   sending, to the EMR system, the access code associated with the confirmation of the association.
8. The server computing device of any of clauses 1-7, wherein the instructions are further configured to cause the one or more processors to perform further operations comprising sending, to the EMR system, glucose data associated with the set of patient records stored by the glucose monitoring system and associated with the patient.
9. The server computing device of clause 8, wherein the patient data associated with the set of patient records stored by the EMR system and associated with the patient are new patient data received from the EMR system when the new patient data becomes available.
10. The server computing device of any of clause 8, wherein the instructions are further configured to cause the one or more processors to perform further operations comprising periodically sending, to the EMR system, glucose data associated with the set of patient records stored by the glucose monitoring system and associated with the patient.
11. The server computing device of any of clauses 1-10, wherein the instructions are further configured to cause the one or more processors to perform further operations comprising periodically receiving, from the EMR system, patient data associated with the set of patient records stored by the EMR system and associated with the patient.
12. The server computing device of any of clauses 1-11, wherein the request to establish a connection further comprises an access code generated by an account access code service and the instructions are further configured to cause the one or more processors to perform further operations comprising, prior to sending the confirmation of the association:
   determining, using the account access code service, that the access code is associated with the set of patient records stored by the glucose monitoring system and associated with the patient; and
   determining, using the account access code service, the validity of the access code.
13. The server computing device of clause 12, wherein the access code is configured to be valid for a predetermined period of time.
14. The server computing device of any of clauses 1-13, wherein the notification that the request to establish the connection cannot been completed further comprises instructions to establish the connection.
15. The server computing device of any of clauses 1-14, wherein the instructions are further configured to cause the one or more processors to perform further operations comprising, subsequent to sending the confirmation of the association:
   receiving, by the server computing device and from the EMR system, a request to access a report generated based on glucose data stored in the set of patient records stored by the glucose monitoring system and associated with the patient;
   generating, by the server computing device and with a report generating system, the requested report; and
   sending, by the server computing device and to the EMR system, the generated report.
16. The server computing device of clause 15, wherein the request to access the report identifies the glucose data for which the report is requested.
17. The server computing device of clause 15, wherein the instructions are further configured to cause the one or more processors to perform further operations comprising:
   requesting, from the EMR system, updated patient data associated with the set of patient records stored by the EMR system and associated with the patient; and
   receiving, from the EMR system, the updated patient data associated with the set of patient records stored by the EMR system and associated with the patient, wherein the requested report is generated based on the updated patient data.
18. The server computing device of clause 15, wherein the instructions are further configured to cause the one or more processors to perform further operations comprising identifying glucose data to include in the requested report based on a datestamp or timestamp associated with the identified glucose data.
19. A data receiving device of a glucose monitoring system comprising: a display; one or more processors; and a memory communicatively coupled with the one or more processors comprising instructions that, when executed by the one or more processors, are configured to cause the one or more processors to perform operations comprising:
   receiving, from a user of the glucose monitoring system, a request to transfer glucose data associated with the user to another computing device;
   encoding the glucose data;
   generating a matrix barcode comprising a uniform resource locator (URL) and the encoded glucose data, wherein the glucose data is formatted as parameters to be included with the URL by a web browser; and
   causing the matrix barcode to be presented on the display.
20. The data receiving device of clause 19, wherein the instructions are further configured to cause the one or more processors to perform further operations comprising selecting the glucose data for encoding based on the glucose data corresponding to a predetermined range of time.
21. The data receiving device of any of clauses 19-20, wherein the instructions to encode the glucose data are further configured to cause the one or more processors to perform further operations comprising:
   calculating one or more derivative values from the glucose data; and
   encoding the derivative values with the glucose data.
22. The data receiving device of any of clauses 19-21 wherein encoding the glucose data comprises reducing a level of precision associated with the glucose data, wherein the level of precision is selected based on an actual value of the glucose data.
23. The data receiving device of any of clauses 19-22, wherein the instructions are further configured to cause the one or more processors to perform further operations comprising:
   generating report elements for a glucose report based on the glucose data; and
   encoding the report elements for the glucose report, wherein the matrix barcode further comprises the encoded glucose report elements.
24. The data receiving device of any of clauses 19-23, wherein the data receiving device is not configured for wireless communication.
25. A multi-purpose device of a glucose monitoring system comprising: a camera; a display; one or more processors; and a memory communicatively coupled with the one or more processors comprising instructions that, when executed by the one or more processors, are configured to cause the one or more processors to perform operations comprising:
   causing a matrix barcode to be scanned via the camera;
   decoding the matrix barcode to obtain a uniform resource locator (URL) and one or more parameters, wherein the one or more parameters comprise at least glucose data;
   presenting, using the URL and the one or more parameters, a web request to a server associated with the URL;
   receiving by the multi-purpose device and from the user associated with the user, an access code associated with the one or more parameters; and
   causing the access code to be output via the display.
26. The multi-purpose device of clause 25, wherein the server associated with the URL is further associated with the glucose monitoring system and wherein the access code is a temporary one-time password associated with the one or more parameters by the server.
27. The multi-purpose device of clause 26, wherein the access code is configured to expired after a predetermined period of time.
28. The multi-purpose device of any of clauses 25-27, wherein the instructions are further configured to cause the one or more processors to perform further operations comprising:
   receiving, by the multi-purpose device and from the server associated with the URL, a report based on the glucose data included in the parameters, wherein the report was generated by the server based on the parameters presented with the URL; and
   causing the report to be output via a display.
29. The multi-purpose device of clause 28, wherein:
   the matrix barcode is generated and displayed by a data receiving device of the glucose monitoring system, and
   the one or more parameters further comprise a device identifier associated with the multi-purpose device, a device identifier associated with the data receiving device, or a patient identifier associated with the user.
30. The multi-purpose device of clause 29, wherein the data receiving device is not configured for wireless communication.
31. A server computing device of a glucose monitoring system comprising: one or more processors; and a memory communicatively coupled with the one or more processors comprising instructions that, when executed by the one or more processors, are configured to cause the one or more processors to perform operations comprising:
   receiving a first web request comprising one or more parameters from a multi-purpose device of the glucose monitoring system, wherein the one or more parameters comprise glucose data associated with a user of the glucose monitoring system;
   generating a report based on the received glucose data;
   associating an access code with the report;
   receiving a second web request comprising one or more parameters, wherein the one or more parameters comprise the access code;
   retrieving the report based on the access code; and
   causing the report to be output on a display.
32. The server computing device of clause 31, wherein the instructions are further configured to cause the one or more processors to perform further operations comprising sending the access code to the multi-purpose device in response to receiving the first web request.
33. The server computing device of any of clauses 31-32, wherein the second web request is received from a computing device associated with an electronic medical records (EMR) system.
34. The server computing device of any of clauses 31-33, wherein the second web request is received from the multi-purpose device.
35. The server computing device of any of clauses 31-34, wherein the second web request is received form a computing device associated with the user of the glucose monitoring system.
36. The server computing device of any of clauses 31-35, wherein the first web request is sent by the multi-purpose device subsequent to scanning a matrix barcode.
37. The server computing device of any of clauses 31-36, wherein the one or more parameters further comprise a device identifier associated with the multi-purpose device, a device identifier associated with a data receiving device, or a patient identifier associated with the user.
38. A glucose monitoring system comprising: one or more processors; and a memory communicatively coupled with the one or more processors comprising instructions that, when executed by the one or more processors, are configured to cause the one or more processors to perform operations comprising:
   establishing a communication session between the glucose monitoring system and an electronic medical records (EMR) system;
   identifying therapy information associated with a user and stored in the EMR system;
   causing the therapy information associated with the user to be transferred from the EMR system to the application server;
   converting the therapy information for use by the glucose monitoring system;
   storing the converted therapy information in a database accessible to the glucose monitoring system;
   causing the therapy information to be displayed for a user of the glucose monitoring system.
39. The glucose monitoring system of clause 38, wherein the therapy information is stored a first structured format; and wherein converting the therapy information comprises mapping the first structure format to a second structured format associated with glucose monitoring system.
40. The glucose monitoring system of any of clauses 38-39, wherein the therapy information is stored in a free text format; and wherein converting the therapy information comprises:
   interpreting the therapy information using a natural language processing; and
   storing the information in a structured format associated with the glucose monitoring system.
41. The glucose monitoring system of any of clauses 38-40, wherein the instructions are further configured to cause the one or more processors to perform further operations comprising:
   receiving additional therapy information through a structured format input;
   converting the additional therapy information for use by the EMR system; and
   transferring the converted additional therapy information to the EMR system for storage.
42. A glucose monitoring system comprising: one or more processors; and a memory communicatively coupled with the one or more processors comprising instructions that, when executed by the one or more processors, are configured to cause the one or more processors to perform operations comprising:
   establishing a communication session between the glucose monitoring system and an electronic medical records (EMR) system;
   receiving a request from a user device for the EMR system to access glucose data associated with a patient and stored in a database accessible to the glucose monitoring system;
   causing a multi-purpose device associated with the glucose monitoring system and the patient to display an access code associated with the glucose data associated with the patient;
   receiving a second access code from the user device;
   comparing the access code displayed by the multi-purpose device and the second access code; and
   in response to determining that the access code displayed by the multi-purpose device and the second access code are equivalent, granting access to the EMR system to access the glucose data associated with the patient.
43. The glucose monitoring system of clause 42, wherein the access code is generated by the multi-purpose device and the glucose monitoring system receives the access code from the multi-purpose device.
44. The glucose monitoring system of any of clauses 42-43, wherein the access code is generated by the glucose monitoring system and the multi-purpose device receives the access code from the glucose monitoring system.
45. The glucose monitoring system of any of clauses 42-44, wherein the instructions are further configured to cause the one or more processors to perform further operations comprising:
   cause the multi-purpose device to display an authorization request, wherein the EMR system is granted access in response to an affirmative response to the authorization request.
46. The glucose monitoring system of any of clauses 42-45, wherein one or more of the access code and the second access code comprise a matrix barcode.

## Claims

1. A glucose monitoring system comprising: one or more processors; and a memory communicatively coupled with the one or more processors comprising instructions that, when executed by the one or more processors, are configured to cause the one or more processors to perform operations comprising:
establishing a communication session between the glucose monitoring system and an electronic medical records (EMR) system;
receiving a request from a user device for the EMR system to access glucose data associated with a patient and stored in a database accessible to the glucose monitoring system;
causing a multi-purpose device associated with the glucose monitoring system and the patient to display an access code associated with the glucose data associated with the patient;
receiving a second access code from the user device;
comparing the access code displayed by the multi-purpose device and the second access code; and
in response to determining that the access code displayed by the multi-purpose device and the second access code are equivalent, granting access to the EMR system to access the glucose data associated with the patient.

2. The glucose monitoring system of claim 1, wherein the access code is generated by the multi-purpose device and the glucose monitoring system receives the access code from the multi-purpose device.

3. The glucose monitoring system of any of claims 1 or 2, wherein the access code is generated by the glucose monitoring system and the multi-purpose device receives the access code from the glucose monitoring system.

4. The glucose monitoring system of any of claims 1-3, wherein the instructions are further configured to cause the one or more processors to perform further operations comprising:
cause the multi-purpose device to display an authorization request, wherein the EMR system is granted access in response to an affirmative response to the authorization request.

5. The glucose monitoring system of any of claims 1-4, wherein one or more of the access code and the second access code comprise a matrix barcode.

6. A server computing device of a glucose monitoring system comprising: one or more processors; and a memory communicatively coupled with the one or more processors comprising instructions that, when executed by the one or more processors, are configured to cause the one or more processors to perform operations comprising:
receiving a first web request comprising one or more parameters from a multi-purpose device of the glucose monitoring system, wherein the one or more parameters comprise glucose data associated with a user of the glucose monitoring system;
generating a report based on the received glucose data;
associating an access code with the report;
receiving a second web request comprising one or more parameters, wherein the one or more parameters comprise the access code;
retrieving the report based on the access code; and
causing the report to be output on a display.

7. The server computing device of claim 6, wherein the instructions are further configured to cause the one or more processors to perform further operations comprising sending the access code to the multi-purpose device in response to receiving the first web request.

8. The server computing device of any of claims 6 or 7, wherein the second web request is received from a computing device associated with an electronic medical records (EMR) system.

9. The server computing device of any of claims 6-8, wherein the second web request is received from the multi-purpose device.

10. The server computing device of any of claims 6-9, wherein the second web request is received form a computing device associated with the user of the glucose monitoring system.

11. The server computing device of any of claims 6-10, wherein the first web request is sent by the multi-purpose device subsequent to scanning a matrix barcode.

12. The server computing device of any of claims 6-11, wherein the one or more parameters further comprise a device identifier associated with the multi-purpose device, a device identifier associated with a data receiving device, or a patient identifier associated with the user.
